# EUROPEAN PATENT APPLICATION

(11) **EP 4 834 917 A1**
(43) Date of publication of application: **04.11.2026**
(21) Application number: 25174112.0
(22) Date of filing: 02.05.2025
(51) Int. Cl.: C07K 14/005, C12N 15/861

(54) **TARGETING PEPTIDES, AAV VARIANTS AND USES THEREOF**

(71) Applicant: AC Immune SA, 1015 Lausanne (CH)
(72) Inventor: NEVOLTRIS, Damien, 1015 Lausanne (CH); OLLIER, Romain, 1015 Lausanne (CH)
(74) Representative: Boult Wade Tennant LLP

(57) **Abstract**

The present invention is in the field of CNS targeting peptides and engineered adeno-associated virus (AAV) capsids and vectors. Described herein are CNS targeting peptides, AAV capsid proteins comprising said CNS targeting peptides, and AAV vectors comprising said AAV capsid proteins. The engineered AAV vectors described have an improved tropism for the CNS.

## Description

### TECHNICAL FIELD

The present invention is in the field of targeting peptides and adeno-associated virus vectors. In particular, the disclosure relates to CNS targeting peptides, engineered AAV capsid proteins comprising said CNS targeting peptides and uses thereof.

### BACKGROUND ART

Adeno-associated viruses (AAVs) are small, non-enveloped viruses belonging to the Dependoparvovirus genus. AAVs comprise single-stranded DNA genome of approximately 4.7 kilobases and require co-infection with a helper virus, such as adenovirus or herpesvirus, for replication (Samulski and Muzyczka 2014). AAV is non-pathogenic in humans, making it an attractive vector for gene therapy (Kuzmin et al. 2021). In gene therapy, AAV vectors are used to deliver therapeutic genes to target cells, offering innovative treatments for various genetic disorders. For example, AAV-based therapies have been approved for conditions like spinal muscular atrophy (Zolgensma^{®}, onasemnogene abeparvovec-xioi), retinal dystrophy (Luxturna^{®}; voretigene neparvovec-rzyl), and hemophilia B (Hemgenix^{®}, etranacogene dezaparvovec-drlb). AAV vectors can infect both dividing and non-dividing cells and can persist in an extrachromosomal state without integrating into the host genome, reducing the risk of insertional mutagenesis (Lundstrom 2023). Thanks to its versatility and favourable safety profile, AAV has become a cornerstone in the development of gene therapies, with ongoing research exploring its potential for treating a wide range of diseases. In recent years, adeno-associated virus (AAV) vector engineering has become an important area of research in gene therapy, aimed at enhancing the specificity and efficiency of gene delivery by AAV vector to target tissues.

### Prior art

WO2024/220719 describes AAVrh10 variants comprising substitution in variable region IV and/or peptide insertion in variable region VIII of the AAV capsid protein.

WO2015/038958 describes AAV9 variants with CNS tropism comprising a peptide insertion in variable region VIII of the AAV capsid protein.

WO2024/16003 describes CNS-specific n-mer motif and AAV9 variants with CNS tropism comprising said CNS-specific n-mer motif.

WO2023/225508 describes AAV2 variants with CNS tropism comprising a peptide insertion in variable region VIII of the AAV capsid protein.

### SUMMARY OF THE INVENTION

AAV vectors present a limited ability to precisely deliver therapeutic transgenes to specific tissues or cell types and high doses of AAV vectors are often required to achieve therapeutic efficacy. However, these elevated doses can result in dose-dependent toxicity, largely due to transduction in non-targeted tissues. Clinical studies have reported adverse effects such as liver toxicity and systemic inflammation (Duan 2023), highlighting the need for more efficient and targeted AAV vectors. This challenge is particularly critical for gene therapy applications in the brain, where the blood-brain barrier (BBB) presents a major obstacle for delivering therapeutic genes to the central nervous system (CNS). Accordingly, it is an object of the present invention to provide novel CNS targeting peptides that can, *inter alia,* when comprised in an AAV capsid protein, direct the AAV capsid to target the CNS, in particular to the brain and/or spinal cord. In a further aspect, the CNS targeting peptide of the invention can, when comprised in an AAV capsid protein, direct the AAV capsid to de-target the liver.

Accordingly, in one aspect, the invention provides a central nervous system (CNS) targeting peptide, which comprises or consists of:
a. an amino acid sequence of any one of SEQ ID NOs: 315, 4-314 or 316-436; or
b. at least 4, at least 5, or at least 6 contiguous amino acids of an amino acid sequence of any one of SEQ ID NOs: 315, 4-314 or 316-436; or
c. an amino acid sequence having at least 57%, at least 71%, or at least 85% sequence identity to the amino acid sequence of SEQ ID NOs: 315, 4-314 or 316-436.

In another aspect, the invention provides an AAV capsid protein comprising a CNS targeting peptide of the invention. The invention also provides an AAV capsid comprising an AAV capsid protein that comprises a CNS targeting peptide of the invention. The AAV capsid protein may be an AAVrh10 capsid protein (apart from comprising a CNS targeting peptide of the invention). The CNS targeting peptide may be inserted, substituted (or a combination thereof) in a variable region of the AAV capsid protein. The CNS targeting peptide may be inserted in VR-VIII of the AAV capsid protein. The CNS targeting peptide may be inserted between amino acids 589 and 590 (in VR-VIII) of an AAVrh10 capsid protein of SEQ ID NO:

The AAV capsid protein may comprise or consist of an amino acid sequence of any one of SEQ ID NOs: 477, 437-476 or 478-497.

In another aspect, the invention provides an AAV vector comprising an AAV capsid protein of the invention. The AAV vector comprises an AAV genome comprising an expression cassette flanked by inverted terminal repeats (ITRs). The expression cassette may comprise from 5' to 3': a promoter and one or more transgenes. The expression cassette may comprise one or more further regulatory elements. The AAV vectors of the invention are capable of transducing cells of the CNS, in particular cells of the brain and/or spinal cord. The AAV vector of the invention may exhibit an increased transduction of cells of the CNS relative to a corresponding AAV vector comprising an AAV capsid protein which does not comprise a CNS targeting peptide of the invention. The AAV vector of the invention may exhibit a decreased transduction of cells of the liver relative to a corresponding AAV vector comprising an AAV capsid protein which does not comprise a CNS targeting peptide of the invention. The AAV vector of the invention may exhibit an increased transduction of cells of the CNS and a decreased transduction of cells of one or more of the liver, kidney, muscle, spleen or heart relative to a corresponding AAV vector comprising an AAV capsid protein which does not comprise a CNS targeting peptide of the invention. The AAV vector may be administered to a subject systemically, in particular parenterally and preferably intravenously. The subject may be a mammal, in particular a rodent, a non-human primate or a human.

The invention also provides a pharmaceutical composition comprising a CNS targeting peptide of the invention, an AAV capsid protein of the invention and/or an AAV vector of the invention, and a pharmaceutically acceptable carrier, adjuvant and/or excipient.

The invention also provides a nucleic acid encoding a CNS targeting peptide of the invention, an AAV capsid protein of the invention and/or an AAV vector of the invention.

The invention also provides a method of producing an AAV vector of the invention, the method comprising introducing into a host cell: (a) a nucleic acid encoding a recombinant viral genome comprising a replication (Rep) gene and a capsid (Cap) gene flanked by inverted terminal repeats (ITRs), wherein the capsid (Cap) gene encodes an AAV capsid protein of the invention as described herein; (b) a nucleic acid comprising an expression cassette flanked by inverted terminal repeats (ITRs); and (c) a nucleic acid comprising an AAV helper virus genome;
and isolating the AAV vector.

The invention also provides an AAV vector of the invention or pharmaceutical composition of the invention for use in human or veterinary medicine. The AAV vector or the pharmaceutical composition may be for use in the prevention, alleviation and/or treatment of a disease, disorder and/or conditions of the CNS. In a similar aspect, the invention also relates to the use of an AAV vector of the invention or pharmaceutical composition of the invention for the manufacture of a medicament for the prevention, alleviation and/or treatment of a disease, disorder and/or conditions of the CNS. In a further similar aspect, the invention also provides a method for preventing, alleviating and/or treating a CNS disease, disorder and/or condition in a subject in need thereof, the method comprising administering a therapeutically effective amount of an AAV vector of the invention or a pharmaceutical composition of the invention to the subject. The AAV vector of the invention or pharmaceutical composition of the invention can be administered to a subject by any conventional route, including injection or by gradual infusion over time. The administration may be via parenteral administration. The administration may, for example, be by infusion or by intravenous, intraperitoneal, intranasal, intravitreous, subcutaneous, intramuscular, intrathecal, intracisternal, intraparenchymal, intrastriatal or intracerebroventricular route.

The invention also provides a CNS targeting peptide of the invention, an AAV capsid protein of the invention, or an AAV vector of the invention for use as a research tool.

### DETAILED DESCRIPTION OF EMBODIMENTS OF THE INVENTION

### Definitions

### Adeno-associated virus

The term "vector" refers to any particle used to transport (by transduction or transfection) a polynucleotide(s) into a host cell. This definition includes both non-viral and viral vectors.

The term "viral vector" includes vectors derived from wild-type viruses and engineered (e.g. modified) viruses. Examples of viral vectors include, but are not limited to, adeno-associated virus (AAV), adenovirus, retrovirus, rhinovirus, lentivirus, hepatovirus, herpes simplex virus (HSV) and any virus-like particle.

As used herein, an "AAV vector" refers to an adeno-associated virus suitably used to transport a polynucleotide(s) into a host cell.

An adeno-associated virus (AAV) is a small, non-enveloped virus belonging to the Dependoparvovirus genus and is used in the art as a vector for the delivery of polynucleotide into host cell. The AAV capsid and genome are key components of an AAV and are crucial for the AAV function. The AAV capsid is composed of three viral proteins: VP1, VP2, and VP3, which form an icosahedral structure made up of 60 subunits, providing stability and facilitating the virus's ability to transduce host cells. VP1 contains a phospholipase A2 (PLA2) domain essential for endosomal escape, while VP2 assists in capsid assembly, and VP3 forms the bulk of the capsid. The AAV genome is a single-stranded DNA (ssDNA) molecule approximately 4.7 kilobases in length, flanked by inverted terminal repeats (ITRs) that are crucial for replication and packaging of the AAV genome in the capsid. These ITRs are short palindromic sequences at both ends of the AAV genome, forming T-shaped hairpin structures that serve as origins of replication and are recognized by the viral replication machinery. The Rep gene encodes proteins necessary for viral replication and packaging, while the Cap gene encodes the capsid proteins (VP1, VP2, and VP3). The VP1, VP2, and VP3 proteins are produced by alternative splicing of the mRNA transcribed from the Cap gene. The VP1 protein results from the translation of the full mRNA transcribed from the Cap gene, while VP2 and VP3 are the result of alternative splicing and are an N-truncated form of VP1. VP2 is approximately 137 amino acids shorter than VP1, while VP3 is approximately 57 amino acids shorter than VP2 (Wörner et al. 2021).

Many different AAV wild-type serotypes exist such as AAV1, AAV2, AAV3, AAV4, AAV5, AAV6, AAV7, AAV8, AAV9, AAV10, AAV11, AAV12, AAVrh10, AAVrh39, AAVrh43, AAVrh74 AAVpo1, AAVpo4, AAVpo5, AAVpo6, and AAVpo7. Several engineered AAVs have been developed such as, for example, AAV2/8, AAV2/9, AAV-DJ, AAV-LK03, AAV-PHP.B or AAV-PHP.eB. An "engineered AAV" is a modified version of a wild-type AAV, designed to enhance its properties for specific applications, particularly in the context of gene therapy. These modifications may include changes to the capsid proteins to alter tissue tropism, improve transduction efficiency, or evade the host immune response. Engineered AAVs are created by techniques such as directed evolution, rational design, or DNA shuffling, allowing researchers to tailor the virus to target specific cells or tissues more effectively.

As used herein, the "tropism" of an AAV refers to the virus's ability to preferentially transduce specific organ(s), tissue(s), cell type(s) and/or cell population(s). Without wishing to be bound by theory, this selective targeting is primarily determined by the capsid proteins of the AAV, which interact with specific receptors on the surface of cells. Different AAV serotypes exhibit varying tropisms, meaning they have different affinities for different organ(s), tissue(s), cell type(s) and/or cell population(s). For example, AAV2 has a natural tropism for skeletal muscles, neurons, and hepatocytes, while AAV9 is known for its ability to cross the blood-brain barrier and target the central nervous system. Identical AAV vectors may present different tropism depending on different parameters, which include but are not limited to, the amount of AAV administered, the delivery method (e.g., intravenous, intramuscular, intrathecal) and the subject administered with the AAV (e.g., animal species and even strains within a species can affect AAV tropism due to differences in receptor expression and immune responses).

The capsid proteins of adeno-associated viruses (AAVs) comprise several variable regions (VRs). These regions are crucial for the AAV's ability to transduce specific cell types, and thus its tropism. The variable regions, also commonly referred to as surface loops, are present on the exterior of the capsid, allowing them to interact with the environment, for example with cell surface receptors. There are nine primary variable regions (VR-I to VR-IX) identified in AAV capsids. These regions contribute to the diversity in receptor binding and immune response evasion among different AAV serotypes. The location of the variable regions on the capsid protein sequence varies depending on the AAV serotypes, some of them are shown in Table 1 with reference to (Stagg et al. 2022). Variable regions of AAVrh10 and AAV1 were defined based on AAV serotypes from the same clade, i.e. of similar sequence and structure. AAVrh10 and AAV8 belong to clade E (Gao et al. 2004) with 93.5% sequence identity and are structurally very similar with variable region sharing the same definition (Mietzsch et al. 2020). AAV1 and AAV6 belong to clade A with 99.2% sequence identity (Gao et al. 2004).

**Table 1. Amino acid position of variable regions (VRs) I to IX in the capsid protein of different AAV serotypes**

| **AAV serotype** | **SEQ ID NO** | **VR-I** | **VR-II** | **VR-III** | **VR-IV** | **VR-V** | **VR-VI** | **VR-VII** | **VR-VIII** | **VR-IX** | **Accession number (GenBank)** |
|---|---|---|---|---|---|---|---|---|---|---|---|
| **AAVrh10** | 1 | 260-271 | 329-334 | 383-391 | 452-472 | 490-508 | 528-545 | 547-559 | 582-597 | 706-714 | AAO88201.1 |
| **AVV9** | 2 | 259-270 | 328-333 | 382-390 | 451-470 | 488-506 | 526-543 | 545-557 | 580-595 | 704-712 | AAS99264.1 |
| **AAV2** | 498 | 25-268 | 326-331 | 380-388 | 449-469 | 487-505 | 525-542 | 544-556 | 579-594 | 703-711 | YP_680426.1 |
| **AAV1** | 499 | 259-269 | 327-332 | 381-389 | 450-470 | 488-506 | 526-543 | 545-557 | 580-595 | 704-712 | NP_049542.1 |
| **AAV5** | 500 | 249-259 | 317-322 | 371-381 | 442-456 | 474-492 | 512-529 | 531-546 | 569-584 | 692-700 | YP_068409.1 |
| **AAV3b** | 501 | 259- 268 | 326-331 | 380-388 | 449-470 | 488-506 | 526-543 | 545-557 | 580-595 | 704-712 | 3KIC_A |
| **AAV4** | 502 | 253-259 | 317-321 | 371-382 | 443-464 | 482-505 | 525-541 | 543-555 | 578-593 | 702-710 | NP_044927.1 |
| **AAV6** | 503 | 259-269 | 327-332 | 381-389 | 450-470 | 488-506 | 526-543 | 545-557 | 580-595 | 704-712 | AAB95450.1 |
| **AAV7** | 504 | 260-270 | 328-333 | 382-390 | 451-472 | 490-508 | 528-545 | 547-558 | 581-596 | 705-713 | YP_077178.1 |
| **AAV8** | 505 | 260-271 | 329-334 | 383-391 | 452-472 | 490-508 | 528-545 | 547-559 | 582-597 | 706-714 | YP_077180.1 |

| | | | | | | | | | | | |
|---|---|---|---|---|---|---|---|---|---|---|---|
| *The variable regions amino acid positions are described with reference to* (Stagg et al. 2022). | | | | | | | | | | | |

### Targeting peptide

As used herein, the term "targeting peptide" refers to a short amino acid sequence capable of directing an agent to a defined target. The target may be one or more organs, one or more tissues, one or more cell types, and/or one or more cell populations. The agent may be any agent (e.g. a therapeutic agent, an imaging agent or a delivery system) provided it can comprise or be suitably linked to the targeting peptide such that the targeting peptide is capable of directing the agent to the target. Examples of agents envisaged within the present invention include, but are not limited to, proteins (e.g. antibodies), liposomes, viral vectors or nanoparticles. A preferred agent in the context of the present invention is an adeno-associated virus (AAV).

Without wishing to be bound by theory, the ability of a targeting peptide to direct an agent to a target may lie in the targeting peptide's ability to interact and/or modify the agent interaction with cell surface molecules such as receptor proteins, glycans of glycoproteins or glycolipids, and/or lipids.

As used herein, a "central nervous system (CNS) targeting peptide" is a targeting peptide capable of directing an agent to one or more organs, one or more tissues, one or more anatomical regions (e.g. cerebral cortex, hippocampus, amygdala, cerebellum, brainstem, basal ganglia, retina), one or more cell types, and/or one or more cell populations of the central nervous system. A CNS targeting peptide may direct an agent to the brain and/or spinal cord and thus may be further defined as a brain and/or spinal cord targeting peptide. In the context of the agent being an AAV, the ability of the targeting peptide to direct the AAV to the target organ(s), tissue(s), cell type(s) and/or cell population(s) may be defined as the ability of the targeting peptide to direct the AAV to transduce the target organ(s), tissue(s), anatomical region(s), cell type(s) and/or cell population(s) and thus modify the AAV tropism. The CNS targeting peptide increases directing of the agent to one or more organs, one or more tissues, one or more anatomical regions (e.g. cerebral cortex, hippocampus, amygdala, cerebellum, brainstem, basal ganglia, retina), one or more cell types, and/or one or more cell populations of the central nervous system as compared to directing of the agent in the absence of the CNS targeting peptide.

In addition to directing an agent to a defined target, a targeting peptide can also prevent or reduce an agent's direction to non-target (also referred to as off-target) organ(s), tissue(s), anatomical region(s), cell type(s) and/or cell population(s). Such capability of a targeting peptide is referred to herein as "de-targeting". Thus, a targeting peptide able to direct an agent to the CNS and prevent the agent from being directed to the liver may be referred to as a "CNS targeting peptide de-targeting the liver", a "CNS targeting peptide capable of liver de-targeting" or a "CNS targeting peptide that de-targets the liver". In the context of the agent being an AAV, a targeting peptide capable of de-targeting organ(s), tissue(s), anatomical region(s), cell type(s) and/or cell population(s) is thus capable of reducing or preventing transduction of said organ(s), tissue(s), cell type(s) and/or cell population(s). The CNS targeting peptide may decrease directing of the agent to one or more non-target organs, one or more non-target tissues, one or more non-target anatomical regions, one or more non-target cell types, and/or one or more non-target cell populations as compared to directing of the agent in the absence of the CNS targeting peptide.

### Expression cassette

As used herein, an "expression cassette" refers to a nucleic acid construct comprising various operably linked polynucleotide elements, including at least one promoter and one transgene destined for expression. The expression cassette may comprise sequences providing or encoding one or more promoters operably linked to a nucleic acid encoding one or more transgenes, a ribosomal binding site, a start codon, a stop codon, a transcription termination sequence, and one or more further regulatory elements such as a poly A (polyadenylation) element.

The term "operably linked" as used herein refers to the arrangement of various nucleic acid elements relative to each other such that the elements are functionally connected and are able to interact with each other in the manner intended. Such elements may include, without limitation, a promoter, an enhancer, a polyadenylation element, one or more miRNA binding sites, one or more introns and/or exons, and a coding sequence of a transgene or transgenes of interest to be expressed. The nucleic acid elements, when properly oriented or operably linked, act together to modulate the activity of one another, and ultimately may affect the level of expression of a product (e.g. a transgene). By "modulate" is meant increasing, decreasing, or maintaining the level of activity of a particular element. As understood by a person of average skill in the art, "operably linked" implies functional activity, and is not necessarily related to a natural positional link.

As used herein, the term "regulatory element" refers to a nucleic acid sequence capable of modulating (e.g. increasing, decreasing or maintaining) the expression of an operably linked sequence (e.g. a gene). Non-limiting examples of regulatory elements include promoter, enhancer, repressor, silencer, intron, UTR, miRNA binding sites, internal ribosome entry site (IRES) sequences, post-translational response element, post-transcriptional regulatory element (e.g. WPRE) and poly A element.

An expression cassette may comprise a nucleic acid sequence encoding for a Woodchuck Hepatitis Virus Posttranscriptional Regulatory Element (WPRE) or any derivative of the WPRE sequence such as oPRE (an optimized and truncated version of WPRE which does not comprise potentially oncogenic elements) at the 3'end. The WPRE sequence is routinely used to increase expression of genes delivered by viral vectors such as AAVs. Without wishing to be bound by theory, inclusion of the sequence in expression cassettes can increase mRNA stability and thus protein yield.

An expression cassette may comprise a sequence encoding an "internal ribosome entry site" (IRES). As used herein, the term "internal ribosome entry site" refers to an RNA element that acts to promote recruitment of ribosomes. The IRES element is therefore able to promote protein translation in a 5' cap independent manner. An IRES element is advantageous in expression cassettes containing more than one gene for expression (such as, for example, separate expression of light and heavy chains of an antibody).

As used herein, the term "promoter" refers to a region of DNA that generally is located upstream of a polynucleotide sequence (e.g. the polynucleotide sequence encoding a transgene) to be transcribed that is needed for transcription to occur, e.g. which initiates transcription. Promoters may be ubiquitous or tissue/cell-specific. Ubiquitous promoters drive gene expression in almost all cell types and tissues, sometimes also across animal species or strains, making them useful for general gene expression studies. Ubiquitous promoters are sometimes referred to as constitutive, universal, general or non-specific promoters. Non-limiting examples of ubiquitous promoters include CMV (cytomegalovirus) promoter, EF1A (Eukaryotic translation elongation factor 1 alpha 1) promoter, CAG (CMV early enhancer fused to modified chicken β-actin promoter) promoter, CBA (CMV early enhancer fused to chicken β-actin promoter and SV40 intron) promoter, CBh (CMV early enhancer fused to modified chicken β-actin promoter, also known as chicken β-actin hybrid promoter) promoter or SV40 (Simian virus 40 enhancer/early promoter) promoter. In contrast, tissue/cell-specific promoters are active only in particular cell types, tissues or system (e.g. the central nervous system), allowing for targeted gene expression. This specificity is crucial for studying gene function in specific biological contexts and for therapeutic applications where precise control of gene expression is needed. Non-limiting examples of tissue/cell-specific promoters, in particular central nervous system-specific promoters, include GFAP (glial fibrillary acidic protein) promoter, gfaABC(1)D (modified GFAP promoter) promoter, CMVe-GFAP (CMV enhancer fused to gfaABC(1)D promoter) promoter, ATP1A2 (Na, K ATPase α2) promoter, CLDN5 (Claudin 5) promoter, ADRB2 (Adrenoceptor beta 2) promoter, TNFRSF6B (TNF receptor superfamily member 6b) promoter, PDYN (prodynorphin) promoter, GH1 (Human growth hormone) promoter, OPALIN (Opalin) promoter, SYN1 (Synapsin 1) promoter, CMVe-SYN1 (CMV enhancer fused to Synapsin 1 promoter) promoter, CAMK2A (Calcium/Calmodulin Dependent Protein Kinase II alpha) promoter, NEFH (neurofilament heavy polypeptide) promoter, NEUROD6 (neuronal differentiation factor 6) promoter, NSE (Neuron-Specific Enolase) promoter, PGK (PhosphoGlycerate Kinase) promoter, OLIG2 (oligodendrocyte transcription factor 2) promoter or CMVe-OLIG2 (CMV enhancer fused to OLIG2 promoter) promoter.

The term promoter includes synthetic promoters. The term "synthetic promoter" as used herein relates to a promoter that does not occur in nature. For example, functional variants of naturally occurring promoters can be used in accordance with the invention. A "functional variant" of a promoter in the context of the present invention is a variant of a reference promoter that retains the ability to function in the same way as the reference promoter. Truncated forms of naturally occurring promoters may be used. The promoter may be operably linked to an enhancer such as the CMV early enhancer or fused to an intron sequence(s). The addition of an operably linked enhancer or intron may enhance or stabilize expression of a transgene. Truncated or modified naturally occurring promoters can be used to facilitate insertion of relatively large transgene encoding sequences into a vector such as an AAV vector.

As used herein, the term "transgene" refers to a gene (therefore a nucleic acid sequence coding for a protein sequence) that has been transferred from one organism to another. In the context of the present invention, one or more transgenes may be part of an expression cassette delivered by an AAV vector to a host cell to be expressed by said host cell. Non-limiting examples of proteins encoded by transgenes delivered by AAV vectors include an antibody (e.g. the light and heavy chain of an antibody, an antibody fragment such as a scFv or a nanobody), fluorescent proteins (e.g., for research purposes) or other functional proteins (e.g., for gene therapy in patient with a loss-of-function mutation resulting in a disease-causing defective protein). The transgenes may thus be therapeutic transgenes. The proteins may be therapeutic proteins.

### Invention embodiments

### CNS targeting peptides

According to one aspect of the invention, a central nervous system (CNS) targeting peptide is provided, which comprises or consists of:
a. an amino acid sequence of any one of SEQ ID NOs: 315, 4-314 or 316-436; or
b. at least 4, at least 5, or at least 6 contiguous amino acids of an amino acid sequence of any one of SEQ ID NOs: 315, 4-314 or 316-436; or
c. an amino acid sequence having at least 57%, at least 71%, or at least 85% sequence identity to the amino acid sequence of SEQ ID NOs: 315, 4-314 or 316-436.

In some embodiments, the CNS targeting peptide comprises or consists of an amino acid sequence having at least 57%, at least 71%, or at least 85% sequence identity to an amino acid sequence of any one of SEQ ID NOs: 315, 4-314 or 316-436.

In some embodiments, the CNS targeting peptide comprises or consists of an amino acid sequence having at least 57%, at least 71%, or at least 85% sequence identity to any one of the amino acid sequences YEVGGTL (SEQ ID NO: 7), SHSPEFK (SEQ ID NO: 6), GGLTPPY (SEQ ID NO: 9), DSRLLLP (SEQ ID NO: 5), WTKSSGY (SEQ ID NO: 35), WTTTSGY (SEQ ID NO: 4), SHSPTFR (SEQ ID NO: 60), SNQVASS (SEQ ID NO: 13), GNQVKTL (SEQ ID NO: 16), SNLTGLL (SEQ ID NO: 11), SGNDPSA (SEQ ID NO: 12) or IGNSSKQ (SEQ ID NO: 36).

In some embodiments, the CNS targeting peptide comprises or consists of an amino acid sequence having at least 57%, at least 71%, or at least 85% sequence identity to any one of the amino acid sequences YEVGGTL (SEQ ID NO: 7), SHSPEFK (SEQ ID NO: 6), GGLTPPY (SEQ ID NO: 9) or DSRLLLP (SEQ ID NO: 5). In an embodiment, the CNS targeting peptide comprises or consists of an amino acid sequence having at least 85% sequence identity to any one of the amino acid sequences YEVGGTL (SEQ ID NO: 7), SHSPEFK (SEQ ID NO: 6), GGLTPPY (SEQ ID NO: 9) or DSRLLLP (SEQ ID NO: 5).

In some embodiments, the CNS targeting peptide comprises or consists of an amino acid sequence having at least 57%, at least 71%, or at least 85% sequence identity to the amino acid sequence YEVGGTL (SEQ ID NO: 7). In a preferred embodiment, the CNS targeting peptide comprises or consists of an amino acid sequence having at least 85% sequence identity to the amino acid sequence YEVGGTL (SEQ ID NO: 7).

In some embodiments, the CNS targeting peptide comprises or consists of an amino acid sequence having at least 57%, at least 71%, or at least 85% sequence identity to an amino acid sequence of any one of SEQ ID NOs: 315, 181, 246, 321, 178, 180, 183, 186, 188, 190, 196, 202, 220, 221, 227, 229, 233, 234, 244, 245, 249, 250, 253, 254, 275, 281, 286, 289, 290, 297, 327, 330, 331, 339, 345, 346, 350, 353, 361, 362, 369, 377, 384, 385, 397, 404, 422, 435 or 436.

In some embodiments, the CNS targeting peptide comprises or consists of an amino acid sequence having at least 57%, at least 71%, or at least 85% sequence identity to any one of the amino acid sequences DGNGVKQ (SEQ ID NO: 315), DNEVRSR (SEQ ID NO: 181), SSHNSVK (SEQ ID NO: 246), IGNSVRQ (SEQ ID NO: 220) or ANQTQSR (SEQ ID NO: 321). In an embodiment, the CNS targeting peptide comprises or consists of an amino acid sequence having at least 85% sequence identity to any one of the amino acid sequences DGNGVKQ (SEQ ID NO: 315), DNEVRSR (SEQ ID NO: 181), SSHNSVK (SEQ ID NO: 246), IGNSVRQ (SEQ ID NO: 220) or ANQTQSR (SEQ ID NO: 321).

In some embodiments, the CNS targeting peptide comprises or consists of an amino acid sequence having at least 57%, at least 71%, or at least 85% sequence identity to the amino acid sequence DGNGVKQ (SEQ ID NO: 315). In a preferred embodiment, the CNS targeting peptide comprises or consists of an amino acid sequence having at least 85% sequence identity to the amino acid sequence DGNGVKQ (SEQ ID NO: 315).

Included in the scope of the embodiments of the invention are functional variants of the CNS targeting peptides described herein. The term "functional variant" as used herein refers to a CNS targeting peptide with substantial or significant sequence identity to a parent CNS targeting peptide, which functional variant retains the biological activity of the CNS targeting peptide of which it is a variant.

The term "sequence identity" as used herein refers to the degree of similarity between two sequences of DNA, RNA, or protein. It is typically expressed as a percentage, indicating how many nucleotides or amino acids are identical when two sequences are aligned (using any suitable tool). For example, if two proteins are 100 amino acids long and 90 of those amino acids are identical, the sequence identity would be 90%. Tools to compute the sequence identity of nucleotides or amino acids are known in the art, such as the Basic Local Alignment Search Tool (BLAST) available from: https://blast.ncbi.nlm.nih.gov/Blast.cgi.

In some embodiments, the CNS targeting peptide comprises or consists of at least 4, at least 5, or at least 6 contiguous amino acids of any one of SEQ ID NOs: 315, 4-314 or 316-436.

In some embodiments, the CNS targeting peptide comprises or consists of at least 4, at least 5, or at least 6 contiguous amino acids of any one of the amino acid sequences YEVGGTL (SEQ ID NO: 7), SHSPEFK (SEQ ID NO: 6), GGLTPPY (SEQ ID NO: 9), DSRLLLP (SEQ ID NO: 5), WTKSSGY (SEQ ID NO: 35), WTTTSGY (SEQ ID NO: 4), SHSPTFR (SEQ ID NO: 60), SNQVASS (SEQ ID NO: 13), GNQVKTL (SEQ ID NO: 16), SNLTGLL (SEQ ID NO: 11), SGNDPSA (SEQ ID NO: 12) or IGNSSKQ (SEQ ID NO: 36).

In some embodiments, the CNS targeting peptide comprises or consists of at least 4, at least 5, or at least 6 contiguous amino acids of any one of the amino acid sequences YEVGGTL (SEQ ID NO: 7), SHSPEFK (SEQ ID NO: 6), GGLTPPY (SEQ ID NO: 9) or DSRLLLP (SEQ ID NO: 5). In an embodiment, the CNS targeting peptide comprises or consists of at least 6 contiguous amino acids of any one of the amino acid sequences YEVGGTL (SEQ ID NO: 7), SHSPEFK (SEQ ID NO: 6), GGLTPPY (SEQ ID NO: 9) or DSRLLLP (SEQ ID NO: 5).

In some embodiments, the CNS targeting peptide comprises or consists of at least 4, at least 5, or at least 6 contiguous amino acids of the amino acid sequence YEVGGTL (SEQ ID NO: 7). In a preferred embodiment, the CNS targeting peptide comprises or consists of at least 6 contiguous amino acids of the amino acid sequence YEVGGTL (SEQ ID NO: 7).

In some embodiments, the CNS targeting peptide comprises or consists of at least 4, at least 5, or at least 6 contiguous amino acids of an amino acid sequence of any one of SEQ ID NOs: 315, 181, 246, 321, 178, 180, 183, 186, 188, 190, 196, 202, 220, 221, 227, 229, 233, 234, 244, 245, 249, 250, 253, 254, 275, 281, 286, 289, 290, 297, 327, 330, 331, 339, 345, 346, 350, 353, 361, 362, 369, 377, 384, 385, 397, 404, 422, 435 or 436.

In some embodiments, the CNS targeting peptide comprises or consists of at least 4, at least 5, or at least 6 contiguous amino acids of any one of the amino acid sequences DGNGVKQ (SEQ ID NO: 315), DNEVRSR (SEQ ID NO: 181), SSHNSVK (SEQ ID NO: 246), IGNSVRQ (SEQ ID NO: 220) or ANQTQSR (SEQ ID NO: 321). In an embodiment, the CNS targeting peptide comprises or consists of at least 6 contiguous amino acids of any one of the amino acid sequences DGNGVKQ (SEQ ID NO: 315), DNEVRSR (SEQ ID NO: 181), SSHNSVK (SEQ ID NO: 246), IGNSVRQ (SEQ ID NO: 220) or ANQTQSR (SEQ ID NO: 321).

In some embodiments, the CNS targeting peptide comprises or consists of at least 4, at least 5, or at least 6 contiguous amino acids of the amino acid sequence DGNGVKQ (SEQ ID NO: 315). In a preferred embodiment, the CNS targeting peptide comprises or consists of at least 6 contiguous amino acids of the amino acid sequence DGNGVKQ (SEQ ID NO: 315).

In some embodiments, the CNS targeting peptide comprises or consists of an amino acid sequence of any one of SEQ ID NOs: 315, 4-314 or 316-436.

In some embodiments, the CNS targeting peptide comprises or consists of an amino acid sequence of any one of SEQ ID NOs: 4-177.

In some embodiments, the CNS targeting peptide comprises or consists of an amino acid sequence of any one of SEQ ID NOs: 45 or 178-436.

In some embodiments, the CNS targeting peptide comprises or consists of an amino acid sequence of any one of SEQ ID NOs: 45, 181, 182, 192, 193, 203, 206, 208, 210, 212, 213, 214, 215, 217, 224, 232, 235, 234, 243, 246, 245, 256, 264, 267, 269, 271, 272, 273, 275, 277, 280, 282, 284, 289, 292, 293, 303, 305, 306, 315, 322, 323, 325, 321, 328, 330, 341, 339, 342, 346, 351, 353, 357, 360, 361, 363, 364, 375, 379, 377, 386, 389, 390, 399, 403, 405, 409, 412, 413, 423, 424, 427, 432, 434, 435, 436.

In some embodiments, the CNS targeting peptide comprises or consists of any one of the amino acid sequences YEVGGTL (SEQ ID NO: 7), SHSPEFK (SEQ ID NO: 6), GGLTPPY (SEQ ID NO: 9), DSRLLLP (SEQ ID NO: 5), WTKSSGY (SEQ ID NO: 35), WTTTSGY (SEQ ID NO: 4), SHSPTFR (SEQ ID NO: 60), SNQVASS (SEQ ID NO: 13), GNQVKTL (SEQ ID NO: 16), SNLTGLL (SEQ ID NO: 11), SGNDPSA (SEQ ID NO: 12) or IGNSSKQ (SEQ ID NO: 36).

In some embodiments, the CNS targeting peptide comprises or consists of any one of the amino acid sequences YEVGGTL (SEQ ID NO: 7), SHSPEFK (SEQ ID NO: 6), GGLTPPY (SEQ ID NO: 9) or DSRLLLP (SEQ ID NO: 5). In a preferred embodiment, the CNS targeting peptide comprises or consists of the amino acid sequence YEVGGTL (SEQ ID NO: 7).

In some embodiments, the CNS targeting peptide comprises or consists of an amino acid sequence of any one of SEQ ID NOs: 315, 181, 246, 321, 178, 180, 183, 186, 188, 190, 196, 202, 220, 221, 227, 229, 233, 234, 244, 245, 249, 250, 253, 254, 275, 281, 286, 289, 290, 297, 327, 330, 331, 339, 345, 346, 350, 353, 361, 362, 369, 377, 384, 385, 397, 404, 422, 435 or 436.

In some embodiments, the CNS targeting peptide comprises or consists of any one of the amino acid sequences DGNGVKQ (SEQ ID NO: 315), DNEVRSR (SEQ ID NO: 181), SSHNSVK (SEQ ID NO: 246), IGNSVRQ (SEQ ID NO: 220) or ANQTQSR (SEQ ID NO: 321). In a preferred embodiment, the CNS targeting peptide comprises or consists of the amino acid sequence DGNGVKQ (SEQ ID NO: 315).

In certain embodiments, amino acid sequence variants of a CNS targeting peptide provided herein are contemplated. For example, it may be desirable to modulate the targeting and/or de-targeting capability of the targeting peptide, and/or other biological properties (e.g. modify immune response). Such CNS targeting peptide variants may include, for example, deletions, and/or insertions and/or substitutions of residues within the amino acid sequences of the CNS targeting peptide. Any combination of deletions, insertions, and substitutions can be made, provided that the final CNS targeting peptide possesses the desired characteristics, e.g., directing an agent to the CNS, in particular the agent being an AAV. In some embodiments, the CNS targeting peptide comprises one or more amino acid substitutions, deletions or insertions. In some embodiments, the CNS targeting peptide comprises one or more amino acid substitutions.

In some embodiments, the CNS targeting peptide is a brain and/or spinal cord targeting peptide. In some embodiment, the CNS targeting peptide is a brain and spinal cord targeting peptide. In some embodiments, the CNS targeting peptide de-targets the liver.

In some embodiments the CNS targeting peptide is fused to or comprised within an agent. In some embodiments, the agent is a protein (in particular an antibody, preferably a therapeutic antibody, or an AAV capsid protein), a liposome, a viral vector or a nanoparticle. A preferred agent in the context of the present invention is an adeno-associated virus (AAV).

### AAV capsids and vectors

In one aspect of the invention, an AAV capsid comprising an AAV capsid protein that comprises a CNS targeting peptide as described herein is provided.

The CNS targeting peptide amino acid sequence may either be inserted or substituted (or a combination thereof) in the AAV capsid protein. For the avoidance of doubt, the insertion of a CNS targeting peptide corresponds to the introduction of a CNS targeting peptide amino acid sequence between two contiguous amino acids of the AAV capsid protein sequence without substitution or deletion of any amino acid of the original AAV capsid protein sequence.

Alternatively, the substitution of a CNS targeting peptide in the AAV capsid corresponds to the substitution of a corresponding number of contiguous amino acids of the AAV capsid protein with the CNS targeting peptide amino acid sequence. A combination of insertion and substitution may also be performed to introduce the CNS targeting peptide amino acid sequence in the AAV capsid protein (e.g., if the CNS targeting peptide is a heptamer, the first 3 amino acids of the CNS targeting peptide heptamer may be substituted in an AAV capsid protein, and the following four amino acids of the CNS targeting peptide heptamer inserted contiguously to the first 3 substituted amino acids).

In some embodiments, the AAV capsid protein comprising a CNS targeting peptide as described herein is a wild-type serotype (apart from comprising a CNS targeting peptide of the invention) or an engineered AAV capsid protein (further engineered in addition to comprising a CNS targeting peptide of the invention). Without wishing to be bound by theory, any engineered AAV capsid protein may comprise a CNS targeting peptide of the invention provided the engineered AAV capsid protein displays a suitable tropism to benefit from a CNS targeting peptide of the invention (i.e. an engineered AAV capsid protein that has not been engineered to de-target the CNS). Examples of suitable engineered AAV capsid protein include but are not limited to AAV2-BR1, AAV-S, AAV-F, AAV-PHP.B AAV-PHP.eB, AAV9.PHP.V1, AAV1RX, AAV1R6, AAV1R7, AAV.CAP-B10, AAV.CAP-B22, AAV.CAP-Mac, AAV VCAP-103, AAV bCap 1, AAV-9P31, V-CAP102, AAV-CGN1, AAV-STRV5, AAV-PAL2, AAV-MDV1A, AAV-MDV1B, AAV-MaCPNS1, AAV-MaCPNS2 or AAV VCAP-100.

In some embodiments, the AAV capsid protein comprising a CNS targeting peptide as described herein is a wild-type serotype AAV capsid protein. In some embodiments, the wild-type serotype AAV capsid protein is an AAV1, AAV2, AAV3, AAV4, AAV5, AAV6, AAV7, AAV8, AAV9, AAV10, AAV11, AAV12, AAV13, AAVrh8, AAVrh9, AAVrh10, AAVrh11, AAVrh12, AAVrh13, AAVrh14, AAVrh14, AAVrh15, AAVrh16, AAVrh17, AAVrh39, AAVrh43, AAVrh74, AAVhu11, AAVhu32, AAVhu68, AAVpo1, AAVpo4, AAVpo5, AAVpo6, or AAVpo7 capsid protein.

In some embodiments, the AAV capsid protein comprising a CNS targeting peptide as described herein is an AAVrh10 capsid protein of SEQ ID NO: 1, AAV2 capsid protein of SEQ ID NO: 498, AAV1 capsid protein of SEQ ID NO: 499, AAV4 capsid protein of SEQ ID NO: 502, AAV8 capsid protein of SEQ ID NO: 505, AAV9 capsid protein of SEQ ID NO: 2, or an AAV capsid protein having at least 95%, at least 96%, at least 97%, at least 98% or at least 99% sequence identity to any one of SEQ ID NOs: 1, 2, 498, 499 or 505. In a preferred embodiment, the AAV capsid protein is an AAV9 capsid protein of SEQ ID NO: 2, an AAVrh10 capsid protein of SEQ ID NO: 1, or an AAV capsid protein having at least 95%, at least 96%, at least 97%, at least 98% or at least 99% sequence identity to SEQ ID NO: 1 or 2. In a more preferred embodiment, the AAV capsid protein is an AAVrh10 capsid protein of SEQ ID NO: 1, or an AAV capsid protein having at least 95%, at least 96%, at least 97%, at least 98% or at least 99% sequence identity to SEQ ID NO: 1. In an even more preferred embodiment, the AAV capsid protein is an AAVrh10 capsid protein of SEQ ID NO: 1.

In some embodiments, the CNS targeting peptide of the invention is substituted or inserted (or a combination thereof) in one or more variable regions (VR) of the AAV capsid protein. In some embodiments, the CNS targeting peptide is substituted or inserted (or a combination thereof) in one or more of VR-I, VR-II, VR-III, VR-IV, VR-V, VR-VI, VR-VII, VR-VIII and VR-IX of the AAV capsid protein. In some embodiments, the CNS targeting peptide is substituted or inserted (or a combination thereof) in VR-I, VR-II, VR-III, VR-IV, VR-V, VR-VI, VR-VII, VR-VIII and/or VR-IX of the AAV capsid protein. In some embodiments, the CNS targeting peptide is substituted in VR-IV and/or inserted in VR-VIII of the AAV capsid protein. In some embodiments, the CNS targeting peptide is substituted in VR-IV of the AAV capsid protein. In a preferred embodiment, the CNS targeting peptide is inserted in VR-VIII of the AAV capsid protein.

In some embodiments, the CNS targeting peptide is substituted or inserted (or a combination thereof) in VR-IV or is substituted or inserted (or a combination thereof) in VR-VIII of an AAVrh10 capsid protein of SEQ ID NO: 1 or an AAV capsid protein having at least 95%, at least 96%, at least 97%, at least 98% or at least 99% sequence identity to SEQ ID NO: 1. In some embodiments, the CNS targeting peptide is substituted or inserted (or a combination thereof) in VR-VIII of an AAVrh10 capsid protein of SEQ ID NO: 1 or an AAV capsid protein having at least 95%, at least 96%, at least 97%, at least 98% or at least 99% sequence identity to SEQ ID NO: 1. In a preferred embodiment, the CNS targeting peptide is inserted in VR-VIII of an AAVrh10 capsid protein of SEQ ID NO: 1 or an AAV capsid protein having at least 95%, at least 96%, at least 97%, at least 98% or at least 99% sequence identity to SEQ ID NO: 1. In a more preferred embodiment, the CNS targeting peptide is inserted in VR-VIII between amino acids 589 and 590 of an AAVrh10 capsid protein of SEQ ID NO: 1 or between the corresponding amino acids of an AAV capsid protein having at least 95%, at least 96%, at least 97%, at least 98% or at least 99% sequence identity to SEQ ID NO: 1. In an even more preferred embodiment, the CNS targeting peptide is inserted in VR-VIII between amino acids 589 and 590 of an AAVrh10 capsid protein of SEQ ID NO: 1.

In some embodiments, the AAV capsid protein comprises or consists of an amino acid sequence of any one of SEQ ID NOs: 477, 437-476 or 478-497.

In some embodiments, the AAV capsid protein comprises or consists of the amino acid sequence of any one of SEQ ID NOs: 437-448.

In some embodiments, the AAV capsid protein comprises or consists of the amino acid sequence of any one of SEQ ID NOs: 438-441.

In some embodiments, the AAV capsid protein comprises or consists of the amino acid sequence of SEQ ID NO: 440.

In some embodiments, the AAV capsid protein comprises or consists of the amino acid sequence of any one of SEQ ID NOs: 477, 449-476 or 478-497.

In some embodiments, the AAV capsid protein comprises or consists of the amino acid sequence of any one of SEQ ID NOs: 477, 451, 466, 458 or 478.

In some embodiments, the AAV capsid protein comprises or consists of the amino acid sequence of SEQ ID NO: 477.

In some embodiments, an AAV capsid protein comprising a CNS targeting peptide of the invention comprises one or more amino acid insertions, substitutions or deletions, wherein the one or more insertions, substitutions or deletions are not within the CNS targeting peptide. In some embodiments, the one or more amino acid insertions, substitutions or deletions alter the tropism, improve the transduction efficiency, modify the immune response, improve the production efficiency and/or enhance the stability of an AAV vector comprising the AAV capsid protein. The term "modify immune response" includes but is not limited to reducing the immunogenicity of the AAV capsid protein and improving escaping neutralizing antibody. Without wishing to be bound by theory, pre-existing immunity to AAV in human, particularly neutralizing antibodies, can hinder the effectiveness of AAV-based gene therapies (Boutin et al. 2010; Verdera et al. 2020). A large portion of the human population has been exposed to wild-type AAVs, leading to the presence of anti-AAV neutralizing antibodies (Boutin et al. 2010; Thwaite R et al. 2014) that can neutralize the viral vectors before they reach their target cells, reducing the efficiency of gene delivery. In some embodiments, the AAV capsid protein is an AAVrh10 comprising a CNS targeting peptide of the invention, wherein the AAV capsid protein comprises one or more substitutions selected from: Q589, N590, A592 or S691 with reference to SEQ ID NO: 1.

In another aspect of the invention, AAV vectors comprising an AAV capsid protein as described herein are provided.

In some embodiments, the AAV vector (comprising an AAV capsid protein as described herein) comprises an AAV genome comprising an expression cassette flanked by inverted terminal repeats (ITRs). Accordingly, the AAV genome may comprise a 5'ITR and a 3'ITR flanking the expression cassette. In some embodiments, the 5' ITR and 3' ITR are from the AAV2 wild type serotype.

In some embodiments, the expression cassette comprises from 5' to 3': a promoter and one or more transgenes. In some embodiments, the promoter is a ubiquitous or a CNS-specific promoter. In some embodiments, the promoter is a ubiquitous promoter and the promoter is a CMV, EF1A, CAG, CBA, CBh or SV40 promoter. In a preferred embodiment, the promoter is a CBh or CAG promoter.

In some embodiments, the promoter is a CNS-specific promoter and the promoter is a gfaABC(1)D, CMVe-GFAP, GFAP, ATP1A2, CLDN5, ADRB2, TNFRSF6B, PDYN, GH1, OPALIN, SYN1, CMVe-SYN1, CAMK2A, NEFH, NEUROD6, NSE, PGK, OLIG2 or CMVe-OLIG2 promoter. In a preferred embodiment, the promoter is a SYN1 or gfaABC(1)D promoter. The CNS-specific promoters described herein may be from any mammal. In some embodiments, the CNS-specific promoter is a mouse or a human promoter. In a more preferred embodiment, the CNS-specific promoter is a human SYN1 (hSYN1) or gfaABC(1)D promoter. In some embodiments, the promoter is a CAG, CBh, hSYN1 or gfaABC(1)D promoter. In some embodiments, the promoter may be operably linked to an enhancer or to one or more intron sequences. In an embodiment, the enhancer is a CMV early enhancer.

In some embodiments, the expression cassette comprises one or more further regulatory elements. In some embodiments, the one or more further regulatory elements is a 5'UTR, a 3'UTR, an intron, an IRES, a WPRE or a miRNA binding site. The one or more 5'UTRs, 3'UTRs or introns may be from any gene and do not necessarily have to correspond to the 5' UTRs, 3'UTRs or introns of the gene(s) selected as transgene in the AAV vector.

In some embodiments, the expression cassette comprises from 5' to 3': a promoter, one or more transgenes and a WPRE.

In some embodiments, the expression cassette comprises from 5' to 3': a promoter, one or more transgenes, one or more miRNA binding sites and a WPRE. Without wishing to be bound by theory, microRNA (miRNA or miR) binding sites (BS) play a significant role in regulating gene expression, and may be used in vector delivery systems to regulate transgene expression. These binding sites are specific sequences in the messenger RNA (mRNA) that miRNAs can attach to, leading to the repression of gene expression. In the context of vector delivery (such as AAV vector delivery), incorporating miRNA binding sites into the vector design allows for targeted regulation of transgene expression. For example, by including miRNA binding sites that are recognized by miRNAs highly expressed in liver cells, transgene expression in liver cells may be reduced or prevented. Such a strategy may be particularly useful in reducing or preventing transgene expression in off-target tissue, improving therapeutic efficacy and safety of vector delivery system.

In some embodiments, one or more miRNA binding sites reduce or prevent the expression of the one or more transgenes in liver cells, cardiac muscle cells, skeletal muscle cells and/or hematopoietic cells (wherein the reduction or prevention of the expression of the one or more transgenes is relative to a corresponding expression cassette without the one or more miRNA binding sites).

In some embodiments, the one or more miRNA binding site is a miR-122 binding site, a miR-1 binding site and/or a miR-142 binding site. In some embodiments, the expression cassette comprises from 3 to 9 miRNA binding sites.

In some embodiments, an AAV vector as described herein is capable of transducing cells of the CNS. In some embodiments, the AAV vector is capable of transducing cells of the brain and/or spinal cord. In some embodiments, the AAV vector is capable of transducing cells of the brain and the spinal cord.

In some embodiments, the AAV vector exhibits, or is capable of, an increased transduction of cells of the CNS, wherein the increase in transduction of cells of the CNS is relative to a corresponding (i.e. otherwise identical) AAV vector comprising an AAV capsid protein which does not comprise the CNS targeting peptide. Suitable methods for testing transduction are known in the art and exemplified herein.

In some embodiments, the AAV vector exhibits, or is capable of, a decreased transduction of cells of the liver, wherein the decrease in transduction of cells of the liver is relative to a corresponding (i.e. otherwise identical) AAV vector comprising an AAV capsid protein which does not comprise the CNS targeting peptide. Suitable methods for testing transduction are known in the art and exemplified herein.

In some embodiments, the AAV vector exhibits an increased transduction of cells of the CNS and a decreased transduction of cells of the liver, wherein the increase in transduction of cells of the CNS and the decrease in transduction of cells of the liver are relative to a corresponding (i.e. otherwise identical) AAV vector comprising an AAV capsid protein which does not comprise the CNS targeting peptide. Suitable methods for testing transduction are known in the art and exemplified herein.

In some embodiments, the AAV vector exhibits, or is capable of, an increased transduction of cells of the CNS and a decreased transduction of cells of one or more of the liver, kidney, muscle, spleen or heart, wherein the increase in transduction of cells of the CNS and the decrease in transduction of cells of one or more of the liver, kidney, muscle, spleen or heart are relative to a corresponding (i.e. otherwise identical) AAV vector comprising an AAV capsid protein which does not comprise the CNS targeting peptide. Suitable methods for testing transduction are known in the art and exemplified herein.

In some embodiments, the increased transduction of cells of the CNS is an increased transduction of cells of the brain and/or spinal cord. Preferably, the increased transduction of cells of the CNS is an increased transduction of cells of the brain and spinal cord. In some embodiments, the increased transduction of cells of the brain and/or spinal cord is at least 2-fold, at least 5-fold, at least 10-fold, at least 50-fold, at least 100-fold, at least 200-fold, at least 300-fold, at least 400-fold or at least 500-fold.

In some embodiments, the decreased transduction of cells of the liver is at least 2-fold, at least 5-fold, at least 10-fold, at least 20-fold, at least 50-fold or at least 100-fold.

In some embodiments, the decreased transduction of cells of one or more of the liver, kidney, muscle, spleen or heart is at least 2-fold, at least 5-fold, at least 10-fold or at least 100-fold.

In some embodiments, the increased transduction of cells of the brain and/or spinal cord (preferably, the brain and spinal cord) is at least 50-fold and the decreased transduction of cells of the liver is at least 5-fold.

As used herein, "a corresponding AAV vector comprising an AAV capsid protein which does not comprise the CNS targeting peptide" refers to an otherwise identical AAV vector apart from the absence of the CNS targeting peptide.

In some embodiments, the AAV vector comprising an AAV capsid protein which does not comprise a CNS targeting peptide of the invention is an AAVrh10 capsid protein of SEQ ID NO: 1.

In some embodiments, the AAV vector exhibits, or is capable of, an increased transduction of cells of the brain of at least 300-fold, an increased transduction of cells of the spinal cord of at least 500-fold, and a decreased transduction of cells of the liver of at least 5-fold, wherein the AAV vector comprises an AVV capsid protein of SEQ ID NO: 440 and wherein the increase in transduction of cells of the brain and spinal cord, and the decrease in transduction of cells of the liver are relative to an AAV vector comprising an AAVrh10 capsid protein of SEQ ID NO: 1.

In some embodiments, the AAV vector exhibits, or is capable of, an increased transduction of cells of the brain of at least 200-fold, an increased transduction of cells of the spinal cord of at least 200-fold, and a decreased transduction of cells of the liver of at least 5-fold, wherein the AAV vector comprises an AVV capsid protein of SEQ ID NO: 439 and wherein the increase in transduction of cells of the brain and spinal cord, and the decrease in transduction of cells of the liver are relative to an AAV vector comprising an AAVrh10 capsid protein of SEQ ID NO: 1.

In some embodiments, the AAV vector exhibits, or is capable of, an increased transduction of cells of the brain of at least 100-fold, an increased transduction of cells of the spinal cord of at least 200-fold, and a decreased transduction of cells of the liver of at least 5-fold, wherein the AAV vector comprises an AVV capsid protein of SEQ ID NO: 441 and wherein the increase in transduction of cells of the brain and spinal cord, and the decrease in transduction of cells of the liver are relative to an AAV vector comprising an AAVrh10 capsid protein of SEQ ID NO: 1.

In some embodiments, the AAV vector exhibits, or is capable of, an increased transduction of cells of the brain of at least 50-fold, an increased transduction of cells of the spinal cord of at least 100-fold, and a decreased transduction of cells of the liver of at least 20-fold, wherein the AAV vector comprises an AVV capsid protein of SEQ ID NO: 438 and wherein the increase in transduction of cells of the brain and spinal cord, and the decrease in transduction of cells of the liver are relative to an AAV vector comprising an AAVrh10 capsid protein of SEQ ID NO: 1. For the avoidance of doubt, it is understood that the increased transduction of cells of the CNS, in particular of cells of the brain and/or spinal cord, and/or the decreased transduction of cells of one or more of the liver, kidney, muscle, spleen or heart, may be *in vitro* or *in vivo.* In some embodiments, the increased transduction of cells of the CNS, in particular of cells of the brain and/or spinal cord (preferably brain and spinal cord), and/or the decreased transduction of cells of one or more of the liver, kidney, muscle, spleen or heart is *in vivo* in a subject, wherein the AAV vector is administered to the subject. In some embodiments, the AAV vector is administered to the subject systemically. In a preferred embodiment, the AAV vector is administered to a subject intravenously.

In some embodiments, the subject is a mammal. In some embodiments, the subject is a rodent or a primate. In a preferred embodiment, the subject is a mouse, a non-human primate or a human. In a more preferred embodiment, the subject is a human.

In another aspect of the invention, a pharmaceutical composition comprising a CNS targeting peptide, an AAV capsid protein and/or an AAV vector as described herein, and a pharmaceutically acceptable carrier, adjuvant and/or excipient, is provided.

In another aspect of the invention, a nucleic acid encoding a CNS targeting peptide, an AAV capsid protein and/or an AAV vector as described herein is provided.

In a further aspect of the invention, a method of producing an AAV vector is provided, the method comprising introducing into a host cell: (a) a nucleic acid encoding a recombinant viral genome comprising a replication (Rep) gene and a capsid (Cap) gene flanked by inverted terminal repeats (ITRs), wherein the capsid (Cap) gene encodes an AAV capsid protein as described herein; (b) a nucleic acid comprising an expression cassette flanked by inverted terminal repeats (ITRs); and (c) a nucleic acid comprising an AAV helper virus genome;
and isolating the AAV vector.

In some embodiments, each of the nucleic acids described in (a)-(c) is in the form of a plasmid. In some embodiments, the host cell is a HEK293T cell. Accordingly, as described in the Examples below, an AAV vector may be produced by co-transfection of suitable host cells, such as HEK293T cells, with the nucleic acids described in (a)-(c) each in the form of a plasmid (i.e. a Rep-Cap plasmid, an AAV plasmid, and a helper plasmid, respectively). The transfected cells can be cultured and viral particles produced. The viral particles may be isolated (i.e. purified) from the host cells, for example, by caesium chloride (CsCl) gradient ultra-centrifugation.

In yet another aspect, the invention provides an AAV vector or pharmaceutical composition as described herein for use in human or veterinary medicine. In some embodiments, the AAV vector or the pharmaceutical composition is for use in the prevention, alleviation and/or treatment of a disease, disorder and/or conditions of the CNS. In a similar aspect, the use of an AAV vector or pharmaceutical composition as described herein for the manufacture of a medicament for the prevention, alleviation and/or treatment of a disease, disorder and/or conditions of the CNS is provided. In a further similar aspect, a method for preventing, alleviating and/or treating a CNS disease, disorder and/or condition in a subject in need thereof is provided, the method comprising administering a therapeutically effective amount of an AAV vector or a pharmaceutical composition (as described herein) to the subject.

The following embodiments are equally applicable to the AAV vector or pharmaceutical composition for use in the prevention, alleviation and/or treatment of a disease, disorder and/or conditions of the CNS, the method for preventing, alleviating and/or treating a CNS disease, disorder and/or condition in a subject in need thereof, and the use of the AAV vector or pharmaceutical composition for the manufacture of a medicament.

Typically, the AAV vector delivers a transgene, which may thus be a therapeutic transgene. The transgene may encode a therapeutic protein. The pharmaceutical composition comprising the CNS targeting peptide of the invention may deliver any suitable therapeutic agent (as described herein).

The AAV vectors or pharmaceutical composition described herein can be administered to a subject by any conventional route, including injection or by gradual infusion over time. The administration may be via parenteral administration. The administration may, for example, be by infusion or by intravenous, intraperitoneal, intranasal, intravitreous, subcutaneous, intramuscular, intrathecal, intracisternal, intraparenchymal, intrastriatal or intracerebroventricular route. As further examples, suitable forms for parenteral injection (including, subcutaneous, intramuscular, intravascular or infusion) include a sterile solution, suspension or emulsion. In some embodiments, the AAV vector or pharmaceutical composition is administered via intravenous, intraperitoneal, intranasal, intravitreous, subcutaneous, intramuscular, intrathecal, intracisternal, intraparenchymal, intrastriatal or intracerebroventricular route. In a preferred embodiment, the AAV vector or pharmaceutical composition is administered via intravenous route.

Pharmaceutically acceptable carriers, diluents, adjuvants and excipients are well known in the pharmaceutical art and are described, for example, in Remington's Pharmaceutical Sciences, 15th or 18th Ed. (Alfonso R. Gennaro, ed.; Mack Publishing Company, Easton, PA, 1990); Remington: the Science and Practice of Pharmacy 19th Ed. (Lippincott, Williams & Wilkins, 1995); Handbook of Pharmaceutical Excipients, 3rd Ed. (Arthur H. Kibbe, ed.; Amer. Pharmaceutical Assoc, 1999); Pharmaceutical Codex: Principles and Practice of Pharmaceutics 12th Ed. (Walter Lund ed.; Pharmaceutical Press, London, 1994); The United States Pharmacopeia: The National Formulary (United States Pharmacopeial Convention); Fiedler's "Lexikon der Hilfstoffe" 5th Ed., Edition Cantor Verlag Aulendorf 2002; "The Handbook of Pharmaceutical Excipients", 4th Ed., American Pharmaceuticals Association, 2003; and Goodman and Gilman's: the Pharmacological Basis of Therapeutics (Louis S. Goodman and Lee E. Limbird, eds.; McGraw Hill, 1992), the disclosures of which are hereby incorporated by reference.

The carriers, diluents, adjuvants and pharmaceutical excipients can be selected with regard to the intended route of administration and standard pharmaceutical practice. These compounds must be acceptable in the sense of being not deleterious to the recipient thereof. See Remington's Pharmaceutical Sciences, 15th or 18th Ed. (Alfonso R. Gennaro, ed.; Mack Publishing Company, Easton, PA, 1990); Remington: the Science and Practice of Pharmacy 19th Ed.(Lippincott, Williams & Wilkins, 1995); Handbook of Pharmaceutical Excipients, 3rd Ed. (Arthur H. Kibbe, ed.; Amer. Pharmaceutical Assoc, 1999); Pharmaceutical Codex: Principles and Practice of Pharmaceutics 12th Ed. (Walter Lund ed.; Pharmaceutical Press, London, 1994); The United States Pharmacopeia: The National Formulary (United States Pharmacopeial Convention); Fiedler's "Lexikon der Hilfstoffe" 5th Ed., Edition Cantor Verlag Aulendorf 2002; "The Handbook of Pharmaceutical Excipients", 4th Ed., American Pharmaceuticals Association, 2003; and Goodman and Gilman's: the Pharmacological Basis of Therapeutics (Louis S. Goodman and Lee E. Limbird, eds.; McGraw Hill, 1992), the disclosures of which are hereby incorporated by reference. Carriers, diluents, adjuvants and pharmaceutical excipients for AAV formulations are known in the art, for example as described and referenced in Chan et al. 2022.

In another aspect of the invention, a CNS targeting peptide of the invention, an AAV capsid protein of the invention, or an AAV vector of the invention is for use as a research tool.

### Numbered embodiments

The invention will now be described by way of numbered embodiments.
1. A central nervous system (CNS) targeting peptide comprising or consisting of:
   a. an amino acid sequence of any one of SEQ ID NOs: 315, 4-314 or 316-436; or
   b. at least 4, at least 5, or at least 6 contiguous amino acids of an amino acid sequence of any one of SEQ ID NOs: 315, 4-314 or 316-436; or
   c. an amino acid sequence having at least 57%, at least 71%, or at least 85% sequence identity to the amino acid sequence of SEQ ID NOs: 315, 4-314 or 316-436.
2. The CNS targeting peptide of embodiment 1, which comprises or consists of an amino acid sequence having at least 57%, at least 71%, or at least 85% sequence identity to an amino acid sequence of any one of SEQ ID NOs: 315, 4-314 or 316-436.
3. The CNS targeting peptide of embodiment 1 or 2, which comprises or consists of an amino acid sequence having at least 57%, at least 71%, or at least 85% sequence identity to an amino acid sequence of any one of SEQ ID NOs: 315, 181, 246, 321, 178, 180, 183, 186, 188, 190, 196, 202, 220, 221, 227, 229, 233, 234, 244, 245, 249, 250, 253, 254, 275, 281, 286, 289, 290, 297, 327, 330, 331, 339, 345, 346, 350, 353, 361, 362, 369, 377, 384, 385, 397, 404, 422, 435 or 436.
4. The CNS targeting peptide of any one of the preceding embodiments, which comprises or consists of an amino acid sequence having at least 57%, at least 71%, or at least 85% sequence identity to any one of the amino acid sequences DGNGVKQ (SEQ ID NO: 315), DNEVRSR (SEQ ID NO: 181), SSHNSVK (SEQ ID NO: 246), IGNSVRQ (SEQ ID NO: 220) or ANQTQSR (SEQ ID NO: 321).
5. The CNS targeting peptide of any one of the preceding embodiments, which comprises or consists of an amino acid sequence having at least 57%, at least 71%, or at least 85% sequence identity to the amino acid sequence DGNGVKQ (SEQ ID NO: 315).
6. The CNS targeting peptide of embodiment 1 or 2, which comprises or consists of an amino acid sequence having at least 57%, at least 71%, or at least 85% sequence identity to any one of the amino acid sequences YEVGGTL (SEQ ID NO: 7), SHSPEFK (SEQ ID NO: 6), GGLTPPY (SEQ ID NO: 9), DSRLLLP (SEQ ID NO: 5), WTKSSGY (SEQ ID NO: 35), WTTTSGY (SEQ ID NO: 4), SHSPTFR (SEQ ID NO: 60), SNQVASS (SEQ ID NO: 13), GNQVKTL (SEQ ID NO: 16), SNLTGLL (SEQ ID NO: 11), SGNDPSA (SEQ ID NO: 12) or IGNSSKQ (SEQ ID NO: 36).
7. The CNS targeting peptide of any one of embodiments 1, 2 or 6, which comprises or consists of an amino acid sequence having at least 57%, at least 71%, or at least 85% sequence identity to any one of the amino acid sequences YEVGGTL (SEQ ID NO: 7), SHSPEFK (SEQ ID NO: 6), GGLTPPY (SEQ ID NO: 9) or DSRLLLP (SEQ ID NO: 5).
8. The CNS targeting peptide of any one of embodiments 1, 2, 6 or 7, which comprises or consists of an amino acid sequence having at least 57%, at least 71%, or at least 85% sequence identity to the amino acid sequence YEVGGTL (SEQ ID NO: 7).
9. The CNS targeting peptide of embodiment 1 or 2, which comprises or consists of at least 4, at least 5, or at least 6 contiguous amino acids of any one of SEQ ID NOs: 315, 4-314 or 316-436.
10. The CNS targeting peptide of any one of embodiments 1 to 3 or 9, which comprises or consists of at least 4, at least 5, or at least 6 contiguous amino acids of an amino acid sequence of any one of SEQ ID NOs: 315, 181, 246, 321, 178, 180, 183, 186, 188, 190, 196, 202, 220, 221, 227, 229, 233, 234, 244, 245, 249, 250, 253, 254, 275, 281, 286, 289, 290, 297, 327, 330, 331, 339, 345, 346, 350, 353, 361, 362, 369, 377, 384, 385, 397, 404, 422, 435 or 436.
11. The CNS targeting peptide of any one of embodiments 1 to 4, 9 or 10, which comprises or consists of at least 4, at least 5, or at least 6 contiguous amino acids of any one of the amino acid sequences DGNGVKQ (SEQ ID NO: 315), DNEVRSR (SEQ ID NO: 181), SSHNSVK (SEQ ID NO: 246), IGNSVRQ (SEQ ID NO: 220) or ANQTQSR (SEQ ID NO: 321).
12. The CNS targeting peptide of any one of embodiments 1 to 5 or 9 to 11, which comprises or consists of at least 4, at least 5, or at least 6 contiguous amino acids of the amino acid sequence DGNGVKQ (SEQ ID NO: 315).
13. The CNS targeting peptide of any one of embodiments 1, 2, 6 or 9, which comprises or consists of at least 4, at least 5, or at least 6 contiguous amino acids of any one of the amino acid sequences YEVGGTL (SEQ ID NO: 7), SHSPEFK (SEQ ID NO: 6), GGLTPPY (SEQ ID NO: 9), DSRLLLP (SEQ ID NO: 5), WTKSSGY (SEQ ID NO: 35), WTTTSGY (SEQ ID NO: 4), SHSPTFR (SEQ ID NO: 60), SNQVASS (SEQ ID NO: 13), GNQVKTL (SEQ ID NO: 16), SNLTGLL (SEQ ID NO: 11), SGNDPSA (SEQ ID NO: 12) or IGNSSKQ (SEQ ID NO: 36).
14. The CNS targeting peptide of any one of embodiments 1, 2, 6, 7, 9 or 13, which comprises or consists of at least 4, at least 5, or at least 6 contiguous amino acids of any one of the amino acid sequences YEVGGTL (SEQ ID NO: 7), SHSPEFK (SEQ ID NO: 6), GGLTPPY (SEQ ID NO: 9) or DSRLLLP (SEQ ID NO: 5).
15. The CNS targeting peptide of any one of embodiments 1, 2, 6 to 9, 13 or 14, which comprises or consists of at least 4, at least 5, or at least 6 contiguous amino acids of the amino acid sequence YEVGGTL (SEQ ID NO: 7).
16. The CNS targeting peptide of any one of embodiments 1, 2 or 9, which comprises or consists of the amino acid sequence of any one of SEQ ID NOs: 315, 4-314 or 316-436.
17. The CNS targeting peptide of any one of embodiments 1 to 3, 9, 10 or 16, which comprises or consists of an amino acid sequence of any one of SEQ ID NOs: 315, 181, 246, 321, 178, 180, 183, 186, 188, 190, 196, 202, 220, 221, 227, 229, 233, 234, 244, 245, 249, 250, 253, 254, 275, 281, 286, 289, 290, 297, 327, 330, 331, 339, 345, 346, 350, 353, 361, 362, 369, 377, 384, 385, 397, 404, 422, 435 or 436.
18. The CNS targeting peptide of any one of embodiments 1 to 4, 9 to 11, 16 or 17, which comprises or consists of any one of the amino acid sequences DGNGVKQ (SEQ ID NO: 315), DNEVRSR (SEQ ID NO: 181), SSHNSVK (SEQ ID NO: 246), IGNSVRQ (SEQ ID NO: 220) or ANQTQSR (SEQ ID NO: 321).
19. The CNS targeting peptide of any one of embodiments 1 to 5, 9 to 12 or 16 to 18, which comprises or consists of the amino acid sequence DGNGVKQ (SEQ ID NO: 315).
20. The CNS targeting peptide of any one of embodiments 1, 2, 6, 9, 13 or 16, which comprises or consists of any one of the amino acid sequences YEVGGTL (SEQ ID NO: 7), SHSPEFK (SEQ ID NO: 6), GGLTPPY (SEQ ID NO: 9), DSRLLLP (SEQ ID NO: 5), WTKSSGY (SEQ ID NO: 35), WTTTSGY (SEQ ID NO: 4), SHSPTFR (SEQ ID NO: 60), SNQVASS (SEQ ID NO: 13), GNQVKTL (SEQ ID NO: 16), SNLTGLL (SEQ ID NO: 11), SGNDPSA (SEQ ID NO: 12) or IGNSSKQ (SEQ ID NO: 36).
21. The CNS targeting peptide of any one of embodiments 1, 2, 6, 7, 9, 13, 14, 16 or 20, which comprises or consists of any one of the amino acid sequences YEVGGTL (SEQ ID NO: 7), SHSPEFK (SEQ ID NO: 6), GGLTPPY (SEQ ID NO: 9) or DSRLLLP (SEQ ID NO: 5).
22. The CNS targeting peptide of any one of embodiments 1, 2, 6 to 9, 13 to 16, 20 or 21, which comprises or consists of the amino acid sequence YEVGGTL (SEQ ID NO: 7).
23. The CNS targeting peptide of any one of the preceding embodiments, which is a brain and/or spinal cord targeting peptide.
24. The CNS targeting peptide of any one of the preceding embodiments, which de-targets the liver.
25. An AAV capsid protein or an AAV capsid comprising an AAV capsid protein, wherein the AAV capsid protein comprises the CNS targeting peptide of any one of the preceding embodiments.
26. The AAV capsid protein or AAV capsid of embodiment 25, wherein the AAV capsid protein is an AAVrh10 capsid protein of SEQ ID NO: 1, AAV2 capsid protein of SEQ ID NO: 498, AAV1 capsid protein of SEQ ID NO: 499, AAV4 capsid protein of SEQ ID NO: 502, AAV8 capsid protein of SEQ ID NO: 505, AAV9 capsid protein of SEQ ID NO: 2, or an AAV capsid protein having at least 95%, at least 96%, at least 97%, at least 98% or at least 99% sequence identity to any one of SEQ ID NOs: 1, 2, 498, 499 or 505.
27. The AAV capsid protein or AAV capsid of embodiment 25 or 26, wherein the AAV capsid protein is an AAVrh10 capsid protein of SEQ ID NO: 1 or an AAV capsid protein having at least 95%, at least 96%, at least 97%, at least 98% or at least 99% sequence identity to SEQ ID NO: 1.
28. The AAV capsid protein or AAV capsid of any one of embodiments 25 to 27, wherein the CNS targeting peptide is substituted or inserted, or a combination thereof, in one or more variable regions (VR) of the AAV capsid protein.
29. The AAV capsid protein or AAV capsid of any one of embodiments 25 to 28, wherein the CNS targeting peptide is substituted or inserted, or a combination thereof, in one or more of VR-I, VR-II, VR-III, VR-IV, VR-V, VR-VI, VR-VII, VR-VIII and VR-IX of the AAV capsid protein.
30. The AAV capsid protein or AAV capsid of any one of embodiments 25 to 29, wherein the AAV capsid protein is an AAVrh10 capsid protein of SEQ ID NO: 1 or an AAV capsid protein having at least 95%, at least 96%, at least 97%, at least 98% or at least 99% sequence identity to SEQ ID NO: 1, and wherein the CNS targeting peptide is inserted in VR-VIII of the AAV capsid protein.
31. The AAV capsid protein or AAV capsid of embodiment 30, wherein the CNS targeting peptide is inserted between amino acids 589 and 590 of the AAV capsid protein.
32. The AAV capsid protein or AAV capsid of any one of embodiments 25 to 31, wherein the AAV capsid protein comprises or consists of an amino acid sequence of any one of SEQ ID NOs: 477, 437-476 or 478-497.
33. The AAV capsid protein or AAV capsid of any one of embodiments 25 to 32, wherein the AAV capsid protein comprises or consists of the amino acid sequence of any one of SEQ ID NOs: 437-448.
34. The AAV capsid protein or AAV capsid of any one of embodiments 25 to 33, wherein the AAV capsid protein comprises or consists of the amino acid sequence of any one of SEQ ID NOs: 438-441.
35. The AAV capsid protein or AAV capsid of any one of embodiments 25 to 34, wherein the AAV capsid protein comprises or consists of the amino acid sequence of SEQ ID NO: 440.
36. The AAV capsid protein or AAV capsid of any one of embodiments 25 to 32, wherein the AAV capsid protein comprises or consists of the amino acid sequence of any one of SEQ ID NOs: 477, 449-476 or 478-497.
37. The AAV capsid protein or AAV capsid of any one of embodiments 25 to 32 or 36, wherein the AAV capsid protein comprises or consists of the amino acid sequence of any one of SEQ ID NOs: 477, 451, 466, 458 or 478.
38. The AAV capsid protein or AAV capsid of any one of embodiments 25 to 32, 36 or 37, wherein the AAV capsid protein comprises or consists of the amino acid sequence of SEQ ID NO: 477.
39. An AAV vector, which comprises the AAV capsid protein or AAV capsid of any one of embodiments 25 to 38 and an AAV genome comprising an expression cassette flanked by inverted terminal repeats (ITRs).
40. The AAV vector of embodiment 39, wherein the expression cassette comprises from 5' to 3': a promoter and one or more transgenes.
41. The AAV vector of embodiment 40, wherein the promoter is a ubiquitous or a CNS-specific promoter.
42. The AAV vector of embodiment 40 or 41, wherein the promoter is a CMV, EF1A, CAG, CBA, CBh or SV40 promoter.
43. The AAV vector of any one of embodiments 40 to 42, wherein the promoter is a CBh or CAG promoter.
44. The AAV vector of embodiment 40 or 41, wherein the promoter is a gfaABC(1)D, CMVe-GFAP, GFAP, ATP1A2, CLDN5, ADRB2, TNFRSF6B, PDYN, GH1, OPALIN, SYN1, CMVe-SYN1, CAMK2A, NEFH, NEUROD6, NSE, PGK, OLIG2 or CMVe-OLIG2 promoter.
45. The AAV vector of any one of embodiments 40, 41 or 44, wherein the promoter is a SYN1 or gfaABC(1)D promoter, preferably a human SYN1 or gfaABC(1)D promoter.
46. The AAV vector of any one of embodiments 40 to 45, wherein the expression cassette comprises one or more further regulatory elements.
47. The AAV vector of embodiment 46, wherein the one or more further regulatory elements is a 5'UTR, a 3'UTR, an intron, an IRES, a WPRE or a miRNA binding site.
48. The AAV vector of any one of embodiments 40 to 47, wherein the expression cassette comprises from 5' to 3': a promoter, one or more transgenes and a WPRE.
49. The AAV vector of any one of embodiments 40 to 47, wherein the expression cassette comprises from 5' to 3': a promoter, one or more transgenes, one or more miRNA binding sites and a WPRE.
50. The AAV vector of embodiment 47 or 49, wherein the one or more miRNA binding site is a miR-122 binding site, a miR-1 binding site and/or a miR-142 binding site.
51. The AAV vector of any one of embodiments 47, 49 or 50, wherein the expression cassette comprises from 3 to 9 miRNA binding sites.
52. The AAV vector of any one of embodiments 39 to 51, which is capable of transducing cells of the CNS.
53. The AAV vector of any one of embodiments 39 to 52, which is capable of transducing cells of the brain and/or spinal cord.
54. The AAV vector of any one of embodiments 39 to 53, which exhibits an increased transduction of cells of the CNS and a decreased transduction of cells of the liver, wherein the increase in transduction of cells of the CNS and the decrease in transduction of cells of the liver are relative to a corresponding AAV vector comprising an AAV capsid protein which does not comprise the CNS targeting peptide.
55. The AAV vector of any one of embodiments 39 to 54, which exhibits an increased transduction of cells of the CNS and a decreased transduction of cells of one or more of the liver, kidney, muscle, spleen or heart, wherein the increase in transduction of cells of the CNS and the decrease in transduction of cells of one or more of the liver, kidney, muscle, spleen or heart are relative to a corresponding AAV vector comprising an AAV capsid protein which does not comprise the CNS targeting peptide.
56. The AAV vector of embodiment 54 or 55, wherein the increased transduction of cells of the CNS is an increased transduction of cells of the brain and/or spinal cord.
57. The AAV vector of embodiment 56, wherein the increased transduction of cells of the brain and/or spinal cord is at least 2-fold, at least 5-fold, at least 10-fold, at least 50-fold, at least 100-fold, at least 200-fold, at least 300-fold, at least 400-fold or at least 500-fold.
58. The AAV vector of any one of embodiments 54 to 57, wherein the decreased transduction of cells of the liver is at least 2-fold, at least 5-fold, at least 10-fold, at least 20-fold, at least 50-fold or at least 100-fold.
59. The AAV vector of embodiment 55, wherein the decreased transduction of cells of one or more of the liver, kidney, muscle, spleen or heart is at least 2-fold, at least 5-fold, at least 10-fold or at least 100-fold.
60. The AAV vector of any one of embodiments 54 to 59, wherein the AAV vector comprises the AAV capsid protein of embodiment 31, and the corresponding AAV vector comprising an AAV capsid protein which does not comprise the CNS targeting peptide is an AAVrh10 capsid protein of SEQ ID NO: 1.
61. A pharmaceutical composition comprising the CNS targeting peptide of any one of embodiments 1 to 24, the AAV capsid protein or AAV capsid of any one of embodiments 25 to 38, or the AAV vector of any one of embodiments 39 to 60 and a pharmaceutically acceptable carrier, adjuvant and/or excipient.
62. A nucleic acid encoding the CNS targeting peptide of any one of embodiments 1 to 24, the AAV capsid protein or AAV capsid of any one of embodiments 25 to 38, or the AAV vector of any one of embodiments 39 to 60.
63. A method of producing an AAV vector, the method comprising introducing into a host cell:
   a. a nucleic acid encoding a recombinant viral genome comprising a replication (Rep) gene and a capsid (Cap) gene flanked by inverted terminal repeats (ITRs), wherein the capsid (Cap) gene encodes an AAV capsid protein of any one of embodiments 25 to 38;
   b. a nucleic acid comprising an expression cassette flanked by inverted terminal repeats (ITRs); and
   c. a nucleic acid comprising an AAV helper virus genome;
   and isolating the AAV vector.
64. The AAV vector of any one of embodiments 39 to 60 or the pharmaceutical composition of embodiment 61 for use in human or veterinary medicine.
65. The AAV vector of any one of embodiments 39 to 60 or the pharmaceutical composition of embodiment 61 for use in the prevention, alleviation and/or treatment of a disease, disorder and/or condition of the CNS.
66. The AAV vector or pharmaceutical composition for use according to embodiment 64 or 65, wherein a therapeutically effective amount of the AAV vector or pharmaceutical composition is administered to a subject.
67. A method for preventing, alleviating and/or treating a CNS disease, disorder and/or condition in a subject in need thereof, comprising administering a therapeutically effective amount of the AAV vector of any one of embodiments 39 to 60 or the pharmaceutical composition of embodiment 61 to the subject.
68. The AAV vector or pharmaceutical composition for use according to embodiment 66, or the method of embodiment 67, wherein the AAV vector or pharmaceutical composition is administered via intravenous, intraperitoneal, intranasal, intravitreous, subcutaneous, intramuscular, intrathecal, intracisternal, intraparenchymal, intrastriatal or intracerebroventricular route.
69. The AVV vector or pharmaceutical composition for use according to embodiment 66 or 68, or the method according to embodiment 67 or 68, wherein the AAV vector or pharmaceutical composition is administered to the subject systemically, preferably via intravenous route.
70. The AAV vector or pharmaceutical composition for use according to any one of embodiments 66 or 68 to 69, or the method according to any one of embodiments 67 to 69, wherein the subject is a mammal, preferably a rodent or a primate.
71. The AAV vector or pharmaceutical composition for use according to embodiment 70, or the method of embodiment 70, wherein the subject is a mouse, a non-human primate or a human.
72. The CNS targeting peptide of any one of embodiments 1 to 24, the AAV capsid protein or AAV capsid of any one of embodiments 25 to 38, or the AAV vector of any one of embodiments 39 to 60, for use as a research tool.

### Sequence Listing

**Table 2. Targeting peptides sequences and SEQ ID Nos**

| **Targeting peptide** | **SEQ ID NO** | **Targeting peptide** | **SEQ ID NO** | **Targeting peptide** | **SEQ ID NO** | **Targeting peptide** | **SEQ ID NO** | **Targeting peptide** | **SEQ ID NO** | **Targeting peptide** | **SEQ ID NO** |
|---|---|---|---|---|---|---|---|---|---|---|---|
| WTTTSGY | 4 | NGNRITA | 77 | GNSLRAI | 149 | ELNSSRS | 221 | SNNAVVK | 293 | SNKSNAS | 365 |
| DSRLLLP | 5 | INNTKSI | 78 | SNNTTHQ | 150 | LSNSVKN | 222 | SNSSHAQ | 294 | RSNATMD | 366 |
| SHSPEFK | 6 | TNRTNTH | 79 | SNTSRTM | 151 | TNRVTSI | 223 | NNGTRTV | 295 | TNLSRST | 367 |
| YEVGGTL | 7 | RAGKNIA | 80 | GTVNDIK | 152 | ESNHIRS | 224 | ENGVRRI | 296 | SNAVSRA | 368 |
| THPNVNP | 8 | VMNSTKE | 81 | TQNITRQ | 153 | MNVTRPA | 225 | SNNHTKT | 297 | SNYVHSA | 369 |
| GGLTPPY | 9 | SNSVSGG | 82 | ANNTTNK | 154 | SNLSKQH | 226 | TNSAHVK | 298 | SNVSSGK | 370 |
| SNDIKEK | 10 | LNASSEQ | 83 | HPNATKV | 155 | QDNRIHQ | 227 | SQNHVKS | 299 | SNTVGKG | 371 |
| SNLTGLL | 11 | LPNTTRV | 84 | SNRTDPS | 156 | TNLVNHK | 228 | SNGIRSN | 300 | TNGTRSA | 372 |
| SGNDPSA | 12 | ENMTRSL | 85 | SNRANSP | 157 | VKHETGR | 229 | SNNSKHM | 301 | TNSITAL | 373 |
| SNQVASS | 13 | HSNQTRL | 86 | TYPGTTL | 158 | SNTVRPT | 230 | WDNHTRT | 302 | ANTIKGV | 374 |
| INSTKGN | 14 | SNTIRAT | 87 | ENVSRGR | 159 | IDNKIRT | 231 | TNTASLR | 303 | SNGTKIA | 375 |
| SNTVEKK | 15 | TNNLVKS | 88 | NNSARSA | 160 | ELNSIKR | 232 | EINHIKN | 304 | NNRVSNH | 376 |
| GNQVKTL | 16 | LENKTRA | 89 | SLNSVHR | 161 | GKNQPVR | 233 | TNTTRVG | 305 | DNQTRKP | 377 |
| INNTKTK | 17 | GNATRTL | 90 | VNNTKYL | 162 | TNKTSGA | 234 | TNKTLSS | 306 | TNVTRSS | 378 |
| TEIVTNK | 18 | YSNRTSI | 91 | SNNAKIN | 163 | SNRTSAL | 235 | SNVTRQN | 307 | QSNTIKA | 379 |
| TNRTREG | 19 | SNDVRSH | 92 | SNVSRSY | 164 | HGNLTRT | 236 | TNRIATP | 308 | TNEIKQF | 380 |
| SNLTRNL | 20 | VNVVGVR | 93 | VNAVHTR | 165 | DNNVRGG | 237 | TNSSKSA | 309 | QSNSVKS | 381 |
| TNETRST | 21 | EPSLLIP | 94 | ETNRLKT | 166 | VTNQTKP | 238 | QSNHTAP | 310 | PDNSVKI | 382 |
| YDRSATG | 22 | SNRATHY | 95 | TNLSNTI | 167 | APHANSR | 239 | AANSVKQ | 311 | TNDSRTH | 383 |
| LTHTTLK | 23 | NENRSSI | 96 | TNLTQSS | 168 | TNTTKVT | 240 | DNVVRPR | 312 | TNHTSSR | 384 |
| ENRTRTI | 24 | GSNHTKT | 97 | TNNARAA | 169 | ETNRTAK | 241 | DTNRTSR | 313 | TSTNYTK | 385 |
| IISNGQA | 25 | TNASKQK | 98 | TNRTTDH | 170 | SNLTREK | 242 | EGNAVRG | 314 | SNLSRST | 386 |
| TNMAREW | 26 | SNRTATR | 99 | ANQVRTT | 171 | HLNSVRE | 243 | DGNGVKQ | 315 | QNSTRST | 387 |
| HTNLVRT | 27 | AGNSTKT | 100 | SSSNSVR | 172 | NSNSVRA | 244 | SNSATIK | 316 | QGNNIKY | 388 |
| SNVTRTS | 28 | WSPTNGF | 101 | ITNSVKD | 173 | GNRSSSH | 245 | DVNTVRV | 317 | TNSVSNR | 389 |
| SDDNRTR | 29 | TINTTRA | 102 | INSVSKS | 174 | SSHNSVK | 246 | RENTVRT | 318 | SNRTQQQ | 390 |
| TNAIRPV | 30 | DINRTPR | 103 | TNTTKAS | 175 | GNGVRGT | 247 | EETKIPR | 319 | TNQTSAI | 391 |
| TNMTSKP | 31 | YSLKGIH | 104 | GNETRAK | 176 | SNTIKSS | 248 | ARNETRS | 320 | SNLTGNR | 392 |
| DSNQTPK | 32 | SNTISRA | 105 | YNLNTTK | 177 | SNRIQHT | 249 | ANQTQSR | 321 | SNNVRSH | 393 |
| ENNRYPN | 33 | SNRIQQV | 106 | PQNSARV | 178 | TNLIKSN | 250 | SNQSRDR | 322 | QGTMQAN | 394 |
| RNDTKEP | 34 | SNTIRQL | 107 | NNTKQGY | 179 | RSNSIHS | 251 | SNTIKGE | 323 | TNKIQSS | 395 |
| WTKSSGY | 35 | INNTRNF | 108 | SNLTRHT | 180 | GNRTTQS | 252 | SNRTTPT | 324 | SNTIPRN | 396 |
| IGNSSKQ | 36 | SASSIGE | 109 | DNEVRSR | 181 | ANSVRAN | 253 | INNRAPQ | 325 | SNVVQSK | 397 |
| ANNANTA | 37 | TNITRSG | 110 | SNQVNRV | 182 | LQNQTKL | 254 | GNRISTQ | 326 | QAGAGNI | 398 |
| MTSNITR | 38 | SPNHVRY | 111 | YVGSAKQ | 183 | ANRANAI | 255 | SNSVSHR | 327 | MENTVRT | 399 |
| TNIANAK | 39 | IPNATTR | 112 | SNISRPV | 184 | TNRVGTT | 256 | DNKIVRT | 328 | TNSARSN | 400 |
| SNNTAMP | 40 | NINHTKT | 113 | TNGTKTL | 185 | LNSVKTP | 257 | TNLSNAS | 329 | TNRTHTL | 401 |
| NNTVKVA | 41 | SNATRSG | 114 | QSNSVKP | 186 | NTNATRS | 258 | NANAIKQ | 330 | INQTRQG | 402 |
| FNVNRGS | 42 | TNYAKSG | 115 | NNSVRPS | 187 | STTRSDA | 259 | SNTIRGG | 331 | TFQNHTK | 403 |
| ETNRTRG | 43 | SNRTTLH | 116 | ENNTKPT | 188 | ANHARGV | 260 | NNNLIRD | 332 | SNSSSGT | 404 |
| ANRTRET | 44 | TNTITPK | 117 | SNSVSSI | 189 | FNHTKSI | 261 | SINHTRA | 333 | SNKISSM | 405 |
| ANNTRSA | 45 | SNQTSHM | 118 | PGNTIRQ | 190 | ESNLARR | 262 | SNRTSAT | 334 | SNQVRAP | 406 |
| TKKGSTS | 46 | SANQTRS | 119 | SNLVVQR | 191 | ASNSVRS | 263 | DNRVQAK | 335 | SSNSIRT | 407 |
| SYGEAVT | 47 | SNATNTT | 120 | SNNTKTI | 192 | STNKIRD | 264 | SNKAHST | 336 | GNTSRAA | 408 |
| ANAVRLQ | 48 | SNHSRTN | 121 | ENSAKQK | 193 | NNTVRHT | 265 | SNTVGHK | 337 | TNSAKSL | 409 |
| EENKTRP | 49 | SNKSASI | 122 | SNLVSRA | 194 | TLNATTR | 266 | GNVIRQL | 338 | SNRTSEA | 410 |
| DNTTRSI | 50 | TNTTRIP | 123 | MNGVRAA | 195 | GNATRQQ | 267 | HNTTKTG | 339 | SNSVTKI | 411 |
| QNETRVF | 51 | GNSTSTT | 124 | DTNVTRA | 196 | SNDVRQQ | 268 | VNAVKSN | 340 | SNQTSHL | 412 |
| EQNNSTK | 52 | EGNRVIS | 125 | SNIVAAS | 197 | HNATKLS | 269 | DSNRNKI | 341 | TNRVAAT | 413 |
| FNENRAK | 53 | SNITVER | 126 | GNHNTIR | 198 | VNSVRGE | 270 | SNNVRPT | 342 | ISTDRPQ | 414 |
| SNNKYTS | 54 | TNVSNKT | 127 | SNVVKSL | 199 | QNQIRPS | 271 | GNNATRT | 343 | LPNTIRT | 415 |
| SNTTSTT | 55 | ENGTRRQ | 128 | QDNVIRT | 200 | DNQVRAK | 272 | TNTIQSR | 344 | SINQTRS | 416 |
| ISNVTRT | 56 | STNSTRG | 129 | SNLIKNT | 201 | TGNAVRS | 273 | TNVTITK | 345 | DNGVRGT | 417 |
| SNIITSH | 57 | QNNTRSS | 130 | ENRARAA | 202 | TNEARAR | 274 | TNTVKSN | 346 | TNDAKHR | 418 |
| DKKELSA | 58 | MNIVAKG | 131 | SNVSRAS | 203 | SGNHIRT | 275 | VRPSHER | 347 | SNSSKHN | 419 |
| NLNSTRL | 59 | ESNTTRT | 132 | NNTVRPL | 204 | TNSIRGG | 276 | SNTIRPS | 348 | GNFSRAG | 420 |
| SHSPTFR | 60 | SNITRGM | 133 | FNGTRGT | 205 | VNATQRT | 277 | SNTTRAH | 349 | TNRTAGP | 421 |
| SLNNTRT | 61 | SSSAQTI | 134 | GNSVREQ | 206 | TNKTAPT | 278 | SANSVKN | 350 | SNATYKG | 422 |
| SNTITGV | 62 | GEASTKE | 135 | VNTVRGL | 207 | SNSVGKI | 279 | MPNTIKL | 351 | SNRTHPA | 423 |
| SNDMRQQ | 63 | SPNQVRS | 136 | SDNHTKI | 208 | SNKSQGQ | 280 | GNSAISI | 352 | TNATGPL | 424 |
| SNLTSKS | 64 | SNRTNIA | 137 | ENRTSIR | 209 | DQNLTRR | 281 | NANSIKN | 353 | EGNSVRV | 425 |
| TNRTSQP | 65 | SNVTRST | 138 | VVNTTQR | 210 | GNDTRKA | 282 | SLNSTKP | 354 | YNITKQN | 426 |
| TNTTRTV | 66 | QNTNIIK | 139 | TNLTHPK | 211 | SNTVRHD | 283 | TNAISGS | 355 | TNVSRLG | 427 |
| SNSIESR | 67 | WSTSKGY | 140 | SNEARVH | 212 | ANTIRSN | 284 | VNTTKLS | 356 | TNSTQSP | 428 |
| ANLISSR | 68 | TASTVDK | 141 | SNLTGRS | 213 | GNVVAAR | 285 | SNRTSTI | 357 | TNAVSPS | 429 |
| LAQNLTR | 69 | LPNRTIQ | 142 | GHNAIRN | 214 | MNKVSAI | 286 | SNITRTM | 358 | TNSTYAR | 430 |
| INTVKSY | 70 | TGTSGST | 143 | GNQVRSS | 215 | TTNSVKT | 287 | SANDIKT | 359 | PNNYTKV | 431 |
| NNNESRP | 71 | VTNRIPS | 144 | SNRTYSD | 216 | ESNTTRQ | 288 | GNSIRSG | 360 | ENHTHAK | 432 |
| YKVGLNT | 72 | MLNSLRG | 145 | HTNASRS | 217 | GVNVTKP | 289 | TNESRAK | 361 | TNATSGG | 433 |
| SNRNNTI | 73 | SNSTKLQ | 146 | SNSVKAN | 218 | SNSSKEY | 290 | TNTARSG | 362 | SNRVTNG | 434 |
| HGNSSRS | 74 | SNNNTRS | 147 | ENRTKHT | 219 | SLNRIHS | 291 | SNNVPSR | 363 | SINSARA | 435 |
| PDNPRTK | 75 | HDHVRQD | 148 | IGNSVRQ | 220 | INNVRSQ | 292 | EANAVKT | 364 | HDNSARS | 436 |
| LSANTVR | 76 | | | | | | | | | | |

**Table 3. AAV capsid protein sequences and SEQ ID Nos**

| |
|---|
| AA Vrh10 - SEQ ID NO: 1 |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN |
| NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQNAAPIVGAVN SQGALPGMVWQNRDVYLQGPIWAKIPHTDGNFHPSPLMGGFGLKHPPPQILIKNTPVPADPPTTFSQAKLASFITQYSTGQVSVEIEW ELQKENSKRWNPEIQYTSNYYKSTNVDFAVNTDGTYSEPRPIGTRYLTRNL |
| AAV9 - SEQ ID NO: 2 |
| MAADGYLPDWLEDNLSEGIREWWALKPGAPQPKANQQHQDNARGLVLPGYKYLGPGNGLDKGEPVNAADAAALEHDKAYDQQ LKAGDNPYLKYNHADAEFQERLKEDTSFGGNLGRAVFQAKKRLLEPLGLVEEAAKTAPGKKRPVEQSPQEPDSSAGIGKSGAQPAK KRLNFGQTGDTESVPDPQPIGEPPAAPSGVGSLTMASGGGAPVADNNEGADGVGSSSGNWHCDSQWLGDRVITTSTRTWALPTYN NHLYKQISNSTSGGSSNDNAYFGYSTPWGYFDFNRFHCHFSPRDWQRLINNNWGFRPKRLNFKLFNIQVKEVTDNNGVKTIANNLT STVQVFTDSDYQLPYVLGSAHEGCLPPFPADVFMIPQYGYLTLNDGSQAVGRSSFYCLEYFPSQMLRTGNNFQFSYEFENVPFHSSY AHSQSLDRLMNPLIDQYLYYLSKTINGSGQNQQTLKFSVAGPSNMAVQGRNYIPGPSYRQQRVSTTVTQNNNSEFAWPGASSWALN GRNSLMNPGPAMASHKEGEDRFFPLSGSLIFGKQGTGRDNVDADKVMITNEEEIKTTNPVATESYGQVATNHQSAQAQAQTGWVQ NQGILPGMVWQDRDVYLQGPIWAKIPHTDGNFHPSPLMGGFGMKHPPPQILIKNTPVPADPPTAFNKDKLNSFITQYSTGQVSVEIE WELQKENSKRWNPEIQYTSNYYKSNNVEFAVNTEGVYSEPRPIGTRYLTRNL |
| AAV-PHP.eB - SEQ ID NO: 3 |
| MAADGYLPDWLEDNLSEGIREWWALKPGAPQPKANQQHQDNARGLVLPGYKYLGPGNGLDKGEPVNAADAAALEHDKAYDQQ LKAGDNPYLKYNHADAEFQERLKEDTSFGGNLGRAVFQAKKRLLEPLGLVEEAAKTAPGKKRPVEQSPQEPDSSAGIGKSGAQPAK KRLNFGQTGDTESVPDPQPIGEPPAAPSGVGSLTMASGGGAPVADNNEGADGVGSSSGNWHCDSQWLGDRVITTSTRTWALPTYN NHLYKQISNSTSGGSSNDNAYFGYSTPWGYFDFNRFHCHFSPRDWQRLINNNWGFRPKRLNFKLFNIQVKEVTDNNGVKTIANNLT |
| STVQVFTDSDYQLPYVLGSAHEGCLPPFPADVFMIPQYGYLTLNDGSQAVGRSSFYCLEYFPSQMLRTGNNFQFSYEFENVPFHSSY AHSQSLDRLMNPLIDQYLYYLSRTINGSGQNQQTLKFSVAGPSNMAVQGRNYIPGPSYRQQRVSTTVTQNNNSEFAWPGASSWALN GRNSLMNPGPAMASHKEGEDRFFPLSGSLIFGKQGTGRDNVDADKVMITNEEEIKTTNPVATESYGQVATNHQSDGTLAVPFKAQA QTGWVQNQGILPGMVWQDRDVYLQGPIWAKIPHTDGNFHPSPLMGGFGMKHPPPQILIKNTPVPADPPTAFNKDKLNSFITQYSTG QVSVEIEWELQKENSKRWNPEIQYTSNYYKSNNVEFAVNTEGVYSEPRPIGTRYLTRNL |
| ACI-AAVrh10-v4 - SEQ ID NO: 437 - (comprising the targeting peptide of SEQ ID NO: 4 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQWTTTSGYNA APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v5 - SEQ ID NO: 438 - (comprising the targeting peptide of SEQ ID NO: 5 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH |
| SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQDSRLLLPNAA PIVGAVNSQGALPGMVWQNRDVYLQGPIWAKIPHTDGNFHPSPLMGGFGLKHPPPQILIKNTPVPADPPTTFSQAKLASFITQYSTGQ VSVEIEWELQKENSKRWNPEIQYTSNYYKSTNVDFAVNTDGTYSEPRPIGTRYLTRNL |
| ACI-AAVrh10-v6 - SEQ ID NO: 439 - (comprising the targeting peptide of SEQ ID NO: 6 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQSHSPEFKNAA PIVGAVNSQGALPGMVWQNRDVYLQGPIWAKIPHTDGNFHPSPLMGGFGLKHPPPQILIKNTPVPADPPTTFSQAKLASFITQYSTGQ VSVEIEWELQKENSKRWNPEIQYTSNYYKSTNVDFAVNTDGTYSEPRPIGTRYLTRNL |
| ACI-AAVrh10-v7 - SEQ ID NO: 440 - (comprising the targeting peptide of SEQ ID NO: 7 underlined) |
| MAADGYLPDWLEDNLSEGIREWWDLKPGAPKPKANQQKQDDGRGLVLPGYKYLGPFNGLDKGEPVNAADAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN |
| GRDSLVNPGVAMATHKDDEERFFPSSGVLMFGKQGAGKDNVDYSSVMLTSEEEIKTTNPVATEQYGVVADNLQQQYEVGGTLNA APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v9 - SEQ ID NO: 441 - (comprising the targeting peptide of SEQ ID NO: 9 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQGGLTPPYNAA PIVGAVNSQGALPGMVWQNRDVYLQGPIWAKIPHTDGNFHPSPLMGGFGLKHPPPQILIKNTPVPADPPTTFSQAKLASFITQYSTGQ VSVEIEWELQKENSKRWNPEIQYTSNYYKSTNVDFAVNTDGTYSEPRPIGTRYLTRNL |
| ACI-AA Vrh10-v11 - SEQ ID NO: 442 - (comprising the targeting peptide of SEQ ID NO: 11 underlined) |
| MAADGYLPDWLEDNLSEGIREWWDLKPGAPKPKANQQKQDDGRGLVLPGYKYLGPFNGLDKGEPVNAADAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQSNLTGLLNAA |
| PIVGAVNSQGALPGMVWQNRDVYLQGPIWAKIPHTDGNFHPSPLMGGFGLKHPPPQILIKNTPVPADPPTTFSQAKLASFITQYSTGQ VSVEIEWELQKENSKRWNPEIQYTSNYYKSTNVDFAVNTDGTYSEPRPIGTRYLTRNL |
| ACI-AAVrh10-v12 - SEQ ID NO: 443 - (comprising the targeting peptide of SEQ ID NO: 12 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQSGNDPSANA APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v13 - SEQ ID NO: 444 - (comprising the targeting peptide of SEQ ID NO: 13 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQSNQVASSNA |
| APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v16 - SEQ ID NO: 445 - (comprising the targeting peptide of SEQ ID NO: 16 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQGNQVKTLNA APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v35 - SEQ ID NO: 446 - (comprising the targeting peptide of SEQ ID NO: 35 underlined) |
| MAADGYLPDWLEDNLSEGIREWWDLKPGAPKPKANQQKQDDGRGLVLPGYKYLGPFNGLDKGEPVNAADAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQWTKSSGYNA |
| APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v36 - SEQ ID NO: 447 - (comprising the targeting peptide of SEQ ID NO: 36 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQIGNSSKQNAA PIVGAVNSQGALPGMVWQNRDVYLQGPIWAKIPHTDGNFHPSPLMGGFGLKHPPPQILIKNTPVPADPPTTFSQAKLASFITQYSTGQ VSVEIEWELQKENSKRWNPEIQYTSNYYKSTNVDFAVNTDGTYSEPRPIGTRYLTRNL |
| ACI-AAVrh10-v60 - SEQ ID NO: 448 - (comprising the targeting peptide of SEQ ID NO: 60 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQSHSPTFRNAA |
| PIVGAVNSQGALPGMVWQNRDVYLQGPIWAKIPHTDGNFHPSPLMGGFGLKHPPPQILIKNTPVPADPPTTFSQAKLASFITQYSTGQ VSVEIEWELQKENSKRWNPEIQYTSNYYKSTNVDFAVNTDGTYSEPRPIGTRYLTRNL |
| ACI-AAVrh10-v178 - SEQ ID NO: 449 - (comprising the targeting peptide of SEQ ID NO: 178 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQPQNSARVNA APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v180 - SEQ ID NO: 450 - (comprising the targeting peptide of SEQ ID NO: 180 underlined) |
| MAADGYLPDWLEDNLSEGIREWWDLKPGAPKPKANQQKQDDGRGLVLPGYKYLGPFNGLDKGEPVNAADAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQSNLTRHTNAA |
| PIVGAVNSQGALPGMVWQNRDVYLQGPIWAKIPHTDGNFHPSPLMGGFGLKHPPPQILIKNTPVPADPPTTFSQAKLASFITQYSTGQ VSVEIEWELQKENSKRWNPEIQYTSNYYKSTNVDFAVNTDGTYSEPRPIGTRYLTRNL |
| ACI-AAVrh10-v181 - SEQ ID NO: 451 - (comprising the targeting peptide of SEQ ID NO: 181 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQDNEVRSRNA APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v183 - SEQ ID NO: 452 - (comprising the targeting peptide of SEQ ID NO: 183 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQYVGSAKQNA |
| APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v186 - SEQ ID NO: 453 - (comprising the targeting peptide of SEQ ID NO: 186 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQSNSVKPNA APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v188 - SEQ ID NO: 454 - (comprising the targeting peptide of SEQ ID NO: 188 underlined) |
| MAADGYLPDWLEDNLSEGIREWWDLKPGAPKPKANQQKQDDGRGLVLPGYKYLGPFNGLDKGEPVNAADAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQENNTKPTNA |
| APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v190 - SEQ ID NO: 455 - (comprising the targeting peptide of SEQ ID NO: 190 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQPGNTIRQNAA PIVGAVNSQGALPGMVWQNRDVYLQGPIWAKIPHTDGNFHPSPLMGGFGLKHPPPQILIKNTPVPADPPTTFSQAKLASFITQYSTGQ VSVEIEWELQKENSKRWNPEIQYTSNYYKSTNVDFAVNTDGTYSEPRPIGTRYLTRNL |
| ACI-AAVrh10-v196 - SEQ ID NO: 456 - (comprising the targeting peptide of SEQ ID NO: 196 underlined) |
| MAADGYLPDWLEDNLSEGIREWWDLKPGAPKPKANQQKQDDGRGLVLPGYKYLGPFNGLDKGEPVNAADAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQDTNVTRANA |
| APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v202 - SEQ ID NO: 457 - (comprising the targeting peptide of SEQ ID NO: 202 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQENRARAANA APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v220 - SEQ ID NO: 458 - (comprising the targeting peptide of SEQ ID NO: 220 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQIGNSVRQNAA |
| PIVGAVNSQGALPGMVWQNRDVYLQGPIWAKIPHTDGNFHPSPLMGGFGLKHPPPQILIKNTPVPADPPTTFSQAKLASFITQYSTGQ VSVEIEWELQKENSKRWNPEIQYTSNYYKSTNVDFAVNTDGTYSEPRPIGTRYLTRNL |
| ACI-AAVrh10-v221 - SEQ ID NO: 459 - (comprising the targeting peptide of SEQ ID NO: 221 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQELNSSRSNAA PIVGAVNSQGALPGMVWQNRDVYLQGPIWAKIPHTDGNFHPSPLMGGFGLKHPPPQILIKNTPVPADPPTTFSQAKLASFITQYSTGQ VSVEIEWELQKENSKRWNPEIQYTSNYYKSTNVDFAVNTDGTYSEPRPIGTRYLTRNL |
| ACI-AAVrh10-v227 - SEQ ID NO: 460 - (comprising the targeting peptide of SEQ ID NO: 227 underlined) |
| MAADGYLPDWLEDNLSEGIREWWDLKPGAPKPKANQQKQDDGRGLVLPGYKYLGPFNGLDKGEPVNAADAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQDNRIHQNA |
| APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v229 - SEQ ID NO: 461 - (comprising the targeting peptide of SEQ ID NO: 229 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQVKHETGRNA APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v233 - SEQ ID NO: 462 - (comprising the targeting peptide of SEQ ID NO: 233 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQGKNQPVRNA |
| APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v234 - SEQ ID NO: 463 - (comprising the targeting peptide of SEQ ID NO: 234 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQTNKTSGANA APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v244 - SEQ ID NO: 464 - (comprising the targeting peptide of SEQ ID NO: 244 underlined) |
| MAADGYLPDWLEDNLSEGIREWWDLKPGAPKPKANQQKQDDGRGLVLPGYKYLGPFNGLDKGEPVNAADAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQNSNSVRANA |
| APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v245 - SEQ ID NO: 465 - (comprising the targeting peptide of SEQ ID NO: 245 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQGNRSSSHNAA PIVGAVNSQGALPGMVWQNRDVYLQGPIWAKIPHTDGNFHPSPLMGGFGLKHPPPQILIKNTPVPADPPTTFSQAKLASFITQYSTGQ VSVEIEWELQKENSKRWNPEIQYTSNYYKSTNVDFAVNTDGTYSEPRPIGTRYLTRNL |
| ACI-AAVrh10-v246 - SEQ ID NO: 466 - (comprising the targeting peptide of SEQ ID NO: 246 underlined) |
| MAADGYLPDWLEDNLSEGIREWWDLKPGAPKPKANQQKQDDGRGLVLPGYKYLGPFNGLDKGEPVNAADAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQSSHNSVKNA |
| APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v249 - SEQ ID NO: 467 - (comprising the targeting peptide of SEQ ID NO: 249 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQSNRIQHTNAA PIVGAVNSQGALPGMVWQNRDVYLQGPIWAKIPHTDGNFHPSPLMGGFGLKHPPPQILIKNTPVPADPPTTFSQAKLASFITQYSTGQ VSVEIEWELQKENSKRWNPEIQYTSNYYKSTNVDFAVNTDGTYSEPRPIGTRYLTRNL |
| ACI-AAVrh10-v250 - SEQ ID NO: 468 - (comprising the targeting peptide of SEQ ID NO: 250 underlined) |
| MAADGYLPDWLEDNLSEGIREWWDLKPGAPKPKANQQKQDDGRGLVLPGYKYLGPFNGLDKGEPVNAADAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQTNLIKSNNAA |
| PIVGAVNSQGALPGMVWQNRDVYLQGPIWAKIPHTDGNFHPSPLMGGFGLKHPPPQILIKNTPVPADPPTTFSQAKLASFITQYSTGQ VSVEIEWELQKENSKRWNPEIQYTSNYYKSTNVDFAVNTDGTYSEPRPIGTRYLTRNL |
| ACI-AAVrh10-v253 - SEQ ID NO: 469 - (comprising the targeting peptide of SEQ ID NO: 253 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQANSVRANNA APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v254 - SEQ ID NO: 470 - (comprising the targeting peptide of SEQ ID NO: 254 underlined) |
| MAADGYLPDWLEDNLSEGIREWWDLKPGAPKPKANQQKQDDGRGLVLPGYKYLGPFNGLDKGEPVNAADAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQLQNQTKLNA |
| APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v275 - SEQ ID NO: 471 - (comprising the targeting peptide of SEQ ID NO: 275 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQSGNHIRTNAA PIVGAVNSQGALPGMVWQNRDVYLQGPIWAKIPHTDGNFHPSPLMGGFGLKHPPPQILIKNTPVPADPPTTFSQAKLASFITQYSTGQ VSVEIEWELQKENSKRWNPEIQYTSNYYKSTNVDFAVNTDGTYSEPRPIGTRYLTRNL |
| ACI-AAVrh10-v281 - SEQ ID NO: 472 - (comprising the targeting peptide of SEQ ID NO: 281 underlined) |
| MAADGYLPDWLEDNLSEGIREWWDLKPGAPKPKANQQKQDDGRGLVLPGYKYLGPFNGLDKGEPVNAADAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQDQNLTRRNA |
| APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v286 - SEQ ID NO: 473 - (comprising the targeting peptide of SEQ ID NO: 286 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQMNKVSAINA APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v289 - SEQ ID NO: 474 - (comprising the targeting peptide of SEQ ID NO: 289 underlined) |
| MAADGYLPDWLEDNLSEGIREWWDLKPGAPKPKANQQKQDDGRGLVLPGYKYLGPFNGLDKGEPVNAADAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQGVNVTKPNA |
| APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v290 - SEQ ID NO: 475 - (comprising the targeting peptide of SEQ ID NO: 290 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQSNSSKEYNAA PIVGAVNSQGALPGMVWQNRDVYLQGPIWAKIPHTDGNFHPSPLMGGFGLKHPPPQILIKNTPVPADPPTTFSQAKLASFITQYSTGQ VSVEIEWELQKENSKRWNPEIQYTSNYYKSTNVDFAVNTDGTYSEPRPIGTRYLTRNL |
| ACI-AAVrh10-v297 - SEQ ID NO: 476 - (comprising the targeting peptide of SEQ ID NO: 297 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQSNNHTKTNA |
| APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v315 - SEQ ID NO: 477 - (comprising the targeting peptide of SEQ ID NO: 315 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQDGNGVKQNA APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v321 - SEQ ID NO: 478 - (comprising the targeting peptide of SEQ ID NO: 321 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQANQTQSRNA |
| APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v327 - SEQ ID NO: 479 - (comprising the targeting peptide of SEQ ID NO: 327 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQSNSVSHRNAA PIVGAVNSQGALPGMVWQNRDVYLQGPIWAKIPHTDGNFHPSPLMGGFGLKHPPPQILIKNTPVPADPPTTFSQAKLASFITQYSTGQ VSVEIEWELQKENSKRWNPEIQYTSNYYKSTNVDFAVNTDGTYSEPRPIGTRYLTRNL |
| ACI-AAVrh10-v330 - SEQ ID NO: 480 - (comprising the targeting peptide of SEQ ID NO: 330 underlined) |
| MAADGYLPDWLEDNLSEGIREWWDLKPGAPKPKANQQKQDDGRGLVLPGYKYLGPFNGLDKGEPVNAADAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQNANAIKQNA |
| APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v331 - SEQ ID NO: 481 - (comprising the targeting peptide of SEQ ID NO: 331 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQSNTIRGGNAA PIVGAVNSQGALPGMVWQNRDVYLQGPIWAKIPHTDGNFHPSPLMGGFGLKHPPPQILIKNTPVPADPPTTFSQAKLASFITQYSTGQ VSVEIEWELQKENSKRWNPEIQYTSNYYKSTNVDFAVNTDGTYSEPRPIGTRYLTRNL |
| ACI-AAVrh10-v339 - SEQ ID NO: 482 - (comprising the targeting peptide of SEQ ID NO: 339 underlined) |
| MAADGYLPDWLEDNLSEGIREWWDLKPGAPKPKANQQKQDDGRGLVLPGYKYLGPFNGLDKGEPVNAADAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQHNTTKTGNA |
| APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v345 - SEQ ID NO: 483 - (comprising the targeting peptide of SEQ ID NO: 345 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQTNVTITKNAA PIVGAVNSQGALPGMVWQNRDVYLQGPIWAKIPHTDGNFHPSPLMGGFGLKHPPPQILIKNTPVPADPPTTFSQAKLASFITQYSTGQ VSVEIEWELQKENSKRWNPEIQYTSNYYKSTNVDFAVNTDGTYSEPRPIGTRYLTRNL |
| ACI-AAVrh10-v346 - SEQ ID NO: 484 - (comprising the targeting peptide of SEQ ID NO: 346 underlined) |
| MAADGYLPDWLEDNLSEGIREWWDLKPGAPKPKANQQKQDDGRGLVLPGYKYLGPFNGLDKGEPVNAADAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQTNTVKSNNA |
| APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v350 - SEQ ID NO: 485 - (comprising the targeting peptide of SEQ ID NO: 350 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQSANSVKNNA APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v353 - SEQ ID NO: 486 - (comprising the targeting peptide of SEQ ID NO: 353 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQNANSIKNNAA |
| PIVGAVNSQGALPGMVWQNRDVYLQGPIWAKIPHTDGNFHPSPLMGGFGLKHPPPQILIKNTPVPADPPTTFSQAKLASFITQYSTGQ VSVEIEWELQKENSKRWNPEIQYTSNYYKSTNVDFAVNTDGTYSEPRPIGTRYLTRNL |
| ACI-AAVrh10-v361 - SEQ ID NO: 487 - (comprising the targeting peptide of SEQ ID NO: 361 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQTNESRAKNA APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v362 - SEQ ID NO: 488 - (comprising the targeting peptide of SEQ ID NO: 362 underlined) |
| MAADGYLPDWLEDNLSEGIREWWDLKPGAPKPKANQQKQDDGRGLVLPGYKYLGPFNGLDKGEPVNAADAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQTNTARSGNA |
| APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v369 - SEQ ID NO: 489 - (comprising the targeting peptide of SEQ ID NO: 369 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQSNYVHSANA APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v377 - SEQ ID NO: 490 - (comprising the targeting peptide of SEQ ID NO: 377 underlined) |
| MAADGYLPDWLEDNLSEGIREWWDLKPGAPKPKANQQKQDDGRGLVLPGYKYLGPFNGLDKGEPVNAADAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQDNQTRKPNA |
| APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v384 - SEQ ID NO: 491 - (comprising the targeting peptide of SEQ ID NO: 384 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQTNHTSSRNAA PIVGAVNSQGALPGMVWQNRDVYLQGPIWAKIPHTDGNFHPSPLMGGFGLKHPPPQILIKNTPVPADPPTTFSQAKLASFITQYSTGQ VSVEIEWELQKENSKRWNPEIQYTSNYYKSTNVDFAVNTDGTYSEPRPIGTRYLTRNL |
| ACI-AAVrh10-v385 - SEQ ID NO: 492 - (comprising the targeting peptide of SEQ ID NO: 385 underlined) |
| MAADGYLPDWLEDNLSEGIREWWDLKPGAPKPKANQQKQDDGRGLVLPGYKYLGPFNGLDKGEPVNAADAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQTSTNYTKNA |
| APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v397 - SEQ ID NO: 493 - (comprising the targeting peptide of SEQ ID NO: 397 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQSNVVQSKNA APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v404 - SEQ ID NO: 494 - (comprising the targeting peptide of SEQ ID NO: 404 underlined) |
| MAADGYLPDWLEDNLSEGIREWWDLKPGAPKPKANQQKQDDGRGLVLPGYKYLGPFNGLDKGEPVNAADAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQSNSSSGTNAA |
| PIVGAVNSQGALPGMVWQNRDVYLQGPIWAKIPHTDGNFHPSPLMGGFGLKHPPPQILIKNTPVPADPPTTFSQAKLASFITQYSTGQ VSVEIEWELQKENSKRWNPEIQYTSNYYKSTNVDFAVNTDGTYSEPRPIGTRYLTRNL |
| ACI-AAVrh10-v422 - SEQ ID NO: 495 - (comprising the targeting peptide of SEQ ID NO: 422 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQSNATYKGNA APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| ACI-AAVrh10-v435 - SEQ ID NO: 496 - (comprising the targeting peptide of SEQ ID NO: 435 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQSINSARANAA |
| PIVGAVNSQGALPGMVWQNRDVYLQGPIWAKIPHTDGNFHPSPLMGGFGLKHPPPQILIKNTPVPADPPTTFSQAKLASFITQYSTGQ VSVEIEWELQKENSKRWNPEIQYTSNYYKSTNVDFAVNTDGTYSEPRPIGTRYLTRNL |
| ACI-AAVrh10-v436 - SEQ ID NO: 497 - (comprising the targeting peptide of SEQ ID NO: 436 underlined) |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEPSPQRSPDSSTGIGKKGQQPAK KRLNFGQTGDSESVPDPQPIGEPPAGPSGLGSGTMAAGGGAPMADNNEGADGVGSSSGNWHCDSTWLGDRVITTSTRTWALPTYN NHLYKQISNGTSGGSTNDNTYFGYSTPWGYFDFNRFHCHFSPRDWQRLINNNWGFRPKRLNFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFEFSYQFEDVPFHSSYAH SQSLDRLMNPLIDQYLYYLSRTQSTGGTAGTQQLLFSQAGPNNMSAQAKNWLPGPCYRQQRVSTTLSQNNNSNFAWTGATKYHLN GRDSLVNPGVAMATHKDDEERFFPSSGVLMFGKQGAGKDNVDYSSVMLTSEEEIKTTNPVATEQYGVVADNLQQQHDNSARSNA APIVGAVNSQGALPGMVWQNRDVYLQGPIWAKIPHTDGNFHPSPLMGGFGLKHPPPQILIKNTPVPADPPTTFSQAKLASFITQYSTG QVSVEIEWELQKENSKRWNPEIQYTSNYYKSTNVDFAVNTDGTYSEPRPIGTRYLTRNL |
| AAV2 - SEQ ID NO: 498 |
| |
| |
| AAV1 - SEQ ID NO: 499 |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPGKKRPVEQSPQEPDSSSGIGKTGQQPAKK RLNFGQTGDSESVPDPQPLGEPPATPAAVGPTTMASGGGAPMADNNEGADGVGNASGNWHCDSTWLGDRVITTSTRTWALPTYN NHLYKQISSASTGASNDNHYFGYSTPWGYFDFNRFHCHFSPRDWQRLINNNWGFRPKRLNFKLFNIQVKEVTTNDGVTTIANNLTST VQVFSDSEYQLPYVLGSAHQGCLPPFPADVFMIPQYGYLTLNNGSQAVGRSSFYCLEYFPSQMLRTGNNFTFSYTFEEVPFHSSYAHS QSLDRLMNPLIDQYLYYLNRTQNQSGSAQNKDLLFSRGSPAGMSVQPKNWLPGPCYRQQRVSKTKTDNNNSNFTWTGASKYNLN GRESIINPGTAMASHKDDEDKFFPMSGVMIFGKESAGASNTALDNVMITDEEEIKATNPVATERFGTVAVNFQSSSTDPATGDVHAM GALPGMVWQDRDVYLQGPIWAKIPHTDGHFHPSPLMGGFGLKNPPPQILIKNTPVPANPPAEFSATKFASFITQYSTGQVSVEIEWEL QKENSKRWNPEVQYTSNYAKSANVDFTVDNNGLYTEPRPIGTRYLTRPL |
| AAV5 - SEQ ID NO: 500 |
| MSFVDHPPDWLEEVGEGLREFLGLEAGPPKPKPNQQHQDQARGLVLPGYNYLGPGNGLDRGEPVNRADEVAREHDISYNEQLEAG DNPYLKYNHADAEFQEKLADDTSFGGNLGKAVFQAKKRVLEPFGLVEEGAKTAPTGKRIDDHFPKRKKARTEEDSKPSTSSDAEAG PSGSQQLQIPAQPASSLGADTMSAGGGGPLGDNNQGADGVGNASGDWHCDSTWMGDRVVTKSTRTWVLPSYNNHQYREIKSGSV DGSNANAYFGYSTPWGYFDFNRFHSHWSPRDWQRLINNYWGFRPRSLRVKIFNIQVKEVTVQDSTTTIANNLTSTVQVFTDDDYQL PYVVGNGTEGCLPAFPPQVFTLPQYGYATLNRDNTENPTERSSFFCLEYFPSKMLRTGNNFEFTYNFEEVPFHSSFAPSQNLFKLANP LVDQYLYRFVSTNNTGGVQFNKNLAGRYANTYKNWFPGPMGRTQGWNLGSGVNRASVSAFATTNRMELEGASYQVPPQPNGMT NNLQGSNTYALENTMIFNSQPANPGTTATYLEGNMLITSESETQPVNRVAYNVGGQMATNNQSSTTAPATGTYNLQEIVPGSVWME |
| RDVYLQGPIWAKIPETGAHFHPSPAMGGFGLKHPPPMMLIKNTPVPGNITSFSDVPVSSFITQYSTGQVTVEMEWELKKENSKRWNP EIQYTNNYNDPQFVDFAPDSTGEYRTTRPIGTRYLTRPL |
| AAV3b - SEQ ID NO: 501 |
| MAADGYLPDWLEDNLSEGIREWWALKPGVPQPKANQQHQDNRRGLVLPGYKYLGPGNGLDKGEPVNEADAAALEHDKAYDQQL KAGDNPYLKYNHADAEFQERLQEDTSFGGNLGRAVFQAKKRILEPLGLVEEAAKTAPGKKRPVDQSPQEPDSSSGVGKSGKQPARK RLNFGQTGDSESVPDPQPLGEPPAAPTSLGSNTMASGGGAPMADNNEGADGVGNSSGNWHCDSQWLGDRVITTSTRTWALPTYNN HLYKQISSQSGASNDNHYFGYSTPWGYFDFNRFHCHFSPRDWQRLINNNWGFRPKKLSFKLFNIQVKEVTQNDGTTTIANNLTSTVQ VFTDSEYQLPYVLGSAHQGCLPPFPADVFMVPQYGYLTLNNGSQAVGRSSFYCLEYFPSQMLRTGNNFQFSYTFEDVPFHSSYAHSQ SLDRLMNPLIDQYLYYLNRTQGTTSGTTNQSRLLFSQAGPQSMSLQARNWLPGPCYRQQRLSKTANDNNNSNFPWTAASKYHLNG RDSLVNPGPAMASHKDDEEKFFPMHGNLIFGKEGTTASNAELDNVMITDEEEIRTTNPVATEQYGTVANNLQSSNTAPTTRTVNDQ GALPGMVWQDRDVYLQGPIWAKIPHTDGHFHPSPLMGGFGLKHPPPQIMIKNTPVPANPPTTFSPAKFASFITQYSTGQVSVEIEWEL QKENSKRWNPEIQYTSNYNKSVNVDFTVDTNGVYSEPRPIGTRYLTRNL |
| AAV4 - SEQ ID NO: 502 |
| MTDGYLPDWLEDNLSEGVREWWALQPGAPKPKANQQHQDNARGLVLPGYKYLGPGNGLDKGEPVNAADAAALEHDKAYDQQL KAGDNPYLKYNHADAEFQQRLQGDTSFGGNLGRAVFQAKKRVLEPLGLVEQAGETAPGKKRPLIESPQQPDSSTGIGKKGKQPAKK KLVFEDETGAGDGPPEGSTSGAMSDDSEMRAAAGGAAVEGGQGADGVGNASGDWHCDSTWSEGHVTTTSTRTWVLPTYNNHLY KRLGESLQSNTYNGFSTPWGYFDFNRFHCHFSPRDWQRLINNNWGMRPKAMRVKIFNIQVKEVTTSNGETTVANNLTSTVQIFADS SYELPYVMDAGQEGSLPPFPNDVFMVPQYGYCGLVTGNTSQQQTDRNAFYCLEYFPSQMLRTGNNFEITYSFEKVPFHSMYAHSQS LDRLMNPLIDQYLWGLQSTTTGTTLNAGTATTNFTKLRPTNFSNFKKNWLPGPSIKQQGFSKTANQNYKIPATGSDSLIKYETHSTLD GRWSALTPGPPMATAGPADSKFSNSQLIFAGPKQNGNTATVPGTLIFTSEEELAATNATDTDMWGNLPGGDQSNSNLPTVDRLTAL |
| GAVPGMVWQNRDIYYQGPIWAKIPHTDGHFHPSPLIGGFGLKHPPPQIFIKNTPVPANPATTFSSTPVNSFITQYSTGQVSVQIDWEIQ KERSKRWNPEVQFTSNYGQQNSLLWAPDAAGKYTEPRAIGTRYLTHHL |
| AAV6 - SEQ ID NO: 503 |
| MAADGYLPDWLEDNLSEGIREWWDLKPGAPKPKANQQKQDDGRGLVLPGYKYLGPFNGLDKGEPVNAADAAALEHDKAYDQQL KAGDNPYLRYNHADAEFQERLQEDTSFGGNLGRAVFQAKKRVLEPFGLVEEGAKTAPGKKRPVEQSPQEPDSSSGIGKTGQQPAKK RLNFGQTGDSESVPDPQPLGEPPATPAAVGPTTMASGGGAPMADNNEGADGVGNASGNWHCDSTWLGDRVITTSTRTWALPTYN NHLYKQISSASTGASNDNHYFGYSTPWGYFDFNRFHCHFSPRDWQRLINNNWGFRPKRLNFKLFNIQVKEVTTNDGVTTIANNLTST VQVFSDSEYQLPYVLGSAHQGCLPPFPADVFMIPQYGYLTLNNGSQAVGRSSFYCLEYFPSQMLRTGNNFTFSYTFEDVPFHSSYAH SQSLDRLMNPLIDQYLYYLNRTQNQSGSAQNKDLLFSRGSPAGMSVQPKNWLPGPCYRQQRVSKTKTDNNNSNFTWTGASKYNLN GRESIINPGTAMASHKDDKDKFFPMSGVMIFGKESAGASNTALDNVMITDEEEIKATNPVATERFGTVAVNLQSSSTDPATGDVHVM GALPGMVWQDRDVYLQGPIWAKIPHTDGHFHPSPLMGGFGLKHPPPQILIKNTPVPANPPAEFSATKFASFITQYSTGQVSVEIEWEL QKENSKRWNPEVQYTSNYAKSANVDFTVDNNGLYTEPRPIGTRYLTRPL |
| AAV7 - SEQ ID NO: 504 |
| MAADGYLPDWLEDNLSEGIREWWDLKPGAPKPKANQQKQDNGRGLVLPGYKYLGPFNGLDKGEPVNAADAAALEHDKAYDQQL KAGDNPYLRYNHADAEFQERLQEDTSFGGNLGRAVFQAKKRVLEPLGLVEEGAKTAPAKKRPVEPSPQRSPDSSTGIGKKGQQPAR KRLNFGQTGDSESVPDPQPLGEPPAAPSSVGSGTVAAGGGAPMADNNEGADGVGNASGNWHCDSTWLGDRVITTSTRTWALPTYN NHLYKQISSETAGSTNDNTYFGYSTPWGYFDFNRFHCHFSPRDWQRLINNNWGFRPKKLRFKLFNIQVKEVTTNDGVTTIANNLTST IQVFSDSEYQLPYVLGSAHQGCLPPFPADVFMIPQYGYLTLNNGSQSVGRSSFYCLEYFPSQMLRTGNNFEFSYSFEDVPFHSSYAHS QSLDRLMNPLIDQYLYYLARTQSNPGGTAGNRELQFYQGGPSTMAEQAKNWLPGPCFRQQRVSKTLDQNNNSNFAWTGATKYHL NGRNSLVNPGVAMATHKDDEDRFFPSSGVLIFGKTGATNKTTLENVLMTNEEEIRPTNPVATEEYGIVSSNLQAANTAAQTQVVNN |
| QGALPGMVWQNRDVYLQGPIWAKIPHTDGNFHPSPLMGGFGLKHPPPQILIKNTPVPANPPEVFTPAKFASFITQYSTGQVSVEIEWE LQKENSKRWNPEIQYTSNFEKQTGVDFAVDSQGVYSEPRPIGTRYLTRNL |
| AAV8 - SEQ ID NO: 505 |
| MAADGYLPDWLEDNLSEGIREWWALKPGAPKPKANQQKQDDGRGLVLPGYKYLGPFNGLDKGEPVNAADAAALEHDKAYDQQL QAGDNPYLRYNHADAEFQERLQEDTSFGGNLGRAVFQAKKRVLEPLGLVEEGAKTAPGKKRPVEPSPQRSPDSSTGIGKKGQQPAR KRLNFGQTGDSESVPDPQPLGEPPAAPSGVGPNTMAAGGGAPMADNNEGADGVGSSSGNWHCDSTWLGDRVITTSTRTWALPTYN NHLYKQISNGTSGGATNDNTYFGYSTPWGYFDFNRFHCHFSPRDWQRLINNNWGFRPKRLSFKLFNIQVKEVTQNEGTKTIANNLTS TIQVFTDSEYQLPYVLGSAHQGCLPPFPADVFMIPQYGYLTLNNGSQAVGRSSFYCLEYFPSQMLRTGNNFQFTYTFEDVPFHSSYAH SQSLDRLMNPLIDQYLYYLSRTQTTGGTANTQTLGFSQGGPNTMANQAKNWLPGPCYRQQRVSTTTGQNNNSNFAWTAGTKYHL NGRNSLANPGIAMATHKDDEERFFPSNGILIFGKQNAARDNADYSDVMLTSEEEIKTTNPVATEEYGIVADNLQQQNTAPQIGTVNS QGALPGMVWQNRDVYLQGPIWAKIPHTDGNFHPSPLMGGFGLKHPPPQILIKNTPVPADPPTTFNQSKLNSFITQYSTGQVSVEIEWE LQKENSKRWNPEIQYTSNYYKSTSVDFAVNTEGVYSEPRPIGTRYLTRNL |

### BRIEF DESCRIPTION OF THE FIGURES

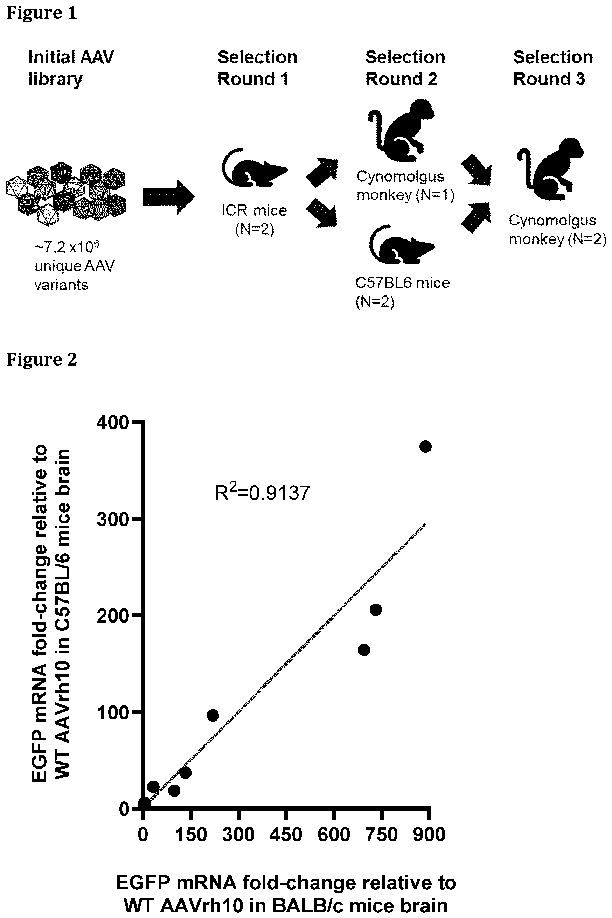

**Figure 1****.** Schematic representation of the *in vivo* strategy used for AAVrh10 directed evolution in mice and NHP.
**Figure 2****.** Correlation plot of EGFP mRNA levels in brain tissue of BALB/c (X-axis) and C57BL/6 (Y-axis) mice, measured 3 weeks after a single administration with wild-type AAVrh10 serotype and AAVrh10 engineered variants. EGFP mRNA levels are presented as fold-change relative to the wild-type AAVrh10 serotype levels. Each circle represents a distinct AAVrh10 variant. The regression line and coefficient of determination (R²) of the linear regression are shown.

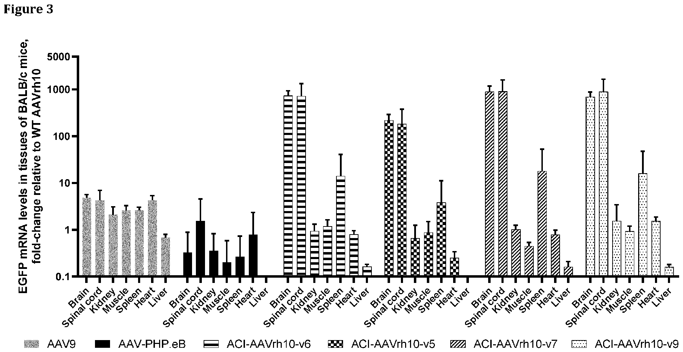

**Figure 3****.** Graphical representation of the EGFP mRNA levels in different tissues of BALB/c mice (N=4), measured 3 weeks after a single administration with wild-type (WT) AAV9 serotype, WT AAVrh10 serotype, AAV-PHP.eB and AAVrh10 variants. EGFP mRNA levels are presented as fold-change relative to the wild-type AAVrh10 serotype levels and reported as mean ± SD.

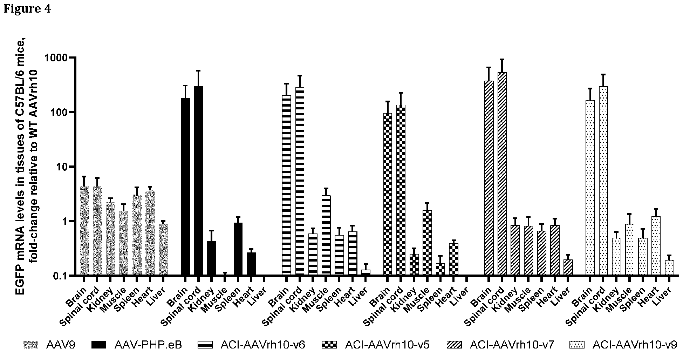

**Figure 4****.** Graphical representation of the EGFP mRNA levels in different tissues of C57BL/6 mice (N=4), measured 3 weeks after a single administration with wild-type (WT) AAV9 serotype, WT AAVrh10 serotype, AAV-PHP.eB and AAVrh10 variants. EGFP mRNA levels are presented as fold-change relative to wild-type AAVrh10 serotype and reported as mean ± SD.

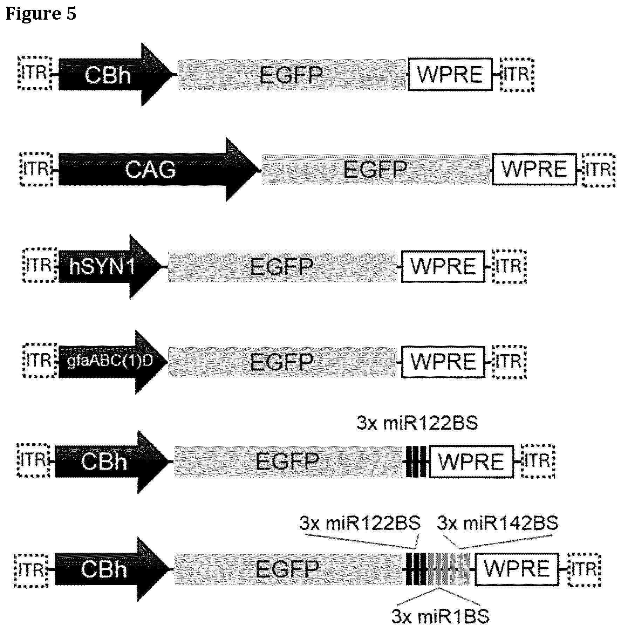

**Figure 5****.** Schematic overview of AAV expression cassettes showing EGFP gene under the control of various promoters. CBh: chicken β-actin hybrid, ITR: inverted terminal repeat, CAG: CMV early enhancer/chicken β actin, hSYN1: Human Synapsin I, gfaABC(1)D: modified GFAP promoter, WPRE: woodchuck hepatitis virus post-transcriptional response element, BS: binding site.

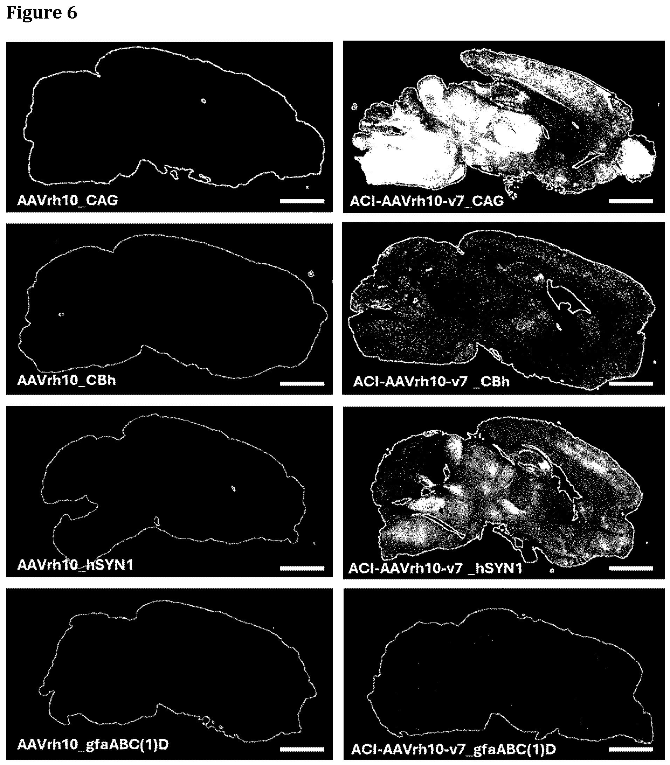

**Figure 6****.** Representative immunofluorescence images showing EGFP expression detected by immunostaining on sagittal brain sections of C57BL/6 mice injected with the vectors described in Table 9. The region of interest (ROI), delimiting the whole brain area used for EGFP signal quantification, is outlined in white. Within the ROI, EGFP signal appears in varying intensities of white. Scale (thick white line in bottom right): 2mm.

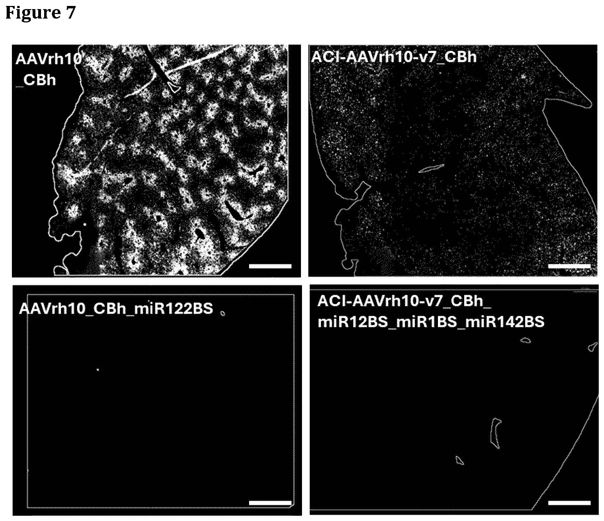

**Figure 7****.** Representative immunofluorescence images showing EGFP expression detected by immunostaining on liver sections of C57BL/6 mice injected with the vectors described in Table 9. The region of interest (ROI), delimiting the liver area used for EGFP signal quantification, is outlined in white. Within the ROI, EGFP signal appears in varying intensities of white. Scale (thick white line in bottom right): 1mm.

### EXAMPLES

To develop AAVs with enhanced brain penetration and improved tropism for the central nervous system, an engineered AAV library was generated using the AAVrh10 VP1 protein as a template, with a randomized heptamer peptide inserted to modulate capsid properties. The pool of AAV variants was selected by directed evolution through consecutive rounds of *in vivo* selection. Species cross reactivity was achieved by alternating between mice and non-human primates (NHPs) during the selection process.

### Example 1. AAV library design and generation

To improve properties of the AAVrh.10 wild-type (WT) vector, a library of randomized heptamer peptides was inserted in the variable regions (VRs) of the AAVrh10 capsid. Using the cryo-electron microscopy structures of AAVrh.10 (Protein Data Bank (PDB) reference: 6V1G), two variable regions - VR-IV (Q452-N472) and VR-VIII (V582-A597) - were identified as candidate sites for peptide insertion within the VP1 sequence of AAVrh10 (SEQ ID NO: 1). Insertion of randomized heptamer peptides was explored between positions Q589 and N590 within the VR-VIII of the VP1 protein. Briefly, a DNA plasmid library was generated by inserting a randomized 21-bp nucleotide sequences using NNK saturation mutagenesis into the Cap gene of AAVrh10. The library of Cap genes were cloned in a AAV compatible plasmid (pAAV) downstream of the AAV5 P41 promoter and the AAV2 splicing sequences as described by Deverman and colleagues (Deverman Benjamin E et al. 2016). The plasmid also included a ZsGreen reporter gene under the control of an EF1a promoter and all coding sequences were flanked by AAV2 inverted terminal repeat (ITR) sequences. The plasmid library was characterized by next generation sequencing (NGS) using a 150 bp paired-end sequencing of the randomized region in the Cap gene. Then, the AAV capsid library was produced by co-transfection of HEK293T cells with AAV library plasmids, a Rep plasmid and a helper plasmid. Viral particles were purified by caesium chloride (CsCl) gradient ultra-centrifugation. Before subjecting the virus library to a first *in vivo* selection round, the AAV library was analysed by NGS which resulted in the identification of 7'170'065 unique sequences.

### Example 2. Directed evolution of AAV capsids for enhanced CNS tropism across species

To identify AAV variants with enhanced tropism for the CNS, the AAV library was exposed to several consecutive rounds of *in vivo* selection alternating between different mice strains and non-human primates (cynomolgus monkeys) as illustrated in Figure 1. In each round of selection, animals received a single intravenous injection of an AAV library. Three to four weeks post-injection, the brain, the spinal cord and the liver of each animal were collected for genomic DNA extraction and analysed by NGS. Heptamer sequences enriched in the brain and spinal cord relative to the initial injected library were selected and subsequently synthesized as oligo pools. These sequences were then cloned into new AAV library vectors to produce AAV particles for subsequent rounds of *in vivo* selection. The method for the *in vivo* selection in mice is described below.

### In vivo selection in ICR and C57BL/6 mice

The AAV library was first injected intravenously at 5.0×10¹¹ vg/mice through the tail vein of ICR mice (selection round 1, N=2, one male and one female). For the second round of selection, C57BL/6 mice (selection round 2, N=2, one male and one female) were injected following the same procedure. Mice were euthanised 3 weeks post-injection. The brain, spinal cord, and liver were collected after perfusion with PBS (phosphate buffer saline). The genomic DNA was extracted from the collected organs and a 150 bp region of the viral DNA, including the 21-nucleotide randomized sequences, was amplified by conventional PCR. A second PCR step was performed to prepare the library for paired-end NGS.

### NGS data analysis

The prepared library was subjected to NGS analysis on a NovaSeq 6000 System. Raw Fastq files from NGS were processed by aligning each read to the AAVrh10 Cap gene containing the randomized heptamer coding sequences using the short read aligner BBMap tool developed by Bushnell et al (Bushnell et al. 2017). The 21 bp variant sequences were extracted from the mapped reads, and only DNA variants from the initial library were retained for analysis. Each DNA variant was counted, and results were normalized to the total number of reads, expressing data in reads per million (RPM). After the first round of selection, a total of ~ 690'000 AAV variants were identified in the brain and spinal cord of ICR mice and were used to generate the library for the subsequent rounds of *in vivo* selection, following the same methodology as previously described.

After the second round of *in vivo* selection performed in C57BL/6 mice, a total of 46'118 unique sequences were identified either in the brain (22'658) or in the spinal cord (37'813) with 14'358 sequences found in both organs. For each unique sequence, the tissue enrichment score was calculated by dividing the RPM in tissue sample by the RPM in the AAV library injected. In addition, a brain-to-liver ratio, representing the preferred brain transduction over the liver, was calculated by dividing the brain enrichment score by the liver enrichment score. Brain tissues from each animal were tested individually and as organ pool generating three NGS data set for each organ (i.e., one data set for C57BL/6 female brain, one for C57BL/6 male brain, and one for C57BL/6 female and male brains combined).

Only AAV variants detected in at least two out of the three NGS data sets were included in the analysis and mean enrichment scores were calculated from the three NGS analyses. The unique heptamer targeting peptides of the AAV variants were identified by NGS from the 21 bp variants sequences. Table 4 shows the targeting peptide sequences and the mean enrichment scores of the corresponding AAV variants in the brain, spinal cord and liver of C57BL76 mice for the AAV variants with a brain enrichment score of at least 50.

**Table 4. Enrichment scores of AAV variants from the second round of in vivo selection performed in C57BL/6 mice**

| **AAV variant targeting peptide** | | **Enrichment score** | | | **Brain-to-liver ratio** |
|---|---|---|---|---|---|
| **Amino acid sequence** | **SEQ ID NO** | **Brain** | **Spinal cord** | **Liver** | |
| WTTTSGY | 4 | 515.7 | 132.1 | 0.6 | 866.8 |
| DSRLLLP | 5 | 308 | 262.4 | 0.2 | 1273.1 |
| SHSPEFK | 6 | 270.4 | 260.3 | 0.4 | 645.2 |
| YEVGGTL | 7 | 267.6 | 110.5 | 0.9 | 281.7 |
| THPNVNP | 8 | 220.8 | 0.1 | 1.3 | 173.7 |
| GGLTPPY | 9 | 205.9 | 212.4 | 0.2 | 1219.0 |
| SNDIKEK | 10 | 195.4 | 3.1 | 1.1 | 181.9 |
| SNLTGLL | 11 | 189.6 | 0.1 | 0.8 | 252.2 |
| SGNDPSA | 12 | 159.1 | 0 | 0.7 | 213.3 |
| SNQVASS | 13 | 153 | 0 | 0.5 | 303.4 |
| INSTKGN | 14 | 140 | 0 | 1 | 145.2 |
| SNTVEKK | 15 | 139.6 | 108.9 | 27.9 | 5.0 |
| GNQVKTL | 16 | 139.4 | 0 | 0.6 | 246.0 |
| INNTKTK | 17 | 137.1 | 0 | 0.7 | 187.7 |
| TEIVTNK | 18 | 134.9 | 0 | 0.5 | 265.6 |
| TNRTREG | 19 | 133.8 | 2.9 | 0 | N.D. |
| SNL TRNL | 20 | 131.2 | 8.4 | 0.7 | 180.7 |
| TNETRST | 21 | 126.3 | 0 | 0.2 | 726.9 |
| YDRSATG | 22 | 125.6 | 0 | 1.3 | 94.0 |
| LTHTTLK | 23 | 123.3 | 0 | 1.5 | 81.8 |
| ENRTRTI | 24 | 121.7 | 0 | 0.3 | 370.3 |
| IISNGQA | 25 | 121.3 | 0.1 | 0.3 | 429.5 |
| TNMAREW | 26 | 117.7 | 0 | 0.1 | 1990.2 |
| HTNLVRT | 27 | 115.4 | 0 | 0.3 | 335.3 |
| SNVTRTS | 28 | 114.9 | 1.8 | 1.1 | 107.5 |
| SDDNRTR | 29 | 109.7 | 6.6 | 1.3 | 84.5 |
| TNAIRPV | 30 | 108.4 | 15.2 | 0.2 | 563.2 |
| TNMTSKP | 31 | 106.4 | 0 | 0.2 | 594.0 |
| DSNQTPK | 32 | 105.4 | 0 | 0.7 | 158.7 |
| ENNRYPN | 33 | 103.2 | 9.1 | 0.7 | 153.6 |
| RNDTKEP | 34 | 102.2 | 0 | 1.5 | 68.7 |
| WTKSSGY | 35 | 100.6 | 61.8 | 0.1 | 825.9 |
| IGNSSKQ | 36 | 99.2 | 0.1 | 1.6 | 61.3 |
| ANNANTA | 37 | 98.2 | 0 | 0.9 | 113.7 |
| MTSNITR | 38 | 97.9 | 0 | 1.4 | 71.8 |
| TNIANAK | 39 | 97.3 | 11.3 | 0.8 | 129.1 |
| SNNTAMP | 40 | 95.5 | 0 | 1.9 | 49.2 |
| NNTVKVA | 41 | 93.1 | 2.6 | 1.3 | 72.3 |
| FNVNRGS | 42 | 92.9 | 0 | 0.5 | 189.0 |
| ETNRTRG | 43 | 92.6 | 20.6 | 0.6 | 164.2 |
| ANRTRET | 44 | 92.6 | 29 | 0.3 | 313.1 |
| ANNTRSA | 45 | 91.7 | 14 | 1 | 88.7 |
| TKKGSTS | 46 | 91.3 | 6.3 | 1.3 | 71.6 |
| SYGEAVT | 47 | 90.9 | 0.1 | 0.4 | 212.0 |
| ANAVRLQ | 48 | 90.5 | 0 | 0.3 | 297.1 |
| EENKTRP | 49 | 90.2 | 0 | 0.2 | 538.4 |
| DNTTRSI | 50 | 89.2 | 0 | 0.6 | 142.4 |
| QNETRVF | 51 | 88.5 | 0 | 1 | 90.3 |
| EQNNSTK | 52 | 88.4 | 0.1 | 2.1 | 42.1 |
| FNENRAK | 53 | 87.3 | 9.4 | 0.8 | 107.8 |
| SNNKYTS | 54 | 86.1 | 0 | 0.7 | 119.3 |
| SNTTSTT | 55 | 85.4 | 0 | 1.6 | 53.5 |
| ISNVTRT | 56 | 82.9 | 2.5 | 0.6 | 133.5 |
| SNIITSH | 57 | 82.6 | 0 | 0.8 | 102.9 |
| DKKELSA | 58 | 82.2 | 0 | 1.6 | 50.7 |
| NLNSTRL | 59 | 82 | 26 | 0.6 | 144.9 |
| SHSPTFR | 60 | 81.9 | 19.2 | 0.3 | 316.5 |
| SLNNTRT | 61 | 81.7 | 33.3 | 1.2 | 67.5 |
| SNTITGV | 62 | 80.9 | 0 | 1.5 | 54.3 |
| SNDMRQQ | 63 | 80.6 | 0.1 | 1.5 | 55.6 |
| SNLTSKS | 64 | 79.9 | 17.7 | 0.6 | 132.8 |
| TNRTSQP | 65 | 79.7 | 3.3 | 0.7 | 116.1 |
| TNTTRTV | 66 | 79.7 | 8 | 0.4 | 198.2 |
| SNSIESR | 67 | 78.9 | 0 | 0.8 | 102.1 |
| ANLISSR | 68 | 77 | 0 | 2.2 | 35.0 |
| LAQNLTR | 69 | 76.8 | 0 | 0.1 | 567.8 |
| INTVKSY | 70 | 75.7 | 34.9 | 0.2 | 426.8 |
| NNNESRP | 71 | 74.8 | 0 | 3.1 | 23.8 |
| YKVGLNT | 72 | 73 | 0 | 0.9 | 78.8 |
| SNRNNTI | 73 | 72.9 | 0 | 1.1 | 68.3 |
| HGNSSRS | 74 | 72.8 | 0 | 0.8 | 91.2 |
| PDNPRTK | 75 | 72.3 | 0 | 0.5 | 141.3 |
| LSANTVR | 76 | 71.9 | 3.4 | 0.4 | 195.8 |
| NGNRITA | 77 | 71.6 | 9.5 | 1.4 | 49.6 |
| INNTKSI | 78 | 71.1 | 2.9 | 0.5 | 137.0 |
| TNRTNTH | 79 | 71 | 6.6 | 0.8 | 84.9 |
| RAGKNIA | 80 | 70.2 | 0 | 1.7 | 42.4 |
| VMNSTKE | 81 | 70.1 | 38.9 | 0.9 | 79.3 |
| SNSVSGG | 82 | 69.8 | 0.1 | 0.4 | 168.0 |
| LNASSEQ | 83 | 69.7 | 0 | 0.4 | 175.3 |
| LPNTTRV | 84 | 69.5 | 42.3 | 0.6 | 122.6 |
| ENMTRSL | 85 | 69.2 | 0 | 0.4 | 159.2 |
| HSNQTRL | 86 | 69 | 3.5 | 0.7 | 100.2 |
| SNTIRAT | 87 | 68.9 | 3.5 | 0.7 | 102.9 |
| TNNLVKS | 88 | 68.9 | 2.9 | 1 | 71.5 |
| LENKTRA | 89 | 68.8 | 1.8 | 0.4 | 191.5 |
| GNATRTL | 90 | 68.4 | 4.1 | 0.4 | 171.5 |
| YSNRTSI | 91 | 68.3 | 13 | 0.8 | 81.4 |
| SNDVRSH | 92 | 68.3 | 0 | 0.4 | 153.1 |
| VNVVGVR | 93 | 68 | 23.9 | 0.5 | 140.9 |
| EPSLLIP | 94 | 67.9 | 4.1 | 0 | N.D. |
| SNRATHY | 95 | 67.2 | 3.4 | 1.5 | 44.3 |
| NENRSSI | 96 | 67.2 | 0 | 0.5 | 132.4 |
| GSNHTKT | 97 | 66.4 | 2.4 | 0.8 | 83.2 |
| TNASKQK | 98 | 66.4 | 0 | 0.2 | 321.4 |
| SNRTATR | 99 | 66 | 0 | 2.4 | 28 |
| AGNSTKT | 100 | 66 | 38.1 | 0.2 | 265.4 |
| WSPTNGF | 101 | 65.7 | 34.9 | 0.1 | 513.7 |
| TINTTRA | 102 | 65.7 | 78 | 1.2 | 56.4 |
| DINRTPR | 103 | 64.9 | 4.1 | 0.5 | 131.4 |
| YSLKGIH | 104 | 64.8 | 32.7 | 0.2 | 289.5 |
| SNTISRA | 105 | 64.6 | 0 | 0.6 | 112.2 |
| SNRIQQV | 106 | 64.4 | 6.3 | 0.4 | 179.7 |
| SNTIRQL | 107 | 64.1 | 0 | 0.4 | 168.7 |
| INNTRNF | 108 | 63.9 | 3.5 | 0.4 | 173.2 |
| SASSIGE | 109 | 63.9 | 0 | 0.7 | 88.2 |
| TNITRSG | 110 | 63.3 | 0 | 0.1 | 662.4 |
| SPNHVRY | 111 | 63.3 | 0 | 0.1 | 849.3 |
| IPNATTR | 112 | 63 | 10.4 | 0.2 | 261.6 |
| NINHTKT | 113 | 62.8 | 42.7 | 1.4 | 44.8 |
| SNATRSG | 114 | 62.5 | 0 | 0.2 | 360.4 |
| TNYAKSG | 115 | 62.2 | 18 | 0.5 | 126.0 |
| SNRTTLH | 116 | 62 | 6.1 | 0.6 | 103.8 |
| TNTITPK | 117 | 61.9 | 36 | 0.6 | 99.9 |
| SNQTSHM | 118 | 61.8 | 0 | 0.4 | 150.5 |
| SANQTRS | 119 | 61.5 | 4.3 | 0.4 | 167.3 |
| SNATNTT | 120 | 60.8 | 0 | 0.2 | 250.1 |
| SNHSRTN | 121 | 60.7 | 0 | 1.5 | 39.7 |
| SNKSASI | 122 | 60.6 | 0.1 | 2.1 | 29.5 |
| TNTTRIP | 123 | 60.2 | 5.3 | 0.6 | 104.7 |
| GNSTSTT | 124 | 59.8 | 0 | 1.1 | 56.7 |
| EGNRVIS | 125 | 59.4 | 15.4 | 0.4 | 132.2 |
| SNITVER | 126 | 59.3 | 0 | 2 | 30.1 |
| TNVSNKT | 127 | 59 | 8.1 | 0.5 | 110.1 |
| ENGTRRQ | 128 | 58.8 | 0 | 1.1 | 55.9 |
| STNSTRG | 129 | 58.5 | 36.8 | 0.4 | 148.0 |
| QNNTRSS | 130 | 58.4 | 17.5 | 0.7 | 81.3 |
| MNIVAKG | 131 | 58.4 | 0 | 1.4 | 41.4 |
| ESNTTRT | 132 | 58.3 | 0.1 | 0 | 1484.3 |
| SNITRGM | 133 | 58.3 | 4.8 | 0.4 | 147.3 |
| SSSAQTI | 134 | 58.2 | 0 | 1.1 | 51.2 |
| GEASTKE | 135 | 58 | 0 | 0.1 | 525.2 |
| SPNQVRS | 136 | 58 | 0 | 0.5 | 115.5 |
| SNRTNIA | 137 | 57.5 | 29.9 | 0.5 | 115.5 |
| SNVTRST | 138 | 57.3 | 46.5 | 0.8 | 67.7 |
| QNTNIIK | 139 | 57.2 | 2.1 | 1.9 | 29.4 |
| WSTSKGY | 140 | 56.8 | 108.8 | 0 | N.D. |
| TASTVDK | 141 | 56.8 | 7.5 | 1.8 | 31.5 |
| LPNRTIQ | 142 | 56.7 | 0 | 0.4 | 146.8 |
| TGTSGST | 143 | 56.5 | 0 | 1.1 | 51.7 |
| VTNRIPS | 144 | 56.2 | 0 | 0.1 | 406.9 |
| MLNSLRG | 145 | 56 | 0 | 0.9 | 65.6 |
| SNSTKLQ | 146 | 55.9 | 2 | 0.3 | 176.0 |
| SNNNTRS | 147 | 55.7 | 0.1 | 1 | 55.7 |
| HDHVRQD | 148 | 55.6 | 0 | 0.7 | 75.6 |
| GNSLRAI | 149 | 54.6 | 14.8 | 0.3 | 183.3 |
| SNNTTHQ | 150 | 54.4 | 3.3 | 0.8 | 68.5 |
| SNTSRTM | 151 | 53.8 | 2.9 | 1.5 | 36.9 |
| GTVNDIK | 152 | 53.7 | 0 | 0.7 | 71.8 |
| TQNITRQ | 153 | 53.6 | 9.4 | 0.5 | 113.5 |
| ANNTTNK | 154 | 53.3 | 11.2 | 3.9 | 13.8 |
| HPNATKV | 155 | 53.1 | 3.5 | 0.1 | 467.7 |
| SNRTDPS | 156 | 52.7 | 0 | 1.6 | 32.8 |
| SNRANSP | 157 | 52.7 | 0 | 1 | 50.9 |
| TYPGTTL | 158 | 52.6 | 0 | 0.8 | 63.5 |
| ENVSRGR | 159 | 52.6 | 38 | 1.9 | 27.3 |
| NNSARSA | 160 | 52.4 | 8.4 | 1.2 | 43.4 |
| SLNSVHR | 161 | 52.3 | 0 | 0.4 | 146.4 |
| VNNTKYL | 162 | 52.1 | 8.4 | 0.2 | 246.2 |
| SNNAKIN | 163 | 52 | 0 | 0.8 | 68.1 |
| SNVSRSY | 164 | 51.5 | 0 | 0.5 | 95.7 |
| VNAVHTR | 165 | 51.3 | 38.6 | 0.7 | 71.9 |
| ETNRLKT | 166 | 51.1 | 27.9 | 0.6 | 89.6 |
| TNLSNTI | 167 | 50.9 | 0 | 1.2 | 41.9 |
| TNLTQSS | 168 | 50.6 | 0 | 0.8 | 61.8 |
| TNNARAA | 169 | 50.4 | 5.4 | 0.6 | 78.4 |
| TNRTTDH | 170 | 50.4 | 0 | 0.2 | 253.4 |
| ANQVRTT | 171 | 50.3 | 0 | 0.3 | 167.0 |
| SSSNSVR | 172 | 50.2 | 36.8 | 0.2 | 283.2 |
| ITNSVKD | 173 | 50.2 | 7.9 | 0.8 | 62.4 |
| INSVSKS | 174 | 50.2 | 5.6 | 1.2 | 42.9 |
| TNTTKAS | 175 | 50.1 | 0.1 | 0.2 | 202 |
| GNETRAK | 176 | 50.1 | 14.4 | 0.4 | 129 |
| YNLNTTK | 177 | 50.1 | 4.7 | 0.2 | 239.2 |

| | | | | | |
|---|---|---|---|---|---|
| *N.D.: AA V variant targeting peptide not detected in liver.* | | | | | |

### In vivo selection in non-human primates

An orthogonal cross species selection was performed in cynomolgus monkey with the same AAV library used for the second round of selection in C57BL/6 mice. The AAV library was administered to a young male cynomolgus monkey (N=1, 1 year of age) by saphenous vein injection at 6×10¹² vg/kg. Three weeks post-injection, tissues were collected after perfusion with PBS and the genomic DNA was extracted from various regions of the brain and the spinal cord. A 150 bp region of the viral DNA, including the 21-nucleotide randomized sequences, was amplified by conventional PCR. A second PCR step was performed to prepare the library for paired-end NGS. Analysis was performed as previously described to identify AAV variants with enhanced CNS transduction properties. In sum, 12'545 unique AAV variant sequences were identified in the brain of the cynomolgus monkey, 4'252 in the spinal cord, and 2'301 in both the brain and spinal cord, for a total of 14'496 unique AAV variant sequences.

A last round of directed evolution in NHP was subsequently performed including 1) all unique AAV variants identified from the selection in NHP and 2) all unique AAV variants presenting a brain enrichment score above 1 after the second round of selection in C57BL/6 mice, which combined resulted in 55'675 unique AAV variants. To improve the quality of the NGS data analysis, each AAV variant amino acid sequences was encoded by three different synonymous DNA sequences (i.e., 167'025 unique DNA sequences). The generation of the new AAV library was performed using synthetic oligo pools as previously described. For the last round of directed evolution, cynomolgus monkeys (N=2, 2 years of age, one male and one female) were injected with the new library at 4×10¹² vg/kg. Three weeks post-injection, tissues were collected after perfusion with PBS. Genomic DNA was extracted from different brain sections, the spinal cord and the liver. The viral DNA was amplified and analysed by NGS as previously described. High-performing AAV capsid variants were selected considering the reproducibility among synonymous DNA sequences and sample replicates, the AAV production efficiency, the brain enrichment scores, and the brain-to-liver ratio (brain enrichment score/liver enrichment score). Table 5 shows the AAV variants targeting peptide sequence and the mean enrichment scores of the AAV variants in the brain, spinal cord and liver of NHP.

**Table 5. Enrichment scores of AAV variants from the second round of in vivo selection performed in NHP**

| **AAV variant targeting peptide** | | **Enrichment score** | | | **Brain-to-liver ratio** |
|---|---|---|---|---|---|
| **Amino acid sequence** | **SEQ ID NO** | **Brain** | **Spinal cord** | **Liver** | |
| PQNSARV | 178 | 52.8 | 0.8 | 0.8 | 70.2 |
| NNTKQGY | 179 | 49.5 | 0 | 0.4 | 131.2 |
| SNLTRHT | 180 | 47.1 | 1.2 | 0.1 | 736.4 |
| DNEVRSR | 181 | 35.4 | 12.5 | 0.6 | 58 |
| SNQVNRV | 182 | 35 | 0 | 0.2 | 187.5 |
| YVGSAKQ | 183 | 34.3 | 0 | 0.5 | 74.8 |
| SNISRPV | 184 | 33.3 | 0 | 0.7 | 48 |
| TNGTKTL | 185 | 30.5 | 2.2 | 0.3 | 105.1 |
| QSNSVKP | 186 | 30.2 | 1.6 | 0.3 | 106.7 |
| NNSVRPS | 187 | 29.4 | 0 | 0.2 | 135.8 |
| ENNTKPT | 188 | 28.4 | 0 | 0.1 | 334.6 |
| SNSVSSI | 189 | 27.6 | 0.8 | 0.3 | 98.4 |
| PGNTIRQ | 190 | 26.6 | 0.4 | 0.5 | 55.9 |
| SNLVVQR | 191 | 25.6 | 0 | 0.5 | 56.6 |
| SNNTKTI | 192 | 25.1 | 0 | 0.2 | 105.4 |
| ENSAKQK | 193 | 25 | 0 | 0.8 | 30.2 |
| SNLVSRA | 194 | 23 | 0.2 | 0.2 | 122.1 |
| MNGVRAA | 195 | 22.6 | 0.4 | 0.6 | 35.4 |
| DTNVTRA | 196 | 22.2 | 0.4 | 0.3 | 83.2 |
| SNIVAAS | 197 | 22.1 | 0 | 0 | 830.5 |
| GNHNTIR | 198 | 22 | 1.9 | 0.5 | 46.3 |
| SNVVKSL | 199 | 21.6 | 0.8 | 0.3 | 76.1 |
| QDNVIRT | 200 | 21 | 2.3 | 0.3 | 62.8 |
| SNLIKNT | 201 | 20.7 | 1.3 | 0.2 | 88.1 |
| ENRARAA | 202 | 20.4 | 2.6 | 0.7 | 27.4 |
| SNVSRAS | 203 | 20.4 | 1.8 | 0.4 | 54.6 |
| NNTVRPL | 204 | 20.4 | 2.8 | 0.2 | 94.1 |
| FNGTRGT | 205 | 20.2 | 0.4 | 0.5 | 41 |
| GNSVREQ | 206 | 19.6 | 1.3 | 0.2 | 101.4 |
| VNTVRGL | 207 | 19.5 | 1.5 | 0.2 | 106.3 |
| SDNHTKI | 208 | 19.3 | 3.4 | 0.4 | 51 |
| ENRTSIR | 209 | 19.2 | 1.5 | 0.5 | 37.9 |
| VVNTTQR | 210 | 19 | 1.5 | 0.4 | 44.3 |
| TNLTHPK | 211 | 19 | 0.2 | 0.3 | 64.2 |
| SNEARVH | 212 | 18.8 | 2 | 0.3 | 56.8 |
| SNLTGRS | 213 | 18.6 | 1.3 | 0.3 | 62.2 |
| GHNAIRN | 214 | 18.3 | 0 | 0.9 | 19.5 |
| GNQVRSS | 215 | 18.2 | 0.8 | 0.4 | 50.8 |
| SNRTYSD | 216 | 17.8 | 0.8 | 0.1 | 201.4 |
| HTNASRS | 217 | 17.3 | 0.1 | 0.4 | 47.5 |
| SNSVKAN | 218 | 16.6 | 1.2 | 0.4 | 47.1 |
| ENRTKHT | 219 | 16.3 | 0 | 0.2 | 74.9 |
| IGNSVRQ | 220 | 16.2 | 1.9 | 0.2 | 71.6 |
| ELNSSRS | 221 | 16.2 | 1.3 | 0.3 | 60.7 |
| LSNSVKN | 222 | 16.2 | 1.7 | 0.3 | 50 |
| TNRVTSI | 223 | 16.1 | 0 | 0.3 | 46.1 |
| ESNHIRS | 224 | 16 | 0.4 | 0.3 | 56.1 |
| MNVTRPA | 225 | 15.8 | 0.2 | 0.3 | 55.1 |
| SNLSKQH | 226 | 15.5 | 0 | 0.1 | 115.4 |
| QDNRIHQ | 227 | 15.4 | 1.1 | 0.6 | 27.9 |
| TNLVNHK | 228 | 15.4 | 0.5 | 0.3 | 44.9 |
| VKHETGR | 229 | 15.2 | 0.6 | 0.3 | 45.5 |
| SNTVRPT | 230 | 15.2 | 1.4 | 0.3 | 55.5 |
| IDNKIRT | 231 | 15.2 | 0.1 | 0.5 | 30.2 |
| ELNSIKR | 232 | 15.1 | 0 | 0.3 | 44.6 |
| GKNQPVR | 233 | 15 | 0 | 1 | 14.5 |
| TNKTSGA | 234 | 14.9 | 0.3 | 0.2 | 68.8 |
| SNRTSAL | 235 | 14.9 | 0.8 | 0.2 | 68.2 |
| HGNLTRT | 236 | 14.9 | 2.3 | 0.4 | 36.3 |
| DNNVRGG | 237 | 14.9 | 2.9 | 0.2 | 60 |
| VTNQTKP | 238 | 14.4 | 1.1 | 0.2 | 58.3 |
| APHANSR | 239 | 14.4 | 0 | 0.6 | 23.2 |
| TNTTKVT | 240 | 14.3 | 1.4 | 0.3 | 50.3 |
| ETNRTAK | 241 | 14.2 | 2.1 | 0.8 | 18.8 |
| SNLTREK | 242 | 14.2 | 0.7 | 0.3 | 48.8 |
| HLNSVRE | 243 | 14.1 | 0.2 | 0.2 | 61.6 |
| NSNSVRA | 244 | 14 | 0.8 | 0.1 | 111.9 |
| GNRSSSH | 245 | 14 | 1.3 | 0.6 | 22.7 |
| SSHNSVK | 246 | 14 | 1.7 | 0.4 | 33.3 |
| GNGVRGT | 247 | 14 | 1.2 | 0.7 | 18.9 |
| SNTIKSS | 248 | 13.6 | 2.5 | 0.4 | 32 |
| SNRIQHT | 249 | 13.4 | 1.6 | 0.4 | 35.9 |
| TNLIKSN | 250 | 13.3 | 0.9 | 0.3 | 50.5 |
| RSNSIHS | 251 | 13.3 | 0.6 | 0.5 | 28 |
| GNRTTQS | 252 | 13.1 | 3.5 | 1 | 13.8 |
| ANSVRAN | 253 | 13 | 0.8 | 0.3 | 47.2 |
| LQNQTKL | 254 | 12.8 | 0.6 | 0.3 | 37.5 |
| ANRANAI | 255 | 12.8 | 0.3 | 0.4 | 34.7 |
| TNRVGTT | 256 | 12.7 | 1.3 | 0.4 | 28.6 |
| LNSVKTP | 257 | 12.5 | 1.1 | 0.4 | 29.2 |
| NTNATRS | 258 | 12.5 | 2.2 | 0.3 | 41.4 |
| STTRSDA | 259 | 12.4 | 1.3 | 0.2 | 63 |
| ANHARGV | 260 | 12.4 | 0.7 | 0.3 | 39.5 |
| FNHTKSI | 261 | 12.4 | 1.1 | 0.3 | 46.9 |
| ESNLARR | 262 | 12.4 | 1 | 0.8 | 15.5 |
| ASNSVRS | 263 | 12.3 | 0.6 | 0.3 | 35.5 |
| STNKIRD | 264 | 11.8 | 2.2 | 0.8 | 15.2 |
| NNTVRHT | 265 | 11.7 | 0.9 | 0.3 | 36.5 |
| TLNATTR | 266 | 11.7 | 0 | 0.4 | 33.1 |
| GNATRQQ | 267 | 11.5 | 1.3 | 0.3 | 35.2 |
| SNDVRQQ | 268 | 11.5 | 1.5 | 0.1 | 92.7 |
| HNATKLS | 269 | 11.4 | 0.4 | 0.4 | 32.3 |
| VNSVRGE | 270 | 11.3 | 1.5 | 0.2 | 64.6 |
| QNQIRPS | 271 | 11.3 | 2.1 | 0.6 | 18.1 |
| DNQVRAK | 272 | 11.1 | 1.2 | 0.6 | 18.3 |
| TGNAVRS | 273 | 11.1 | 1.3 | 0.3 | 36.9 |
| TNEARAR | 274 | 10.8 | 1.3 | 0.4 | 27 |
| SGNHIRT | 275 | 10.6 | 0.6 | 0.6 | 16.5 |
| TNSIRGG | 276 | 10.5 | 1 | 0.3 | 41.7 |
| VNATQRT | 277 | 10.4 | 0.5 | 0.5 | 19.4 |
| TNKTAPT | 278 | 10.2 | 1.8 | 0.4 | 26.4 |
| SNSVGKI | 279 | 10.1 | 1.3 | 0.1 | 83.7 |
| SNKSQGQ | 280 | 10.1 | 0.1 | 0.8 | 12.1 |
| DQNLTRR | 281 | 10 | 1.8 | 0.4 | 24.8 |
| GNDTRKA | 282 | 9.9 | 0 | 0.3 | 38.3 |
| SNTVRHD | 283 | 9.8 | 1.5 | 0.2 | 40.1 |
| ANTIRSN | 284 | 9.7 | 1.4 | 0.4 | 23.4 |
| GNVVAAR | 285 | 9.6 | 2.6 | 0.4 | 26.2 |
| MNKVSAI | 286 | 9.5 | 0 | 0.3 | 29.3 |
| TTNSVKT | 287 | 9.5 | 0.3 | 0.2 | 46.5 |
| ESNTTRQ | 288 | 9.5 | 3.3 | 0.1 | 130.3 |
| GVNVTKP | 289 | 9.4 | 1.4 | 0.2 | 47.1 |
| SNSSKEY | 290 | 9.3 | 1.8 | 0.1 | 68.8 |
| SLNRIHS | 291 | 9.3 | 0 | 0.4 | 21.8 |
| INNVRSQ | 292 | 9.3 | 0.7 | 0.3 | 29.9 |
| SNNAVVK | 293 | 9.3 | 0.4 | 0.3 | 37.1 |
| SNSSHAQ | 294 | 9.3 | 0 | 0.3 | 34.7 |
| NNGTRTV | 295 | 9.3 | 0.2 | 0.5 | 19 |
| ENGVRRI | 296 | 9.3 | 1 | 0.8 | 12.1 |
| SNNHTKT | 297 | 9.1 | 2 | 0.3 | 27.2 |
| TNSAHVK | 298 | 9.1 | 0.5 | 0.3 | 26.2 |
| SQNHVKS | 299 | 9 | 3.2 | 0.4 | 22.9 |
| SNGIRSN | 300 | 8.9 | 0.6 | 0.6 | 16 |
| SNNSKHM | 301 | 8.9 | 0 | 0.2 | 39.1 |
| WDNHTRT | 302 | 8.9 | 0 | 0.2 | 51.3 |
| TNTASLR | 303 | 8.9 | 0.2 | 0.3 | 32.9 |
| EINHIKN | 304 | 8.9 | 2.1 | 0.3 | 26.2 |
| TNTTRVG | 305 | 8.8 | 0.6 | 0.1 | 69.9 |
| TNKTLSS | 306 | 8.7 | 0.7 | 0.5 | 18.4 |
| SNVTRQN | 307 | 8.7 | 0.7 | 0.3 | 25.3 |
| TNRIATP | 308 | 8.5 | 0.4 | 0.6 | 14.1 |
| TNSSKSA | 309 | 8.4 | 1 | 0.2 | 50.4 |
| QSNHTAP | 310 | 8.4 | 0 | 0.5 | 15.8 |
| AANSVKQ | 311 | 8.3 | 3.2 | 0.4 | 21.1 |
| DNVVRPR | 312 | 8.3 | 0.7 | 0.5 | 15.9 |
| DTNRTSR | 313 | 8.2 | 0 | 0.6 | 12.7 |
| EGNAVRG | 314 | 8.2 | 1.2 | 0.1 | 62 |
| DGNGVKQ | 315 | 8.1 | 0.7 | 0.2 | 35.2 |
| SNSATIK | 316 | 8.1 | 0 | 0.4 | 19.1 |
| DVNTVRV | 317 | 8.1 | 1.6 | 0.3 | 27.9 |
| RENTVRT | 318 | 8.1 | 0.7 | 0.4 | 19 |
| EETKIPR | 319 | 8.1 | 0.9 | 0.2 | 36.5 |
| ARNETRS | 320 | 8.1 | 0.7 | 0.6 | 14.1 |
| ANQTQSR | 321 | 8 | 0.7 | 0.6 | 13.6 |
| SNQSRDR | 322 | 8 | 0.5 | 0.6 | 13.7 |
| SNTIKGE | 323 | 8 | 2 | 0.5 | 16.4 |
| SNRTTPT | 324 | 8 | 1.4 | 0.3 | 28.3 |
| INNRAPQ | 325 | 8 | 1.8 | 0.6 | 12.9 |
| GNRISTQ | 326 | 8 | 2.1 | 0.6 | 13.3 |
| SNSVSHR | 327 | 7.9 | 1.4 | 0.2 | 50 |
| DNKIVRT | 328 | 7.9 | 0.1 | 0.2 | 38.1 |
| TNLSNAS | 329 | 7.9 | 0 | 0.1 | 63.4 |
| NANAIKQ | 330 | 7.8 | 3.2 | 0.3 | 30.5 |
| SNTIRGG | 331 | 7.8 | 0.6 | 0.3 | 25.6 |
| NNNLIRD | 332 | 7.8 | 0 | 0.2 | 38.1 |
| SINHTRA | 333 | 7.8 | 1.3 | 0.2 | 39.1 |
| SNRTSAT | 334 | 7.6 | 1 | 0.3 | 30.2 |
| DNRVQAK | 335 | 7.6 | 0.5 | 0.5 | 14.7 |
| SNKAHST | 336 | 7.6 | 0.7 | 0.4 | 17.8 |
| SNTVGHK | 337 | 7.5 | 1.4 | 0.4 | 18.4 |
| GNVIRQL | 338 | 7.5 | 1 | 0.5 | 14.2 |
| HNTTKTG | 339 | 7.4 | 0.7 | 0.2 | 31.6 |
| VNAVKSN | 340 | 7.4 | 1.1 | 0.3 | 22 |
| DSNRNKI | 341 | 7.3 | 0.6 | 0.1 | 67.6 |
| SNNVRPT | 342 | 7.3 | 0.6 | 0.2 | 31.6 |
| GNNATRT | 343 | 7.2 | 1.4 | 0.5 | 13.3 |
| TNTIQSR | 344 | 7.2 | 1.4 | 0.5 | 15.6 |
| TNVTITK | 345 | 7.1 | 1.5 | 0.3 | 21.9 |
| TNTVKSN | 346 | 7.1 | 0.9 | 0.3 | 26.9 |
| VRPSHER | 347 | 7.1 | 1.2 | 0.4 | 16 |
| SNTIRPS | 348 | 7.1 | 1.1 | 0.3 | 23.8 |
| SNTTRAH | 349 | 7.1 | 0.1 | 0.1 | 78.3 |
| SANSVKN | 350 | 7 | 1.8 | 0.5 | 13.8 |
| MPNTIKL | 351 | 7 | 0.4 | 0.3 | 23.9 |
| GNSAISI | 352 | 7 | 1.3 | 0.2 | 29 |
| NANSIKN | 353 | 6.9 | 3.3 | 0.4 | 18.3 |
| SLNSTKP | 354 | 6.9 | 1 | 0.1 | 105.4 |
| TNAISGS | 355 | 6.9 | 0 | 0.3 | 22.4 |
| VNTTKLS | 356 | 6.9 | 1.3 | 0.2 | 30.6 |
| SNRTSTI | 357 | 6.8 | 0.5 | 0.3 | 26.6 |
| SNITRTM | 358 | 6.8 | 1.6 | 0.3 | 25.3 |
| SANDIKT | 359 | 6.8 | 0.8 | 0.2 | 42.3 |
| GNSIRSG | 360 | 6.8 | 0.5 | 0.2 | 31.4 |
| TNESRAK | 361 | 6.7 | 0.5 | 0.4 | 16.2 |
| TNTARSG | 362 | 6.7 | 1.1 | 0.7 | 9.5 |
| SNNVPSR | 363 | 6.7 | 0.6 | 0.4 | 15.9 |
| EANAVKT | 364 | 6.7 | 0.5 | 0.3 | 25.4 |
| SNKSNAS | 365 | 6.7 | 0.6 | 0.6 | 11.5 |
| RSNATMD | 366 | 6.7 | 0 | 0.4 | 16.3 |
| TNLSRST | 367 | 6.7 | 0.3 | 0.4 | 16.4 |
| SNAVSRA | 368 | 6.5 | 11.6 | 0.3 | 22.5 |
| SNYVHSA | 369 | 6.4 | 1.8 | 0.3 | 18.3 |
| SNVSSGK | 370 | 6.4 | 1.1 | 0.6 | 11.3 |
| SNTVGKG | 371 | 6.4 | 2.1 | 0.6 | 11.1 |
| TNGTRSA | 372 | 6.4 | 2 | 0.4 | 17.7 |
| TNSITAL | 373 | 6.4 | 0 | 0.1 | 49 |
| ANTIKGV | 374 | 6.4 | 1.4 | 0.3 | 25 |
| SNGTKIA | 375 | 6.3 | 0.7 | 0.3 | 20.9 |
| ANNTRSA | 45 | 6.3 | 1.7 | 0.1 | 42.6 |
| NNRVSNH | 376 | 6.2 | 1.8 | 0.5 | 13.5 |
| DNQTRKP | 377 | 6.1 | 0.6 | 0.4 | 13.6 |
| TNVTRSS | 378 | 6.1 | 0.4 | 0.2 | 39.7 |
| QSNTIKA | 379 | 6.1 | 2 | 0.4 | 16.6 |
| TNEIKQF | 380 | 6.1 | 0.3 | 0.3 | 18.3 |
| QSNSVKS | 381 | 6.1 | 3.1 | 0.4 | 15.6 |
| PDNSVKI | 382 | 6.1 | 0 | 0.5 | 12.3 |
| TNDSRTH | 383 | 6.1 | 2.4 | 0.2 | 29.4 |
| TNHTSSR | 384 | 6 | 1 | 0.2 | 26 |
| TSTNYTK | 385 | 6 | 1.9 | 0.3 | 22.6 |
| SNLSRST | 386 | 6 | 0.5 | 0.4 | 15.4 |
| QNSTRST | 387 | 6 | 0.5 | 0.1 | 41 |
| QGNNIKY | 388 | 6 | 0.6 | 0.2 | 29.5 |
| TNSVSNR | 389 | 6 | 0.4 | 0.3 | 17.2 |
| SNRTQQQ | 390 | 5.9 | 1.1 | 0.3 | 17.1 |
| TNQTSAI | 391 | 5.9 | 1.3 | 0.2 | 37.1 |
| SNLTGNR | 392 | 5.9 | 0 | 0.5 | 12.8 |
| SNNVRSH | 393 | 5.8 | 0.9 | 0.1 | 40.1 |
| QGTMQAN | 394 | 5.8 | 0 | 0.2 | 30 |
| TNKIQSS | 395 | 5.8 | 2.3 | 0.5 | 11.9 |
| SNTIPRN | 396 | 5.8 | 0.5 | 0.2 | 23.9 |
| SNVVQSK | 397 | 5.7 | 0.7 | 0.4 | 16.2 |
| QAGAGNI | 398 | 5.7 | 0 | 0.3 | 22.6 |
| MENTVRT | 399 | 5.6 | 2 | 0.5 | 11.1 |
| TNSARSN | 400 | 5.6 | 1.8 | 0.3 | 17.3 |
| TNRTHTL | 401 | 5.6 | 0 | 0.1 | 40.6 |
| INQTRQG | 402 | 5.6 | 3.3 | 0.4 | 14.2 |
| TFQNHTK | 403 | 5.6 | 1.1 | 0.2 | 26 |
| SNSSSGT | 404 | 5.5 | 0 | 0.3 | 17.9 |
| SNKISSM | 405 | 5.5 | 1.1 | 0.2 | 23.7 |
| SNQVRAP | 406 | 5.4 | 2.2 | 0.3 | 16.8 |
| SSNSIRT | 407 | 5.4 | 0.5 | 0.5 | 11.1 |
| GNTSRAA | 408 | 5.4 | 1.3 | 0.4 | 14.7 |
| TNSAKSL | 409 | 5.4 | 0.9 | 0.4 | 13.1 |
| SNRTSEA | 410 | 5.4 | 1.1 | 0.2 | 25.9 |
| SNSVTKI | 411 | 5.4 | 0 | 0.1 | 56.8 |
| SNQTSHL | 412 | 5.4 | 0.1 | 0.3 | 17 |
| TNRVAAT | 413 | 5.3 | 0.8 | 0.4 | 13.9 |
| ISTDRPQ | 414 | 5.3 | 1.4 | 0.1 | 39.4 |
| LPNTIRT | 415 | 5.3 | 1.7 | 0.3 | 15.6 |
| SINQTRS | 416 | 5.3 | 2.2 | 0.5 | 11.5 |
| DNGVRGT | 417 | 5.3 | 3.1 | 0.4 | 14.6 |
| TNDAKHR | 418 | 5.3 | 1.1 | 0.4 | 12.6 |
| SNSSKHN | 419 | 5.2 | 1.2 | 0.2 | 22.2 |
| GNFSRAG | 420 | 5.2 | 0.6 | 0.3 | 16.3 |
| TNRTAGP | 421 | 5.2 | 2.3 | 0.4 | 14.1 |
| SNATYKG | 422 | 5.1 | 0.5 | 0.2 | 22.8 |
| SNRTHPA | 423 | 5.1 | 0.4 | 0.4 | 11.7 |
| TNATGPL | 424 | 5.1 | 1.1 | 0.2 | 29.1 |
| EGNSVRV | 425 | 5.1 | 1.7 | 0.1 | 64.1 |
| YNITKQN | 426 | 5.1 | 0 | 0.3 | 15.7 |
| TNVSRLG | 427 | 5.1 | 0.7 | 0.3 | 14.9 |
| TNSTQSP | 428 | 5.1 | 1.5 | 0.1 | 39.9 |
| TNAVSPS | 429 | 5.1 | 2.6 | 0.3 | 14.8 |
| TNSTYAR | 430 | 5.1 | 0 | 0.1 | 64.5 |
| PNNYTKV | 431 | 5.1 | 0 | 0.1 | 44.1 |
| ENHTHAK | 432 | 5 | 0.1 | 0.1 | 66.1 |
| TNATSGG | 433 | 5 | 0.9 | 0.1 | 37.9 |
| SNRVTNG | 434 | 5 | 0.2 | 0.2 | 30.2 |
| SINSARA | 435 | 3.4 | 0.9 | 0.5 | 6.4 |
| HDNSARS | 436 | 1.7 | 20 | 0.4 | 4.7 |

### Cross-reactive AAV variants

The results of the second selection round in mice and the second selection round in NHP were analysed to identify AAV capsids variants with a brain enrichment score in mice and NHP superior to 1. A total of 1'076 AAV variants showed species cross-reactivity (i.e. transducing both mice and NHP brains). Out of these 1'076 cross-reactive AAV variants, 76 are high-performing capsids in NHP and are reported in Table 6.

**Table 6. AAV variants with NHP and mouse brain cross-reactivity**

| **AAV variants targeting peptide** | | **Enrichment score** | |
|---|---|---|---|
| **Amino acid sequence** | **SEQ ID NO** | **NHP brain** | **C57BL/6 mouse brain** |
| DNEVRSR | 181 | 35.4 | 5.5 |
| SNQVNRV | 182 | 35 | 10.7 |
| SNNTKTI | 192 | 25.1 | 22.5 |
| ENSAKQK | 193 | 25 | 12.6 |
| SNVSRAS | 203 | 20.4 | 20.9 |
| GNSVREQ | 206 | 19.6 | 8 |
| SDNHTKI | 208 | 19.3 | 5.7 |
| VVNTTQR | 210 | 19 | 1.6 |
| SNEARVH | 212 | 18.8 | 27.8 |
| SNLTGRS | 213 | 18.6 | 6.5 |
| GHNAIRN | 214 | 18.3 | 8.2 |
| GNQVRSS | 215 | 18.2 | 7.7 |
| HTNASRS | 217 | 17.3 | 8.9 |
| ESNHIRS | 224 | 16 | 14.1 |
| ELNSIKR | 232 | 15.1 | 25.5 |
| SNRTSAL | 235 | 14.9 | 20 |
| TNKTSGA | 234 | 14.9 | 8.2 |
| HLNSVRE | 243 | 14.1 | 38.7 |
| GNRSSSH | 245 | 14 | 17.3 |
| SSHNSVK | 246 | 14 | 25.7 |
| TNRVGTT | 256 | 12.7 | 19.7 |
| STNKIRD | 264 | 11.8 | 9.7 |
| GNATRQQ | 267 | 11.5 | 16.8 |
| HNATKLS | 269 | 11.4 | 32.2 |
| QNQIRPS | 271 | 11.3 | 2.4 |
| DNQVRAK | 272 | 11.1 | 27.4 |
| TGNAVRS | 273 | 11.1 | 14.3 |
| SGNHIRT | 275 | 10.6 | 9.2 |
| VNATQRT | 277 | 10.4 | 7.7 |
| SNKSQGQ | 280 | 10.1 | 22.9 |
| GNDTRKA | 282 | 9.9 | 10.1 |
| ANTIRSN | 284 | 9.7 | 3.1 |
| GVNVTKP | 289 | 9.4 | 20.2 |
| INNVRSQ | 292 | 9.3 | 11.6 |
| SNNAVVK | 293 | 9.3 | 8.4 |
| TNTASLR | 303 | 8.9 | 11.8 |
| TNTTRVG | 305 | 8.8 | 28.1 |
| TNKTLSS | 306 | 8.7 | 27.2 |
| DGNGVKQ | 315 | 8.1 | 38.1 |
| ANQTQSR | 321 | 8 | 5.2 |
| SNQSRDR | 322 | 8 | 7.5 |
| SNTIKGE | 323 | 8 | 5.9 |
| INNRAPQ | 325 | 8 | 15.9 |
| DNKIVRT | 328 | 7.9 | 4.1 |
| NANAIKQ | 330 | 7.8 | 13.7 |
| HNTTKTG | 339 | 7.4 | 8.5 |
| DSNRNKI | 341 | 7.3 | 42.3 |
| SNNVRPT | 342 | 7.3 | 15.9 |
| TNTVKSN | 346 | 7.1 | 11.8 |
| MPNTIKL | 351 | 7 | 16.1 |
| NANSIKN | 353 | 6.9 | 10.1 |
| SNRTSTI | 357 | 6.8 | 3.6 |
| GNSIRSG | 360 | 6.8 | 14.4 |
| TNESRAK | 361 | 6.7 | 7.1 |
| SNNVPSR | 363 | 6.7 | 8.1 |
| EANAVKT | 364 | 6.7 | 3.3 |
| ANNTRSA | 45 | 6.3 | 91.7 |
| SNGTKIA | 375 | 6.3 | 6.8 |
| DNQTRKP | 377 | 6.1 | 29 |
| QSNTIKA | 379 | 6.1 | 11.1 |
| SNLSRST | 386 | 6 | 10.5 |
| TNSVSNR | 389 | 6 | 11.6 |
| SNRTQQQ | 390 | 5.9 | 16.6 |
| MENTVRT | 399 | 5.6 | 6.1 |
| TFQNHTK | 403 | 5.6 | 7.5 |
| SNKISSM | 405 | 5.5 | 3.8 |
| TNSAKSL | 409 | 5.4 | 8.5 |
| SNQTSHL | 412 | 5.4 | 8.5 |
| TNRVAAT | 413 | 5.3 | 11.3 |
| SNRTHPA | 423 | 5.1 | 12.6 |
| TNATGPL | 424 | 5.1 | 28.7 |
| TNVSRLG | 427 | 5.1 | 8.7 |
| ENHTHAK | 432 | 5 | 15.3 |
| SNRVTNG | 434 | 5 | 6.9 |
| SINSARA | 435 | 3.4 | 5.6 |
| HDNSARS | 436 | 1.7 | 25 |

In summary, the applied directed evolution strategy comprising the insertion of a randomized heptamer targeting peptide in wild-type AAVrh10 and multiple selection rounds in alternating animal species resulted in the identification of 433 AAVrh10 variants with enhanced CNS tropism and liver de-targeting ability, of which 173 have a preferred mice CNS tropism (Table 4), 259 have a preferred NHP CNS tropism (Table 5), and 76 have a cross-reactive CNS tropism (Table 6).

### Example 3. In vivo characterization of AAV variants selected for enhanced CNS tropism in BALB/c and C57BL/6 mice.

To evaluate the biodistribution and transduction efficiency of AAVrh10 capsid variants identified from the selection in mice, a panel of 12 capsids (selected based on brain, spinal cord and liver enrichment scores, brain-to-liver ratio and sequence diversity) was produced and administered to BALB/c and C57BL/6 mice. Each AAV variant was produced individually in HEK293T cells using triple transfection system as described above. Of note, the Rep gene plasmid comprised the Cap gene of the corresponding variant and the plasmid AAV comprised the EGFP gene under the control of the chicken β-actin hybrid (CBh) promoter and a unique 8 bp DNA barcode inserted 5' of the EGFP gene and enabling AAV variant identification by NGS. Viral particles were purified by CsCl gradient ultra centrifugation. In addition to the 12 AAVrh10 variants, three controls - wild-type AAVrh10, wild-type AAV9 and AAV-PHP.eB (an AAV9 variant with an improved CNS tropism (Chan Ken Y et al. 2017)) were produced following the same protocol. Prior to injection, the 15 purified AAV preparations were mixed at equivalent ratio. The mixture of pooled AAVs was administered intravenously via tail vein injection in C57BL/6 mice (N=4, 2 males, 2 females, 6-8 weeks of age) and in BALB/c mice (N=4, 2 males, 2 females, 6-8 weeks of age) in single dose of 1x10¹² vg/mouse.

Three weeks post-injection, mice were euthanised and perfused with PBS, followed by brain, spinal cord, liver, heart, spleen, kidney and lung collection. Total mRNA was extracted from all the tissues and used for reverse transcription to obtain cDNA. The barcode regions, unique for each AAV vector, were amplified by conventional PCR using barcoded primers. Amplicons were analysed using NGS to determine the representation of each variant in the different tissues tested.

Raw Fastq files from NGS were processed to trim regions flanking individual barcodes. The extracted barcode sequences were counted, and normalized to the total number of reads, expressing data in reads per million (RPM). RPM values from each variant were normalized to RPM of the parent wild-type serotype AAVrh10. The fold-change in mRNA level over wild-type (WT) AAVrh10 are reported in Table 7 for C57BL/6 mice and Table 8 for BAL/C mice.

**Table 7. Fold-change in EGFP mRNA expression relative to wild-type AAVrh10 in different tissues of C57BL/6 mice**

| **AAV** | **SEQ ID NO** | **EGFP mRNA fold-change relative to WT AAVrh10** | | | | | | |
|---|---|---|---|---|---|---|---|---|
| | | **Brain** | **Spinal cord** | **Kidney** | **Muscle** | **Spleen** | **Heart** | **Liver** |
| ACI-AAVrh10-v4 | 437 | 22.38 | 20.94 | 0.32 | 0.8 | 0.44 | 0.69 | 0.01 |
| ACI-AAVrh10-v6 | 439 | 205.77 | 287.02 | 0.59 | 3.0 | 0.56 | 0.65 | 0.13 |
| ACI-AAVrh10-v5 | 438 | 96.51 | 135.58 | 0.25 | 1.6 | 0.17 | 0.39 | 0.05 |
| ACI-AAVrh10-v7 | 440 | 374.54 | 535.04 | 0.85 | 0.8 | 0.67 | 0.84 | 0.20 |
| ACI-AAVrh10-v9 | 441 | 164.30 | 296.20 | 0.50 | 0.9 | 0.50 | 1.23 | 0.20 |
| ACI-AAVrh10-v11 | 442 | 5.21 | 5.13 | 0.66 | 0.2 | 0.53 | 0.39 | 0.41 |
| ACI-AAVrh10-v36 | 447 | 4.37 | 3.74 | 0.85 | 0.5 | 0.88 | 0.29 | 0.41 |
| ACI-AAVrh10-v12 | 443 | 4.73 | 2.00 | 0.66 | 0.4 | 0.58 | 1.36 | 0.08 |
| ACI-AAVrh10-v60 | 448 | 18.47 | 42.10 | 0.26 | 1.3 | 0.32 | 0.34 | 0.28 |
| ACI-AAVrh10-v16 | 445 | 5.64 | 5.05 | 1.31 | 0.5 | 1.33 | 0.44 | 0.26 |
| ACI-AAVrh10-v13 | 444 | 5.87 | 5.97 | 2.18 | 0.6 | 1.62 | 0.40 | 0.55 |
| ACI-AAVrh10-v35 | 446 | 37.10 | 50.09 | 0.30 | 2.3 | 0.34 | 0.63 | 0.07 |
| AAV-PHP.eB | 3 | 181.76 | 302.64 | 0.43 | 0.1 | 0.93 | 0.27 | 0.01 |
| AAV9 | 2 | 4.36 | 4.37 | 2.23 | 1.5 | 3.07 | 3.71 | 0.88 |

**Table 8. Fold-change in EGFP mRNA expression relative to wild-type AAVrh10 in different tissues of in BALB/c mice**

| **AAV** | **SEQ ID NO** | **EGFP mRNA fold-change relative to WT AAVrh10** | | | | | | |
|---|---|---|---|---|---|---|---|---|
| | | **Brain** | **Spinal cord** | **Kidney** | **Muscle** | **Spleen** | **Heart** | **Liver** |
| ACI-AAVrh10-v4 | 437 | 32.21 | 20.30 | 0.61 | 0.52 | 0.54 | 0.31 | 0.01 |
| ACI-AAVrh10-v6 | 439 | 731.87 | 720.35 | 0.94 | 1.19 | 14.06 | 0.80 | 0.16 |
| ACI-AA Vrh10-v5 | 438 | 218.85 | 182.23 | 0.66 | 0.88 | 3.84 | 0.25 | 0.03 |
| ACI-AAVrh10-v7 | 440 | 888.12 | 902.60 | 1.01 | 0.44 | 17.95 | 0.79 | 0.16 |
| ACI-AAVrh10-v9 | 441 | 694.71 | 895.89 | 1.55 | 0.92 | 16.04 | 1.52 | 0.16 |
| ACI-AAVrh10-v11 | 442 | 6.41 | 5.95 | 1.95 | 0.35 | 0.67 | 0.41 | 0.31 |
| ACI-AAVrh10-v36 | 447 | 2.91 | 1.71 | 1.11 | 0.33 | 0.84 | 0.12 | 0.33 |
| ACI-AAVrh10-v12 | 443 | 6.17 | 4.85 | 1.71 | 0.89 | 0.78 | 1.47 | 0.08 |
| ACI-AAVrh10-v60 | 448 | 97.45 | 104.65 | 0.35 | 0.89 | 2.58 | 0.51 | 0.49 |
| ACI-AAVrh10-v16 | 445 | 4.39 | 2.92 | 0.74 | 0.45 | 1.43 | 0.22 | 0.17 |
| ACI-AAVrh10-v13 | 444 | 5.75 | 3.40 | 1.09 | 0.56 | 2.61 | 0.24 | 0.39 |
| ACI-AAVrh10-v35 | 446 | 132.92 | 118.01 | 0.42 | 1.84 | 2.45 | 0.32 | 0.10 |
| AAV-PHP.eB | 3 | 0.33 | 1.53 | 0.36 | 0.20 | 0.27 | 0.78 | 0.02 |
| AAV9 | 2 | 4.86 | 4.26 | 2.09 | 2.56 | 2.61 | 4.30 | 0.68 |

All twelve AAVrh10 variants presented an improved CNS tropism and lower off-target tissue transduction (de-targeting) compared to WT AAVrh10 in both mice strains. Moreover, the mRNA fold-change measured in the brain highly correlated between BALB/c and C57BL/6 strains (R²=0.9137) (Figure 2). As expected (Dayton Robert et al. 2018), AAV-PHP.eB only presented CNS transduction capacity in C57BL/6 mice (Table 7 and 8).

Among the capsids tested, ACI-AAVrh10-v7, ACI-AAVrh10-v6, ACI-AAVrh10-v9 and ACI-AAVrh10-v5 showed over 100-fold increase in mRNA level compared with wild-type AAVrh10 in the brain and/or spinal cord of both BALB/c and C57BL/6 mice (Figure 3 and 4). All four variants exhibited a favourable off-target tissue de-targeting profile in both mice strains compared to wild-type AAVrh10 ranging from 0.2 to 0.03-fold-reduction in EGFP mRNA level in liver (Table 7 and 8). In C57BL/6 mice, EGFP expression was reduced in all peripheral organs for ACI-AAVrh10-v7, -v6, -v9 and -v5 compared to wild-type AAVrh10, except for ACI-AAVrh10-v6 in muscle. In BALB/c mice, EGFP expression was reduced in most of the peripheral organs for ACI-AAVrh10-v7, -v6, -v9 and -v5 compared to wild-type AAVrh10, except for ACI-AAVrh10-v6 in muscle, ACI-AAVrh10-v6, -v5, -v7 and -v9 in spleen, and ACI-AAVrh10-v9 in kidney. All four AAVrh10 variants displayed a higher CNS transduction capacity than AAV-PHP.eB in BALB/c mice (Table 8, Figure 3), and ACI-AAVrh10-v6 and -v7 further showed an improved brain transduction capacity compared to AAV-PHP.eB in C57BL/6 mice (Table 7, Figure 4).

Altogether, the results show that all tested AAVrh10 variants present an improved CNS tropism and a lower off-target tissues transduction at least in the liver in both strains of mouse compared to AAV rh10 wild-type. The highest performing AAV variants are ACI-AAVrh10-v5, -v6, -v7 and -v9 which present a high CNS transduction ability and a low off-target tissues transduction profile in both BALB/c and C57BL/6 mice.

### Example 4. Cell-type and tissue selective expression properties of ACI-AAVrh10-v7 capsid using engineered genetic cargo design

To evaluate the cellular tropism of the engineered ACI-AAVrh10-v7 capsid in the CNS, the EGFP gene was delivered in C57BL/6 mice and expressed under the control of four different promoters. Two strong ubiquitous promoters, CAG (Miyazaki et al. 1989) and CBh (Gray et al. 2011), and two cell-selective promoters, human Synapsin 1 (hSYN1) (Kügler et al. 2003) for neurons and gfaABC(1)D (a modified GFAP promoter described in (Lee et al. 2008)) for astrocytes, were tested. For comparison, the same four promoters driving expression of the EGFP gene were also packaged with the wild-type AAVrh10 capsid as controls.

As shown in Table 7 of Example 3, a residual EGFP expression under CBh promoter was observed in off-target organs (i.e., tissues other than brain and spinal cord) when delivered with the ACI-AAVrh10-v7 capsid. To suppress transgene expression in off-target tissues while maintaining robust expression in brain and spinal cord, microRNA (miRNA or miR) binding sites (BS) were incorporated into the 3' untranslated region (UTR) of the EGFP gene. These miRNA BS were selected based on the abundance of complementary miRNA in specific tissues (Landgraf et al. 2007). To specifically silence expression in the liver, three repeats of the miR-122 BS (sequence fully complementary to miR-122-5p described in (Landgraf et al. 2007)) were inserted in 3' of the EGFP UTR. To reduce expression in liver, muscle and spleen, another construct was designed comprising three miR-122 BS, three miR-1 BS (to silence expression in muscle and heart) and three miR-142 BS (to silence expression in spleen). MiR-1 BS and miR-142 BS are complementary to mmu-miR-1a-3p and mmu-miR-142a-3p, respectively (Landgraf et al. 2007). The engineered genetic cargo designs were packaged in wild-type AAVrh10 and ACI-AAVrh10-v7 vectors. The constructs are listed in Table 9 and illustrated in Figure 5.

**Table 9. AAVrh10 and ACI-AAVrh10-v7 vectors administered in C57BL/6 mice**

| **Group** | **Capsid** | **Expression cassette** | **Number of mice** |
|---|---|---|---|
| 1 | AAVrh10 | CAG_EGFP_WPRE | 3 |
| 2 | AAVrh10 | CBh_EGFP_WPRE | 3 |
| 3 | AAVrh10 | hSYN1_EGFP_WPRE | 3 |
| 4 | AAVrh10 | gfaABC(1)D_EGFP_WPRE | 3 |
| 5 | ACI-AAV1h10-v7 | CAG_EGFP_WPRE | 3 |
| 6 | ACI-AAV1h10-v7 | CBh_EGFP_WPRE | 3 |
| 7 | ACI-AAVrh10-v7 | hSYN1_EGFP_WPRE | 3 |
| 8 | ACI-AAV1h10-v7 | gfaABC(1)D_EGFP_WPRE | 3 |
| 9 | ACI-AAVrh10-v7 | CBh_EGFP_3xmiR-122BS_WPRE | 3 |
| 10 | ACI-AAV1h10-v7 | CBh_EGFP_3xmiR-122BS_3xmiR-1BS 3xmiR-142BS_WPRE | 3 |

A single dose of AAV vector listed in Table 9 was injected intravenously in C57BL/6 mice (N=3 per group). Four weeks post-injection, mice were euthanised and perfused with PBS prior to organ collection. For each animal, one brain hemisphere and one portion of the liver were collected and fixed by immersion in freshly prepared 4% paraformaldehyde in PBS pH 7.4 for three hours at room temperature. All samples were then transferred to 15 % sucrose/PBS and stored in this solution at 4 °C. Samples were cryo-sectioned at 10 µm thickness using a cryotome. Three tissue sections per animal were used for immunofluorescence analysis. EGFP expression was detected using a goat polyclonal anti-EGFP (Abcam) primary antibody and an anti-goat IgG H+L Alexa Fluor 488-conjugated (Abcam) secondary antibody. Whole-slide scans of the stained sections were recorded using a slide scanner. In the brain, transduction efficiency was measured as the percentage of EGFP positive cells. Total cell number was determined using the DAPI fluorescence (nuclear marker). A circular region of interest (ROI) was computationally drawn around each nucleus and used to detect EGFP positive cells using a fixed threshold set on the mean EGFP fluorescence of each ROI. The number of EGFP positive cells was normalized to the total number of cells counted on the entire brain section. In the liver, the EGFP signal was quantified on the whole section of liver tissue defined as the ROI. The immunoreactive area was calculated as the percentage of the ROI exhibiting a positive EGFP signal. Quantification of the percentage of EGFP positive cells in the brain is reported in Table 10 and quantification of the % of EGFP immunoreactive area in the liver is reported in Table 11. Representative fluorescence microscopy images of the EGFP expression in the brain and in the liver are shown in Figure 6 and 7, respectively.

**Table 10. Mean % of EGFP positive cells in brain section of C57BL/6 mice**

| **Promoter** | **miR BS** | **EGFP+ cells %** | | **Fold-increase of ACI-AAVrh10-v7 relative to AAVrh10** |
|---|---|---|---|---|
| | | **AAVrh10** | **ACI-AAvrh10-v7** | |
| CAG | - | 0.19 | 44.74 | 236 |
| CBh | - | 0.02 | 2.29 | 115 |
| CBh | 122 | NA | 1.41 | - |
| CBh | 122_1_142 | NA | 2.38 | - |
| hSYN1 | - | 0.02 | 7.99 | 400 |
| gfaABC(1)D | - | 0.02 | 0.23 | 12 |

| | | | | |
|---|---|---|---|---|
| *NA: not tested* | | | | |

**Table 11. Mean % of EGFP immunoreactive area in liver section of C57BL/6 mice**

| **Promoter** | **miR BS** | **EGFP immunoreactive area %** | | **Fold-decrease of ACI-AAVrh10-v7 relative to AAVrh10** |
|---|---|---|---|---|
| | | **AAVrh10** | **ACI-AAvrh10-v7** | |
| CAG | - | 37.6 | 7.6 | 5 |
| CBh | - | 32.7 | 2.4 | 14 |
| CBh | 122 | NA | 0 | - |
| CBh | 122_1_142 | NA | 0 | - |
| hSYN1 | - | 0 | 0 | - |
| gfaABC(1)D | - | 0 | 0 | - |

| | | | | |
|---|---|---|---|---|
| *NA: not tested* | | | | |

In all groups injected with the AAVrh10 wild-type vectors, the EGFP signal in the brain was very weak with a mean percentage of EGFP positive cells ranging from 0.02% to 0.2% regardless of the promoter used (Table 10 and Figure 6). In contrast, mice injected with the ACI-AAVrh10-v7 vector showed a higher mean percentage of EGFP-positive cells in the brain compared to those injected with AAVrh10, across all four promoters tested. The percentage of EGFP-positive cells ranged from 0.23% with the gfaABC(1)D promoter to 44.74% with the CAG promoter (Table 10). Depending on the promoter, this corresponded to a 12- to 400-fold increase in EGFP-positive cells for ACI-AAVrh10-v7 relative to wild-type AAVrh10 (Table 10).

In the liver, mice injected with the ACI-AAVrh10-v7 vector comprising a ubiquitous promoter (CBh or CAG) showed a reduction in EGFP expression compared to those injected with AAVrh10. The mean percentage of EGFP-immunoreactive area decreased approximately 5-fold with the CAG promoter and 14-fold with the CBh promoter as compared to wild-type AAVrh10 vectors (Table 11). No EGFP signal was detected in the liver of mice injected with either AAVrh10 or ACI-AAVrh10-v7 using CNS-specific promoters hSYN1 or gfaABC(1)D.

The addition of miR-122 BS (three miR-122 BS alone or in combination with three miR-1 BS and three miR-142 BS) in the 3' UTR of the EGFP gene completely abolished the expression of EGFP in the liver, with no immunoreactive area detectable (Table 11 and Figure 7). Importantly, the use of miR BS did not impact the EGFP expression in the brain (Table 10. This demonstrates that three miR-122 BS can achieve liver-specific silencing, while miR-122, miR-1, and miR-142 BS do not impair transduction efficiency of the ACI-AAVrh10-v7 vector in the brain.

Altogether, these findings demonstrate that the engineered ACI-AAVrh10-v7 capsid enhances CNS transduction and reduces off-target liver transduction compared to the wild-type AAVrh10 capsid using the same genetic cargo. Additionally, the use of CNS-specific promoters (hSYN1 or gfaABC(1)D) or liver-specific silencing sequences, such as miR-122 BS, completely abolished EGFP expression in the liver. These results confirm the CNS selectivity of the promoters and the liver-silencing capacity of miR-122 BS when delivered by the ACI-AAVrh10-v7 vector.

### Example 5. In vivo characterization of AAV variants selected for enhanced CNS tropism in NHP

To evaluate the biodistribution and transduction efficiency of AAVrh10 capsid variants identified from the two rounds of selection in NHP, a panel of 49 capsids (described in Table 12) were selected based on virus production efficiency, brain, spinal cord and liver enrichment scores, frequency in NHP brain samples, brain-to-liver ratio, sequence diversity and cross-reactivity in mouse, and are being produced and administered to NHP.

**Table 12. AAVrh10 capsid variants selected for in vivo characterization in NHP**

| **AAV variant targeting peptide** | | **AAV variant capsid protein SEQ ID NO** |
|---|---|---|
| **Amino acid sequence** | **SEQ ID NO** | |
| PQNSARV | 178 | 449 |
| SNLTRHT | 180 | 450 |
| DNEVRSR | 181 | 451 |
| YVGSAKQ | 183 | 452 |
| QSNSVKP | 186 | 453 |
| ENNTKPT | 188 | 454 |
| PGNTIRQ | 190 | 455 |
| DTNVTRA | 196 | 456 |
| ENRARAA | 202 | 457 |
| IGNSVRQ | 220 | 458 |
| ELNSSRS | 221 | 459 |
| QDNRIHQ | 227 | 460 |
| VKHETGR | 229 | 461 |
| GKNQPVR | 233 | 462 |
| TNKTSGA | 234 | 463 |
| NSNSVRA | 244 | 464 |
| GNRSSSH | 245 | 465 |
| SSHNSVK | 246 | 466 |
| SNRIQHT | 249 | 467 |
| TNLIKSN | 250 | 468 |
| ANSVRAN | 253 | 469 |
| LQNQTKL | 254 | 470 |
| SGNHIRT | 275 | 471 |
| DQNLTRR | 281 | 472 |
| MNKVSAI | 286 | 473 |
| GVNVTKP | 289 | 474 |
| SNSSKEY | 290 | 475 |
| SNNHTKT | 297 | 476 |
| DGNGVKQ | 315 | 477 |
| ANQTQSR | 321 | 478 |
| SNSVSHR | 327 | 479 |
| NANAIKQ | 330 | 480 |
| SNTIRGG | 331 | 481 |
| HNTTKTG | 339 | 482 |
| TNVTITK | 345 | 483 |
| TNTVKSN | 346 | 484 |
| SANSVKN | 350 | 485 |
| NANSIKN | 353 | 486 |
| TNESRAK | 361 | 487 |
| TNTARSG | 362 | 488 |
| SNYVHSA | 369 | 489 |
| DNQTRKP | 377 | 490 |
| TNHTSSR | 384 | 491 |
| TSTNYTK | 385 | 492 |
| SNVVQSK | 397 | 493 |
| SNSSSGT | 404 | 494 |
| SNATYKG | 422 | 495 |
| SINSARA | 435 | 496 |
| HDNSARS | 436 | 497 |

Unless defined otherwise, all technical and scientific terms used herein have the same meanings as commonly understood by one of ordinary skill in the art to which this invention belongs. All publications and patents specifically mentioned herein are incorporated by reference in their entirety for all purposes in connection with the invention.

The present invention is not to be limited in scope by the specific embodiments described herein. Indeed, various modifications of the invention in addition to those described herein will become apparent to those skilled in the art from the foregoing description and accompanying figures. Such modifications are intended to fall within the scope of the appended claims. Moreover, all aspects and embodiments of the invention described herein are considered to be broadly applicable and combinable with any and all other consistent embodiments, including those taken from other aspects of the invention (including in isolation) as appropriate.

### References

Boutin S, Monteilhet V, Veron P, Leborgne C, Benveniste O, Montus MF, Masurier C. 2010. Prevalence of Serum IgG and Neutralizing Factors Against Adeno-Associated Virus (AAV) Types 1, 2, 5, 6, 8, and 9 in the Healthy Population: Implications for Gene Therapy Using AAV Vectors. Hum Gene Ther. 21(6):704-712. doi:10.1089/hum.2009.182.
Bushnell B, Rood J, Singer E. 2017. BBMerge - Accurate paired shotgun read merging via overlap. PLoS One. 12(10):E185056. doi:10.1371/journal.pone.0185056.
Chan Ken Y, Jang Min J, Yoo Bryan B, Greenbaum Alon, Ravi Namita, Wu Wei-Li, Sánchez-Guardado Luis, Lois Carlos, Mazmanian Sarkis K, Deverman Benjamin E, et al. 2017. Engineered AAVs for efficient noninvasive gene delivery to the central and peripheral nervous systems. Nature Neuroscience. 20(8):1172-1179. doi:10.1038/nn.4593.
Chan, Maturana, Engel. 2022. Optimized formulation buffer preserves adeno-associated virus-9 infectivity after 4 °C storage and freeze/thawing cycling. J Virol Methods. 309. doi:10.1016/j.jviromet.2022.114598.
Dayton Robert, Grames Mychal, Klein Ronald. 2018. More expansive gene transfer to the rat CNS: AAV PHP.EB vector dose-response and comparison to AAV PHP.B. Gene Ther. 25(5):392-400. doi:10.1038/s41434-018-0028-5.
Deverman Benjamin E, Pravdo Piers L, Simpson Bryan P, Kumar Sripriya Ravindra, Chan Ken Y, Banerjee Abhik, Wu Wei-Li, Yang Bin, Huber Nina, Pasca Sergiu P, et al. 2016. Cre-dependent selection yields AAV variants for widespread gene transfer to the adult brain. Nature Biotechnology.(2):204-209. doi:10.1038/nbt.3440.
Duan D. 2023. Lethal immunotoxicity in high-dose systemic AAV therapy. Mol Ther. 31(11):3123-3126. doi:10.1016/j.ymthe.2023.10.015.
Gao G, Vandenberghe LH, Alvira MR, Lu Y, Calcedo R, Zhou X, Wilson JM. 2004. Clades of Adeno-Associated Viruses Are Widely Disseminated in Human Tissues. J Virol. 78(12):6381-6388. doi:10.1128/JVI.78.12.6381-6388.2004.
Gray SJ, Foti SB, Schwartz JW, Bachaboina L, Taylor-Blake B, Coleman J, Ehlers MD, Zylka MJ, McCown TJ, Samulski RJ. 2011. Optimizing Promoters for Recombinant Adeno-Associated Virus-Mediated Gene Expression in the Peripheral and Central Nervous System Using Self-Complementary Vectors. Hum Gene Ther. 22(9):1143-1153. doi:10.1089/hum.2010.245.
Kügler S, Kilic E, Bähr M. 2003. Human synapsin 1 gene promoter confers highly neuron-specific long-term transgene expression from an adenoviral vector in the adult rat brain depending on the transduced area. Gene Ther. 10(4):337-347. doi:10.1038/sj.gt.3301905.
Kuzmin DA, Shutova MV, Johnston NR, Smith OP, Fedorin VV, Kukushkin YS, van der Loo JCM, Johnstone EC. 2021. The clinical landscape for AAV gene therapies. Nature Reviews Drug Discovery. 20(3):173-174. doi:10.1038/d41573-021-00017-7.
Landgraf P, Rusu M, Sheridan R, Sewer A, Iovino N, Aravin A, Pfeffer S, Rice A, Kamphorst AO, Landthaler M, et al. 2007. A Mammalian microRNA Expression Atlas Based on Small RNA Library Sequencing. Cell. 129(7):1401-1414. doi:10.1016/j.cell.2007.04.040.
Lee Y, Messing A, Su M, Brenner M. 2008. GFAP promoter elements required for region-specific and astrocyte-specific expression. Glia. 56(5):481-493. doi:10.1002/glia.20622.
Lundstrom K. 2023. Viral Vectors in Gene Therapy: Where Do We Stand in 2023? Viruses. 15(3):698. doi:10.3390/v15030698.
Mietzsch M, Barnes C, Hull JA, Chipman P, Xie J, Bhattacharya N, Sousa D, McKenna R, Gao G, Agbandje-McKenna M. 2020. Comparative Analysis of the Capsid Structures of AAVrh.10, AAVrh.39, and AAV8. J Virol. 94(6):E19-E1769. doi:10.1128/JVI.01769-19.
Miyazaki J, Takaki S, Araki K, Tashiro F, Tominaga A, Takatsu K, Yamamura K. 1989. Expression vector system based on chicken β-actin promoter directs efficient production of Interleukin-5. Gene. 79:269-277. doi:10.1016/0378-1119(89)90209-6.
Samulski RJ, Muzyczka N. 2014. AAV-Mediated Gene Therapy for Research and Therapeutic Purposes. Annu Rev Virol. 1(1):427-451. doi:10.1146/annurev-virology-031413-085355.
Stagg SM, Yoshioka C, Davulcu O, Chapman MS. 2022. Cryo-electron Microscopy of Adeno-associated Virus. Chem Rev. 122(17):14018-14054. doi:10.1021/acs.chemrev.1c00936.
Thwaite R, Pagès G, Chillón M, Bosch A. 2014. AAVrh.10 immunogenicity in mice and humans. Relevance of antibody cross-reactivity in human gene therapy. Gene Ther. 22(2). doi:10.1038/gt.2014.103.
Verdera HC, Kuranda K, Mingozzi F. 2020. AAV Vector Immunogenicity in Humans: A Long Journey to Successful Gene Transfer. Mol Ther. 28(3):723-746. doi:10.1016/j.ymthe.2019.12.010.
Wörner TP, Bennett A, Habka S, SnijderJ, Friese O, Powers T, Agbandje-McKenna M, Heck AJR. 2021. Adeno-associated virus capsid assembly is divergent and stochastic. Nature Communications. 12(1). doi:10.1038/s41467-021-21935-5.
Wu Zhijian, Yang Hongyan, Colosi Peter. 2010. Effect of Genome Size on AAV Vector Packaging. Mol Ther. 18(1):80-86. doi:10.1038/mt.2009.255.

## Claims

1. A central nervous system (CNS) targeting peptide comprising or consisting of an amino acid sequence of any one of SEQ ID NOs: 315, 4-314 or 316-436.

2. The CNS targeting peptide of claim 1, which comprises or consists of the amino acid sequence DGNGVKQ (SEQ ID NO: 315), DNEVRSR (SEQ ID NO: 181), SSHNSVK (SEQ ID NO: 246), IGNSVRQ (SEQ ID NO: 220) or ANQTQSR (SEQ ID NO: 321).

3. The CNS targeting peptide of claim 1 or 2, which comprises or consists of the amino acid sequence DGNGVKQ (SEQ ID NO: 315).

4. The CNS targeting peptide of claim 1, which comprises or consists of the amino acid sequence YEVGGTL (SEQ ID NO: 7), SHSPEFK (SEQ ID NO: 6), GGLTPPY (SEQ ID NO: 9) or DSRLLLP (SEQ ID NO: 5).

5. The CNS targeting peptide of claim 1 or 4, which comprises or consists of the amino acid sequence YEVGGTL (SEQ ID NO: 7).

6. An AAV capsid protein or an AAV capsid comprising an AAV capsid protein, wherein the AAV capsid protein comprises the CNS targeting peptide of any one of the preceding claims.

7. The AAV capsid protein or AAV capsid of claim 6, wherein the AAV capsid protein is an AAVrh10 capsid protein of SEQ ID NO: 1, AAV2 capsid protein of SEQ ID NO: 498, AAV1 capsid protein of SEQ ID NO: 499, AAV4 capsid protein of SEQ ID NO: 502, AAV8 capsid protein of SEQ ID NO: 505, AAV9 capsid protein of SEQ ID NO: 2, or an AAV capsid protein having at least 95%, at least 96%, at least 97%, at least 98% or at least 99% sequence identity to any one of SEQ ID NOs: 1, 2, 498, 499 or 505.

8. The AAV capsid protein or AAV capsid of claim 6 or 7, wherein the AAV capsid protein is an AAVrh10 capsid protein of SEQ ID NO: 1 or an AAV capsid protein having at least 95%, at least 96%, at least 97%, at least 98% or at least 99% sequence identity to SEQ ID NO: 1.

9. The AAV capsid protein or AAV capsid of any one of claims 6 to 8, wherein the AAV capsid protein is an AAVrh10 capsid protein of SEQ ID NO: 1 or an AAV capsid protein having at least 95%, at least 96%, at least 97%, at least 98% or at least 99% sequence identity to SEQ ID NO: 1, and wherein the CNS targeting peptide is inserted in VR-VIII of the AAV capsid protein.

10. The AAV capsid protein or AAV capsid of claim 9, wherein the CNS targeting peptide is inserted between amino acids 589 and 590 of the AAV capsid protein.

11. The AAV capsid protein or AAV capsid of any one of claims 6 to 10, wherein the AAV capsid protein comprises or consists of an amino acid sequence of any one of SEQ ID NOs: 477, 437-476 or 478-497.

12. An AAV vector, which comprises the AAV capsid protein or AAV capsid of any one of claims 6 to 11 and an AAV genome comprising an expression cassette flanked by inverted terminal repeats (ITRs), wherein the expression cassette comprises from 5' to 3': a ubiquitous or a CNS-specific promoter and one or more transgenes.

13. The AAV vector of claim 12, wherein the expression cassette comprises from 5' to 3': a CAG, CBh, hSYN1 or gfaABC(1)D promoter, one or more transgenes, and a WPRE.

14. The AAV vector of claim 12 or 13, which exhibits an increased transduction of cells of the brain and spinal cord and a decreased transduction of cells of the liver, wherein the increase in transduction of cells of the brain and spinal cord and the decrease in transduction of cells of the liver are relative to a corresponding AAV vector comprising an AAV capsid protein which does not comprise the CNS targeting peptide.

15. The AAV vector of claim 14, wherein the increased transduction of cells of the brain and spinal cord is at least 50-fold and the decreased transduction of cells of the liver is at least 5-fold.
